(19) Europäisches Patentamt
European Patent Office
Office européen des brevets

(11) Veröffentlichungsnummer: **0 183 994 B1**

# (12) EUROPÄISCHE PATENTSCHRIFT

(45) Veröffentlichungstag der Patentschrift: **21.03.90**

(21) Anmeldenummer: **85113864.4**

(22) Anmeldetag: **31.10.85**

(51) Int. Cl.[4]: **C07D 495/14**, C07D 455/02, C07D 455/06, C07D 471/04, C07D 513/04, C07D 513/18, C07D 513/22, A61K 31/435, A61K 31/55, A61K 31/54 // (C07D495/14, 333:00, 221:00, 221:00),(C07D513/14, 333:00, 279:00, 277:00),(C07D513/14, 333:00, 277:00, 223:00),(C07D513/14, 333:00, 281:00, 277:00),(C07D495/14, 333:00, 223:00), C07D221:00

(54) **Tricyclische Pyridonderivate.**

(30) Priorität: **06.11.84 CH 5304/84**
**05.09.85 CH 3836/85**

(43) Veröffentlichungstag der Anmeldung: **11.06.86 Patentblatt 86/24**

(45) Bekanntmachung des Hinweises auf die Patenterteilung: **21.03.90 Patentblatt 90/12**

(84) Benannte Vertragsstaaten: **AT BE CH DE FR IT LI LU NL SE**

(56) Entgegenhaltungen:
**EP-A- 0 044 573**

**PHARMAZIE, Band 23, 1968, Seiten 301-303, Berlin, DD; G. BUCHMANN et al.: "Beitrag Synthese von 1.3-disubstituierten 4 H-Chinolizonen-(4) auf Basis Pyridyl-2-acetonitril"**
**JOURNAL OF ORGANIC CHEMISTRY, Band 37, nr. 11, 1972, Seiten 1823-1825; S.I. GOLDBERG et al.: "Leguminosae alkaloids. VIII. Development of an improved synthesis of anagyrine as a potential route to other lupin alkaloids"**
**CHEMICAL ABSTRACTS, Band 82, 1975, Seite 417, Nr. 43159u, Columbus, Ohio, US; T. KATO et al.: "Synthesis of methylpyridine derivatives. XXVII. Syntheses of 1,3-disubstituted-4-oxoquinolizine and 2,4-disubstituted-1-oxobenzo[c]quinolizine derivatives" 000**
**CANADIAN JOURNAL OF CHEMISTRY, Band 63, Nr. 4, 1985, Seiten 882-886, Ottawa, CA; D.M. McKINNON et al.: "The preparation of some fused isothiazole derivatives"**

(73) Patentinhaber: **F. HOFFMANN-LA ROCHE AG, Postfach 3255, CH-4002 Basel(CH)**

(72) Erfinder: **Fischer, Ulf, Dr., Madlenweg 2, CH-4402 Frenkendorf(CH)**
Erfinder: **Schneider, Fernand, Dr., Marignanostrasse 28, CH-4059 Basel(CH)**
Erfinder: **Widmer, Ulrich, Dr., Alleeweg 13, CH-4310 Rheinfelden(CH)**

(74) Vertreter: **Lederer, Franz, Dr. et al, Van der Werth, Lederer & Riederer Patentanwälte Lucile-Grahn-Strasse 22, D-8000 München 80(DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft Verbindungen der allgemeinen Formel

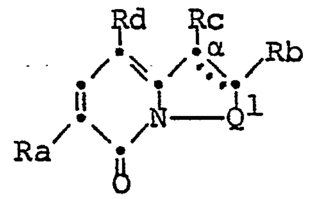

I

worin $Q^1$ zusammen mit dem Stickstoffatom eine Gruppe der Formel >N–$CH_2CH_2$– (a), >N–$CH_2CH_2CH_2$– (b), >N–CH=CH– (c), >N–$CH_2$–CH=CH– (d), >N–$CH_2$–$S(O)_p$– (e), >N–$CH_2CH_2$–$S(O)_p$– (f) oder >N–CH=CH–$S(O)_p$– (g), p die Zahl 0, 1 oder 2 und Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >$C_\alpha$–S–CH=CH– (h), >$C_\alpha$–CH=CH–S– (i) oder >$C_\alpha$–CH=CH–CH=CH– (j), die gestrichelte Linie eine zusätzliche Bindung, Rd die Gruppe der Formel $-(A^1)_m-(CO)_n-(Q^2A^2)_q-R^1$, m, n und q je die Zahl 0 oder 1, $A^1$ niederes Alkylen, $A^2$ niederes Alkylen, eine direkte Bindung oder die Gruppe –CO–, $Q^2$ ein Sauerstoffatom oder die Gruppe $-NR^2-$, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen und gegebenenfalls durch eine ($C_{3-6}$)-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte ($C_{3-7}$)-Cycloalkylgruppe oder zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe >N–$R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Maßgabe, daß n die Zahl 0 bedeutet, wenn q die Zahl 1 und $A^2$ die Gruppe –CO– bedeuten, daß $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe –CO– bedeuten, und daß $R^1$ eine von Hydroxy, Cyano, Nitro, Halogen, niederes Alkoxycarbonyl, niederes Alkoxy und $-NR^3R^4$ verschiedene Bedeutung hat, wenn q die Zahl 1 und $A^2$ eine direkte Bindung bedeuten,
und pharmazeutisch annehmbare Säureadditionssalze von Verbindungen der Formel I mit einem oder mehreren basischen Substituenten.

Diese neuen tricyclischen Pyridonderivate besitzen wertvolle pharmakologische Eigenschaften und können zur Bekämpfung oder Verhütung von Krankheiten verwendet werden. Sie sind inbesondere muskelrelaxierend, sedativ-hypnotisch, anxiolytisch und/oder antikonvulsiv wirksam und können demnach bei der Bekämpfung oder Verhütung von Muskelspannungen, Spannungszuständen, Schlaflosigkeit, Angstzuständen und/oder Konvulsionen verwendet werden.

Gegenstand der vorliegenden Erfindung sind: Die obigen Verbindungen der Formel I und die erwähnten Salze davon als solche und als therapeutische Wirkstoffe; ein Verfahren und Zwischenprodukte zu deren Herstellung; die Herstellung der Zwischenprodukte und deren Verwendung zur Herstellung von therapeutischen Wirkstoffen; Arzneimittel auf der Basis dieser neuen Wirkstoffe und deren Herstellung; die Verwendung der neuen Stoffe bei der Bekämpfung oder Verhütung von Krankheiten; sowie deren Verwendung zur Herstellung von muskelrelaxierend, sedativ-hypnotisch, anxiolytisch und/oder antikonvulsiv wirksamen Mitteln.

In Pharmazie 23, 301–303 (1968), J. Org. Chem. 37, 1823–1825 (1972) und Chem. Pharm. Bull. 22, 744–751 (1974) werden einige bicyclische Pyridonderivate, nämlich 1,3-disubstituierte 4-Oxo-chinolizinderivate, als chemische Substanzen beschrieben. In EP-A 0 044 573 werden 7-Phenylchinolizine als Zwischenprodukte für die Herstellung von antipsychotisch, antiemetisch und analgetisch wirksamen 7-Phenylchinolizidinen beschrieben.

Der Ausdruck "nieder" bezeichnet Reste und Verbindungen mit höchstens sieben, vorzugsweise höchstens vier Kohlenstoffatomen. Der Ausdruck "Alkyl" für sich allein genommen oder in Kombinationen, wie Alkanoyl, Alkanoyloxy und Alkoxyalkyl, bezeichnet geradkettige oder verzweigte, gesättigte Kohlenwasserstoffreste, wie Methyl, Äthyl, Propyl, Isopropyl und t-Butyl. Der Ausdruck "Cycloalkyl" bezeichnet cyclische, gesättigte Kohlenwasserstoffreste, wie Cyclohexyl. Der Ausdruck "Alkoxy" bezeichnet über ein Sauerstoffatom gebundene Alkylgruppen, wie Methoxy und Äthoxy. Der Ausdruck "Hydroxyalkyl" bezeichnet durch Hydroxy substituierte Alkylgruppen, wie 2-Hydroxyäthyl. Die Ausdrücke "Alkanoyl" und "Alkanoyloxy" bezeichnen Fettsäurereste, wie Acetyl und Acetoxy. Der Ausdruck "Alkylen" bezeichnet geradkettige oder verzweigte, gesättigte Kohlenwasserstoffreste mit zwei freien Valenzen, wie Methylen, 1,2-Äthylen und 1,3-Propylen. Der Ausdruck "Halogen" bezeichnet die vier Formen Fluor, Chlor, Brom und Jod.

Der Ausdruck "Aryl" bezeichnet vorzugsweise Phenylgruppen, welche gegebenenfalls durch Halogen, Trifluormethyl, niederes Alkyl, niederes Alkoxy, Nitro, Amino oder mono- oder di(niederes Alkyl)amino substituiert sind.

Die über ein Kohlenstoffatom gebundenen, 5-gliedrigen, gesättigten, partiell ungesättigten oder aromatischen Heterocyclen enthalten als Heteroringglieder vorzugsweise ein Sauerstoff- oder Schwefelatom oder eine Imino- oder niedere Alkyliminogruppe und gegebenenfalls ein oder zwei Stickstoffatome, wobei das Kohlenstoffatom, über das der Heterocyclus gebunden ist, sich vorzugsweise neben einem oder zwischen zwei Heteroatomen befindet. Beispiele solcher Heterocyclen, welche noch wie erwähnt substituiert sein können, sind: 2-Oxazolin-2-yl, 3-Methyl-1,2,4-oxadiazol-5-yl, 2-Thiazolin-2-yl, 2-Tetrahydrofuryl und 2-Thiazolyl.

Der Ausdruck "3- bis 7-gliedriger, gesättigter N-Heterocyclus, der als Ringglied noch ein Sauerstoff- oder Schwefelatom oder die Gruppe >N-$R^5$ enthalten kann" als Bedeutungsmöglichkeit für -$NR^3R^4$ bezeichnet einerseits Heterocyclen mit nur einem Heteroatom, nämlich dem Stickstoffatom, über das sie gebunden sind, und andererseits Heterocyclen mit zwei Heteroatomen, nämlich dem besagten Stickstoffatom und einem Sauerstoff- oder Schwefelatom oder einem zweiten Stickstoffatom. Beispiele derartiger Heterocyclen, welche noch wie erwähnt substituiert sein können, sind: 2-(niederes Alkoxyalkyl)-1-azetidinyl, 3-(niederes Alkoxy)1-azetidinyl, 3-Hydroxy-1-azetidinyl, 2-(niederes Hydroxyalkyl)-1-azetidinyl, 2-(niederes Alkanoyloxyalkyl)-1-pyrrolidinyl, 3- Oxo-1-pyrrolidinyl, 2-(niederes Alkoxycarbonyl)-1-pyrrolidinyl, 3-(niederes Alkoxy)-1-pyrrolidinyl, 3-Hydroxy-1-pyrrolidinyl, 2-(niederes Alkoxyalkyl)-1-pyrrolidinyl, 2-(niederes Hydroxyalkyl)-1-pyrrolidinyl, 2-(niederes Hydroxyalkyl)-4-hydroxy-1-pyrrolidinyl, 2-(niederes Alkoxyalkyl)-4-(niederes Alkoxy)-1-pyrrolidinyl, 4-Morpholinyl, 2,6-Di(niederes Alkyl)-4-morpholinyl, 4-Thiomorpholinyl, 1-Piperazinyl, 1-(niederes Alkyl)-4-piperazinyl, 1-(niederes Alkoxylalkyl)-4-piperazinyl, 1-(niederes Alkanoyl)-4-piperazinyl, 4-(niederes Hydroxyalkyl)-1-piperidinyl, 4-Oxo-1-piperidinyl, 4-(niederes Alkoxy)-1-piperidinyl, 4-(niederes Alkoxycarbonyl)-1-piperidinyl, 4-Hydroxy-1-piperidinyl, 4-(niederes Alkylcarbamoyl)-1-piperidinyl, 4-(niederes Alkanoyloxy)-1-piperidinyl, 2-(niederes Alkoxyalkyl)-1-piperidinyl, 2-(niederes Hydroxyalkyl)-1-piperidinyl, 3-(niederes Alkoxy)-1-piperidinyl, 4,4-(niederes Alkylendioxy)-1-piperidinyl und 3-Hydroxy-1-piperidinyl.

Das Symbol $Q^1$ bedeutet zusammen mit dem Stickstoffatom vorzugsweise die Gruppe der Formel >N-$CH_2CH_2$- (a) oder >N-CH=CH- (c). Das Symbol Ra bedeutet vorzugsweise eine gegebenenfalls durch m-Halogen oder m-Trifluormethyl substituierte Phenylgruppe, wobei die Bedeutungsmöglichkeit Phenyl besonders bevorzugt ist. Die Symbole Rb und Rc bedeuten zusammen mit dem mit $\alpha$ bezeichneten Kohlenstoffatom vorzugsweise eine gegebenenfalls durch Halogen substituierte Gruppe der Formel >$C_\alpha$-S-CH=CH- (h) oder >$C_\alpha$-CH=CH-CH=CH- (j), insbesondere die Gruppe der Formel >$C_\alpha$-S-CH=CH- oder >$C_\alpha$-CH=CCl-CH=CH-, wobei die gestrichelte Linie eine zusätzliche Bindung bedeutet. In einer bevorzugten Ausführungsform bedeuten $Q^2$ ein Sauerstoffatom, $A^2$ die Gruppe -CO-, $R^1$ die Gruppe -$NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann, und entweder m und q die Zahl 0 und n die Zahl 1 oder m und q die Zahl 1 und n die Zahl 0. In einer besonders bevorzugten Ausführungsform bedeuten $A^1$ Methylen, $Q^2$ ein Sauerstoffatom, $A^2$ die Gruppe -CO-, $R^1$ die Gruppe -$NR^3R^4$, $R^3$ niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten 1-Azetidinyl-, 1-Pyrrolidinyl-, 1-Piperidinyl- oder 4-Morpholinylgruppe und entweder m und q die Zahl 0 und n die Zahl 1 oder m und q die Zahl 1 und n die Zahl 0. In einer ganz besonders bevorzugten Ausführungsform bedeuten $A^1$ Methylen, $Q^2$ ein Sauerstoffatom, $A^2$ die Gruppe -CO-, $R^1$ die Gruppe -$NR^3R^4$, $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl, 2-(niederes Hydroxyalkyl)-1-pyrrolidinyl, 4-Hydroxy-1-piperidinyl, 4-(niederes Alkoxy)-1-piperidinyl, 4-Morpholinyl oder 2,6-di(niederes Alkyl)-4-morpholinyl und entweder m und q die Zahl 0 und n die Zahl 1 oder m und q die Zahl 1

und n die Zahl 0. In einer speziellen Ausführungsform bedeuten m und q die Zahl 0, n die Zahl 1 und $R^1$ Hydroxy oder niederes Alkoxy.

Im Rahmen der vorliegenden Erfindung ganz besonders bevorzugte Verbindungen der Formel I sind:

10-Chlor-6,7-dihydro-N-(2-methoxyäthyl)-4-oxo-3-phenyl-4H-benzo[a]chinolizin-1-carboxamid,
1-[(10-Chlor-6,7-dihydro-4-oxo-3-phenyl-4H-benzo[a]chinolizin-1-yl)carbonyl]-4-piperidinol,
(4,5-Dihydro-7-oxo-8-phenyl-7H-thieno[2,3-a]chinolizin-10-yl)methyl-4-morpholincarboxylat,
4-[(6,7-Dihydro-4-oxo-3-phenyl-10-chlor-4H-benzo[a]chinolizin-1-yl)carbonyl]-2,6-dimethylmorpholin,
(S)-1-[(7-Oxo-8-phenyl-7H-thieno[2,3-a]chinolizin-10-yl)carbonyl]-2-pyrrolidinmethanol,
(S)-2-Methoxymethyl-1-[(7-oxo-8-phenyl-7H-thieno[2,3-a]chinolizin-10-yl)carbonyl]pyrrolidin,
1-[(10-Chlor-4-oxo-3-phenyl-4H-benzo[a]chinolizin-1-yl)carbonyl]-3-methoxypyrrolidin,
(S)-1-[(10-Chlor-4-oxo-3-phenyl-4H-benzo[a]chinolizin-1-yl)carbonyl]-2-pyrrolidinmethanol,
cis-4-[(4,5-Dihydro-7-oxo-8-phenyl-7H-thieno[2,3-a]chinolizin-10-yl)carbonyl]-2,6-dimethylmorpholin,
1-[(10-Chlor-6,7-dihydro-4-oxo-3-phenyl-4H-benzo[a]chinolizin-1-yl)carbonyl]-4-methoxypiperidin,
1-[(10-Chlor-3-phenyl-4-oxo-4H-benzo[a]chinolizin-1-yl)carbonyl]-4-methoxypiperidin,
N-Aethyl-N-(2-methoxyäthyl)-7-oxo-8-phenyl-7H- thieno[2,3-a]chinolizin-10-carboxamid,
N-(2-Methoxyäthyl)-N-methyl-7-oxo-8-phenyl-7H- thieno[2,3-a]chinolizin-10-carboxamid,
(R)-2-(Methoxymethyl)-1-[(7-oxo-8-phenyl-7H- thieno[2,3-a]chinolizin-10-yl)carbonyl]pyrrolidin,
1-[(10-Chlor-6,7-dihydro-4-oxo-3-phenyl-4H-benzo[a]chinolizin-1-yl)carbonyl]-3-methoxypyrrolidin,
1-[(10-Chlor-6,7-dihydro-4-oxo-3-phenyl-4H-benzo[a]chinolizin-1-yl)carbonyl]-3-äthoxypyrrolidin,
(R)-3-Methoxy-1-[(7-oxo-8-phenyl-7H-thieno[2,3-a]chinolizin-10-yl)carbonyl]pyrrolidin,
(S)-3-Methoxy-1-[(7-oxo-8-phenyl-7H-thieno[2,3-a]chinolizin-10-yl)carbonyl]pyrrolidin,
(S)-1-[(10-Chlor-6,7-dihydro-4-oxo-3-phenyl-4H-benzo[a]chinolizin-1-yl)carbonyl]-2-(methoxymethyl)pyrrolidin,
1-[(4,5-Dihydro-7-oxo-8-phenyl-7H-thieno[2,3-a]chinolizin-10-yl)carbonyl]-3-methoxypyrrolidin,
3-Methoxy-1-[(7-oxo-8-phenyl-7H-thieno[2,3-a]chinolizin-10-yl)carbonyl]azetidin,
(R)-1-[(10-Chlor-4-oxo-3-phenyl-4H-benzo[a]chinolizin- 1-yl)carbonyl]-2-pyrrolidinmethanol,
(S)-1-[(10-Chlor-4-oxo-3-phenyl-4H-benzo[a]chinolizin- 1-yl)carbonyl]-2-(methoxymethyl)pyrrolidin und
(R)-1-[(10-Chlor-4-oxo-3-phenyl-4H-benzo[a]chinolizin-1- yl)carbonyl]-2-(methoxymethyl)pyrrolidin.

Weitere bevorzugte Verbindungen der Formel I sind:

8-(m-Fluorphenyl)-4,5-dihydro-7-oxo-7H-thieno[2,3-a]chinolizin-10-carbonsäure-methylester,
4,5-Dihydro-7-oxo-8-phenyl-7H-thieno[2,A3-a]chinolizin- 10-carbonsäure-methylester,
4,5-Dihydro-7-oxo-8-phenyl-7H-thieno[2,3-a]chinolizin- 10-carbonsäure-äthylester,
4,5-Dihydro-7-oxo-8-phenyl-7H-thieno[2,3-a]chinolizin- 10-carbonsäure-isopropylester,
4,5-Dihydro-7-oxo-8-phenyl-7H-thieno[2,3-a]chinolizin- 10-carbonsäure-tert-butylester,
8-(m-Chlorphenyl)-4,5-dihydro-7-oxo-7H-thieno[2,3-a]chinolizin-10-carbonsäure-methylester,
10-Chlor-6,7-dihydro-4-oxo-3-phenyl-4H-benzo[a]chinolizin-1-carbonsäure-methylester und
6,7-Dihydro-3-phenyl-4-oxo-4H-benzo[a]chinolizin-1-carbonsäure-methylester.

Die Verbindungen der Formel I und die pharmazeutisch annehmbaren Säureadditionssalze von Verbindungen der Formel I mit einem basischen Substituenten können erfindungsgemäss hergestellt werden, indem man

a) eine Verbindung der allgemeinen Formel

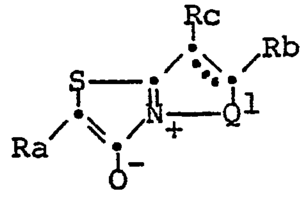

II

worin $Q^1$, Ra, Rb, Rc und die gestrichelte Linie die obige Bedeutung besitzen, bei erhöhter Temperatur mit einer Verbindung der allgemeinen Formel

HC≡C-Rd' III oder $H_2C$=CH-Rd' IV

worin Rd' Cyano, Nitro oder die Gruppe der Formel $-CO-(Q^2A^2)_q-R^1$ bedeutet, und q, $A^2$, $Q^2$ und $R^1$ die obige Bedeutung besitzen, oder mit Phenylvinylsulfoxid umsetzt und das erhaltene Cycloadditionsprodukt gegebenenfalls mit einer starken Base behandelt, oder

b) eine Verbindung der allgemeinen Formel ROOC-C(Ra)=CHR' V
worin R niederes Alkyl und R' Wasserstoff oder niederes Alkoxy bedeuten, und Ra die obige Bedeutung besitzt, wenn R' Wasserstoff bedeutet, bei erhöhter Temperatur oder, wenn R' niederes Alkoxy bedeutet, in Gegenwart einer starken Base mit einer Verbindung der allgemeinen Formel

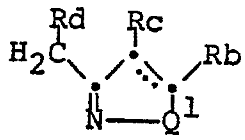
VI

worin $Q^1$, Rb, Rc, Rd und die gestrichelte Linie die obige Bedeutung besitzen, umsetzt und das erhaltene Cyclokondensationsprodukt, wenn R' Wasserstoff bedeutet, dehydriert, oder

c) eine Verbindung der Formel I, welche eine veresterte Carboxygruppe besitzt, hydrolysiert, oder

d) eine Carbonsäure der allgemeinen Formel

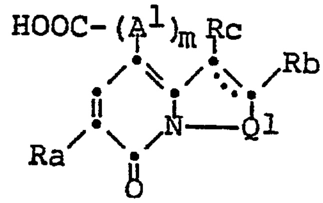
Ia

worin $A^1$, $Q^1$, Ra, Rb, Rc, m und die gestrichelte Linie die obige Bedeutung besitzen, mit einem Alkohol der allgemeinen Formel $HO\text{-}A^{21}\text{-}R^1$ VII
worin $A^{21}$ niederes Alkylen oder eine direkte Bindung bedeuten und $R^1$ die obige Bedeutung besitzt, verestert, oder

e) eine Carbonsäure der obigen Formel Ia oder eine Carbonsäure der allgemeinen Formel

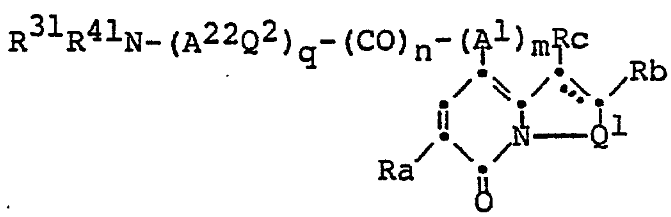
VIIIa

worin $A^{22}$ niederes Alkylen oder die Gruppe -CO- und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- oder Schwefelatom oder die Gruppe $>N\text{-}R^5$ enthalten kann, und $A^1$, $Q^1$, $Q^2$, Ra, Rb, Rc, $R^5$, m, n, q und die gestrichelte Linie die obige Bedeutung besitzen, oder ein reaktives Derivat davon mit einem Amin der allgemeinen Formel
$HNR^2\text{-}A^{21}\text{-}R^1$ IX oder $HNR^3R^4$ X
worin $A^{21}$, $R^1$, $R^2$, $R^3$ und $R^4$ die obige Bedeutung besitzen, bzw. mit Ammoniak oder einem mono- oder di(niederen Alkyl)amin in das entsprechende Amid überführt, oder

f) eine Verbindung der allgemeinen Formel

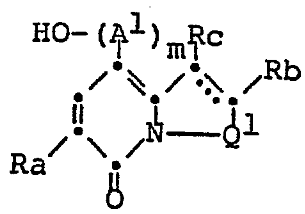
Ib'

worin $A^1$, $Q^1$, Ra, Rb, Rc, m und die gestrichelte Linie die obige Bedeutung besitzen,, in Gegenwart einer Base mit einer Verbindung der allgemeinen Formel
$X\text{-}A^{21}\text{-}R^1$ XI
worin X eine Abgangsgruppe bedeutet, und $A^{21}$ und $R^1$ die obige Bedeutung besitzen, bzw. eine Verbindung der Formel I, welche eine freie Hydroxygruppe besitzt, mit einer Verbindung der allgemeinen Formel

R-X XII

worin R niederes Alkyl bedeutet, und X obige Bedeutung besitzt, umsetzt, oder

g) eine Verbindung der allgemeinen Formel

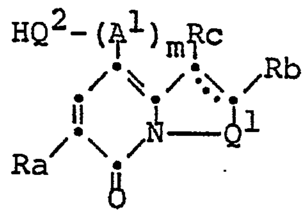

Ib

worin $A^1$, $Q^1$, $Q^2$, Ra, Rb, Rc, m und die gestrichelte Linie die obige Bedeutung besitzen, in Gegenwart eines säurebindenden Mittels mit einem reaktiven Derivat einer Carbonsäure der allgemeinen Formel $R^1$-COOH XIII

worin $R^1$ die obige Bedeutung besitzt, umsetzt, oder

h) eine Verbindung der allgemeinen Formel

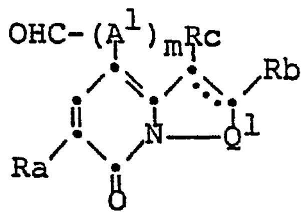

Ic

worin $A^1$, $Q^1$, Ra, Rb, Rc, m und die gestrichelte Linie die obige Bedeutung besitzen, in Gegenwart eines Reduktionsmittels mit einem Amin der obigen Formel IX oder X umsetzt, oder

i) eine Verbindung der allgemeinen Formel

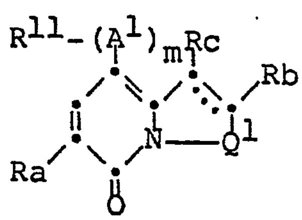

Id

worin $R^{11}$ Nitro, Cyano oder niederes Alkoxycarbonyl bedeutet, und $A^1$, $Q^1$, Ra, Rb, Rc, m und die gestrichelte Linie die obige Bedeutung besitzen, oder eine Verbindung der obigen Formel Ia oder ein reaktives Derivat davon reduziert, oder

j) einen Alkohol der obigen Formel Ib' oder einen Alkohol der allgemeinen Formel

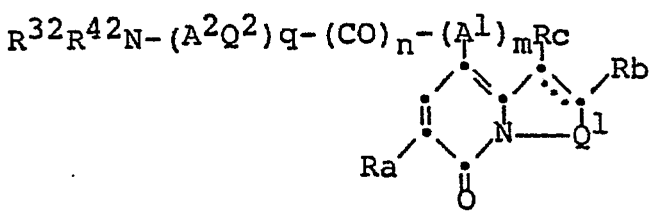

Ie

worin $A^1$, $A^2$, $Q^1$, $Q^2$, Ra, Rb, Rc, m, n, q und die gestrichelte Linie die obige Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigten N-Heterocyclus bedeuten, der als Ringglied noch ein Sauerstoff- oder Schwefelatom oder die Gruppe >N-$R^5$ enthalten kann, und $R^5$ die obige Bedeutung besitzt, oxidiert, oder

k) ein Isocyanat der allgemeinen Formel

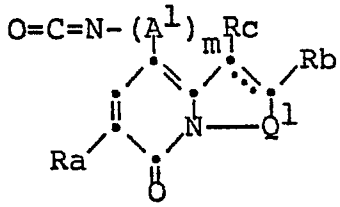 VIIIb oder $O=C-N-R^{33}$ XIV

worin $A^1$, $Q^1$, Ra, Rb, Rc, m und die gestrichelte Linie die obige Bedeutung besitzen, und $R^{33}$ Wasserstoff, niederes Alkyl oder ($C_{3-7}$)-Cycloalkyl bedeutet, mit einem niederen Alkohol oder einem Amin der obigen Formel X bzw. mit einer Verbindung der obigen Formel Ib umsetzt, oder

l) eine Verbindung der allgemeinen Formel

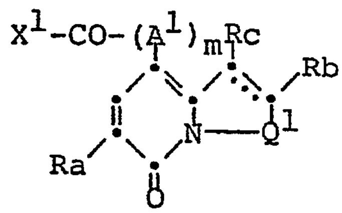 VIIIc

worin $X^1$ ein Halogenatom bedeutet, und $A^1$, $Q^1$, Ra, Rb, Rc, m und die gestrichelte Linie die obige Bedeutung besitzen, mit einem niederen Alkylmagnesiumhalogenid umsetzt, oder

m) eine Verbindung der allgemeinen Formel

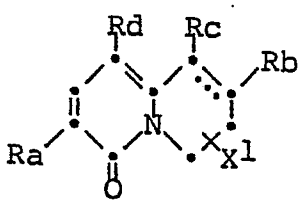 XVa oder 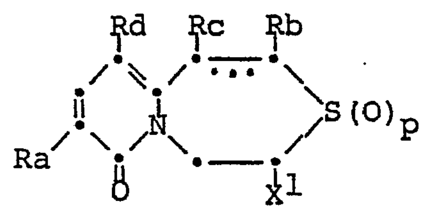 XVb

worin Ra, Rb, Rc, Rd, $X^1$, die gestrichelte Linie und p die obige Bedeutung besitzen, in Gegenwart einer Base dehydrohalogeniert, oder

n) eine Verbindung der allgemeinen Formel

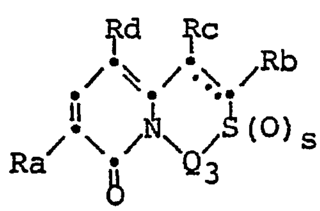 If

worin $Q^3$ die Gruppe $-CH_2-$, $-CH_2CH_2-$ oder $-CH=CH-$ und s die Zahl 0 oder 1 bedeuten, und Ra, Rb, Rc, Rd und die gestrichelte Linie die obige Bedeutung besitzen, S-oxidiert, oder

o) eine Verbindung der allgemeinen Formel

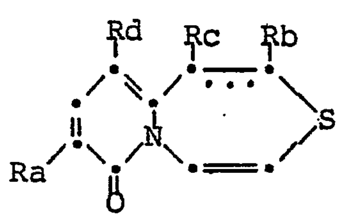 Ig

worin Ra, Rb, Rc, Rd und die gestrichelte Linie die obige Bedeutung besitzen, erhitzt, oder

p) eine Verbindung der allgemeinen Formel

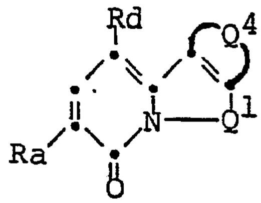

Ih

worin $Q^4$ die obige Gruppe (h) oder (i) bedeutet und $Q^1$, Ra und Rd obige Bedeutung besitzen, am Thiophenring halogeniert, oder

q) eine Verbindung der obigen Formel VIIIc in Gegenwart einer Base mit einer Verbindung der allgemeinen Formel

HYN=C($NH_2$)-R" XVI,

$H_2N$-CHR"-CHR"'-Y'H XVII oder

$H_2N$-NH-C(R")=Y" XVIII

worin Y ein Sauerstoffatom oder die Gruppe -NR"'-, Y' ein Sauerstoffatom oder die Gruppe -NH-, Y" ein Sauerstoff- oder Schwefelatom und R" und R"' je Wasserstoff oder niederes Alkyl bedeuten, umsetzt und das erhaltene Produkt cyclisiert, oder

r) eine Verbindung der allgemeinen Formel

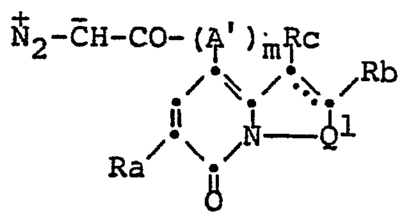

VIIId

worin A' $C_{1-6}$-Alkylen bedeutet, und $Q^1$, Ra, Rb, Rc, die gestrichelte Linie und m die obige Bedeutung besitzen, mit einem niederen Alkohol umsetzt, oder

s) eine Carbonsäure der Formel Ia, worin m die Zahl 0 bedeutet, decarboxyliert, oder

t) eine Verbindung der Formel I, worin Rd Wasserstoff bedeutet, am Pyridonring halogeniert, oder

u) in einer Verbindung der allgemeinen Formel

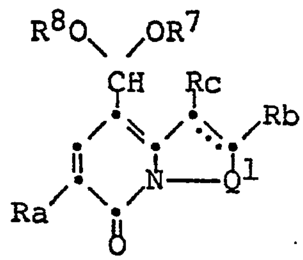

VIIIe

worin $R^7$ und $R^8$ je niederes Alkyl oder zusammen niederes Alkylen bedeuten, und $Q^1$, Ra, Rb, Rc und die gestrichelte Linie die obige Bedeutung besitzen, die Acetalgruppe spaltet, oder

v) eine Verbindung der Formel I, worin $Q^1$ zusammen mit dem Stickstoffatom die Gruppe >N-CH=CH- bedeutet, hydriert, oder

w) eine Verbindung der allgemeinen Formel

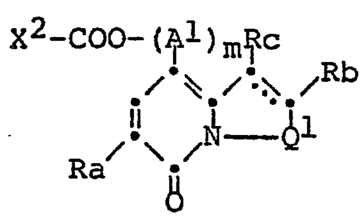

VIIIf

worin $X^2$ Phenoxy bedeutet, und $A^1$, $Q^1$, Ra, Rb, Rc, die gestrichelte Linie und m die obige Bedeutung besitzen, mit einem Amin der obigen Formel X umsetzt, und

x) erwünschtenfalls eine erhaltene Verbindung der Formel I mit einem basischen Substituenten in ein pharmazeutisch annehmbares Säureadditionssalz überführt.

Bei verschiedenen der obigen erfindungsgemässen Verfahren müssen die in den Ausgangsstoffen allfällig vorhandenen reaktionsfähigen Amino-, Carboxy- und/oder Hydroxylgruppen durch Schutzgruppen blockiert sein. Diese Fälle sind für den Fachmann ohne weiteres erkennbar, und auch die Auswahl der jeweils geeigneten Schutzgruppen bereitet ihm keine Schwierigkeiten.

Gemäss Verfahrensvariante a) können Verbindungen der Formel I hergestellt werden, worin Rd Wasserstoff, Cyano, Nitro oder die Gruppe der Formel -CO-$(Q^2A^2)_q$-$R^1$ bedeutet und q, $A^2$, $Q^2$ und $R^1$ obige Bedeutung besitzen. Die Umsetzung erfolgt zweckmässigerweise in einem inerten Lösungsmittel, das bei erhöhter Temperatur, vorzugsweise oberhalb 80°C siedet. Geeignete Lösungsmittel sind beispielsweise aromatische Kohlenwasserstoffe, wie Benzol, Toluol und Xylol, wobei die Reaktion vorzugsweise bei der Rückflusstemperatur des Lösungsmittels durchgeführt wird.

Bei der Umsetzung einer Verbindung der Formel II mit einer Verbindung der Formel III oder mit Phenylvinylsulfoxid erhält man, wenn man bei erhöhter Temperatur arbeitet, direkt die entsprechende Verbindung der Formel I. Bei der Umsetzung einer Verbindung der Formel II mit einer Verbindung der Formel IV erhält man als Cycloadditionsprodukt zunächst die entsprechende Epithio-Verbindung der allgemeinen Formel

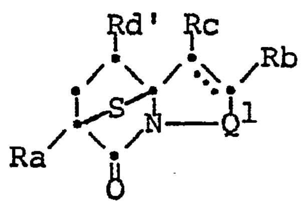

XIX

worin Ra, Rb, Rc, Rd', $Q^1$ und die gestrichelte Linie obige Bedeutung besitzen, welche anschliessend durch Behandeln mit einer starken Base in die entsprechende Verbindung der Formel I übergeführt wird. Geeignete Basen sind beispielsweise die niederen Alkalimetallalkoholate, wie Natriummethylat, wobei man als Lösungsmittel zweckmässigerweise den entsprechenden niederen Alkohol verwendet. Die Reaktion wird vorzugsweise bei der Rückflusstemperatur des Lösungsmittels durchgeführt.

Die Reaktion einer Verbindung der Formel V, worin R' Wasserstoff bedeutet, mit einer Verbindung der Formel VI gemäss Verfahrensvariante b) kann ohne Lösungsmittel oder in Gegenwart eines bei erhöhter Temperatur siedenden Lösungsmittels durchgeführt werden. Geeignete Lösungsmittel sind beispielsweise aromatische Kohlenwasserstoffe, wie Benzol, Toluol und Xylol. Die Cyclokondensation wird jedoch vorzugsweise ohne Lösungsmittel in einem Temperaturbereich von etwa 80°C bis etwa 150°C durchgeführt. Das so erhaltene Cyclokondensationsprodukt, nämlich eine Verbindung der allgemeinen Formel

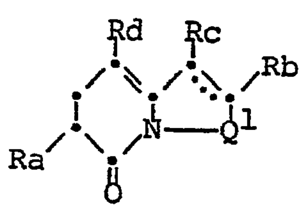

XXa

worin Ra, Rb, Rc, Rd, $Q^1$ und die gestrichelte Linie obige Bedeutung besitzen, wird anschliessend mit einem geeigneten Oxidationsmittel, wie Mangandioxid, dehydriert. Geeignete Lösungsmittel sind beispielsweise aromatische Kohlenwasserstoffe, wie Benzol, Toluol und Xylol. Die Dehydrierung wird vorzugsweise in einem Temperaturbereich von etwa Raumtemperatur bis zur Siedetemperatur des gewählten Lösungsmittels, vorzugsweise bei der Siedetemperatur, durchgeführt.

Durch Reaktion einer Verbindung der Formel V, worin R' niederes Alkoxy bedeutet, in Gegenwart einer starken Base, wie Natriumhydrid, und in einem inerten Lösungsmittel, vorzugsweise in einem Aether, wie Tetrahydrofuran, mit einer Verbindung der Formel VI gemäss Verfahrensvariante b) erhält man die entsprechende Verbindung der Formel I. Die Reaktionstemperatur liegt in einem Bereich von Raumtemperatur bis zur Siedetemperatur der Reaktionsmischung.

Die Verbindungen der Formeln XIX und XXa sind neu und ebenfalls Gegenstand der vorliegenden Erfindung.

Verbindungen der Formel I, welche eine veresterte Carboxygruppe besitzen, können gemäss Verfahrensvariante c) hydrolysiert werden, wobei man die entsprechenden freien Carbonsäuren erhält. Die Hydrolyse kann nach an sich bekannten Methoden durchgeführt werden. Die Hydrolyse wird vorzugsweise mit einem Alkalimetallhydroxid, wie Natriumhydroxid und Kaliumhydroxid, in einem niederen Alkohol, wie Methanol und Aethanol, oder in einem Gemisch aus einem niederen Alkohol und Wasser durchgeführt. Die Reaktionstemperatur liegt zweckmässigerweise in einem Bereich von Raumtemperatur bis zur Siedetemperatur des Reaktionsgemisches, wobei man vorzugsweise bei der Siedetemperatur des Reaktionsgemisches arbeitet.

Durch Veresterung einer Carbonsäure der Formel Ia mit einem Alkohol der Formel VII gemäss Verfahrensvariante d) können Verbindungen der Formel I hergestellt werden, worin Rd die Gruppe der Formel $-(A^1)_m$-CO-O-$A^{21}$-$R^1$ bedeutet und $A^1$, $A^{21}$, $R^1$ und m obige Bedeutung besitzen. Die Veresterung kann beispielsweise in Gegenwart eines Veresterungsreagenzes in einem inerten organischen Lösungsmittel durchgeführt werden. Geeignete Reagenzien sind beispielsweise N-Methyl-2-chlorpyridiniumjodid und dergleichen, organische Sulfonsäurehalogenide, wie Methylsulfonylchlorid, p-Toluolsulfonylchlorid und Mesitylensulfonsäurechlorid, und dergleichen. Geeignete Lösungsmittel sind beispielsweise halogenierte Kohlenwasserstoffe, wie Methylenchlorid, Chloroform und dergleichen. Geeignete Basen sind beispielsweise tertiäre Amine, wie Triäthylamin, Tri-n-butylamin und dergleichen. Die Reaktion wird vorzugsweise in einem Temperaturbereich von Raumtemperatur bis zur Rückflusstemperatur des Lösungsmittels durchgeführt.

Die gewünschte Veresterung kann auch dadurch bewerkstelligt werden, dass man die Carbonsäure der Formel Ia zunächst in ein reaktives Derivat überführt und dieses dann in Gegenwart einer Base mit einem Alkohol der Formel VII umsetzt. Als reaktive Derivate verwendet man vorzugsweise die entsprechenden Carbonsäurechloride. Als Basen eignen sich beispielsweise die bereits erwähnten tertiären Amine. Die Reaktion wird vorzugsweise in einem Temperaturbereich von etwa Raumtemperatur bis zur Rückflusstemperatur des Reaktionsgemisches durchgeführt, wobei man zweckmässigerweise bei Raumtemperatur arbeitet.

Die Veresterung mit einem Alkohol der Formel VII, worin $A^{21}$ niederes Alkylen und $R^1$ Wasserstoff bedeuten, d.h. mit einem niederen Alkohol, kann auch durch Umsetzen der Carbonsäure mit einem di(niederen Alkyl)acetal des N,N-Dimethylformamids durchgeführt werden. Die Umsetzung mit einem di(niederen Alkyl)acetal des N,N-Dimethylformamids wird vorzugsweise in einem inerten Lösungsmittel, beispielsweise in einem aromatischen Kohlenwasserstoff, wie Benzol, bei der Rückflusstemperatur des Reaktionsgemisches durchgeführt.

Durch Reaktion einer Carbonsäure der Formel Ia oder eines reaktiven Derivates davon mit einem Amin der Formel IX oder X gemäss Verfahrensvariante e) können Verbindungen der Formel I hergestellt werden, worin Rd die Gruppe $-(A^1)_m$-CO-$NR^2$-$A^{21}$-$R^1$ oder $-(A^1)_m$-CO-$NR^3R^4$ bedeutet und $A^1$, $A^{21}$, $R^1$, $R^2$, $R^3$, $R^4$ und m obige Bedeutung besitzen.

Durch Umsetzen einer Carbonsäure der Formel VIIIa oder eines reaktiven Derivates davon mit Ammoniak oder einem mono- oder di(niederen Alkyl)amin gemäss Verfahrensvariante e) können entsprechende Verbindungen der Formel I hergestellt werden, worin $R^1$ eine Gruppe der Formel $-NR^3R^4$ und $R^3$ und $R^4$ zusammen mit dem Stickstoffatom einen 3- bis 7-gliedrigen, gegebenenfalls durch eine oder zwei niedere Alkylgruppen und durch eine Carbamoyl- oder mono- oder di(niedere Alkyl)carbamoylgruppe substituierten, gesättigten N-Heterocyclus bedeuten, der als Ringglied noch ein Sauerstoff- oder Schwefelatom oder die Gruppe >N-$R^5$ enthalten kann, und $R^5$ obige Bedeutung besitzt.

Verwendet man als Ausgangsstoff die freie Carbonsäure der Formel Ia oder VIIIa, so wird die Amidierungsreaktion vorzugsweise in Gegenwart eines Kondensationsmittels, wie N-Methyl-2-chlorpyridiniumjodid, in einem inerten organischen Lösungsmittel und in Gegenwart einer Base durchgeführt. Geeignete Lösungsmittel sind beispielsweise aromatische Kohlenwasserstoffe wie Benzol, Toluol und Xylol. Geeignete Basen sind beispielsweise die weiter oben erwähnten tertiären Amine. Bevorzugte reaktive Carbonsäurederivate, welche in Gegenwart einer Base direkt mit dem entsprechenden Amin umgesetzt werden können, sind die entsprechenden Carbonsäurechloride. Geeignete Basen sind wiederum die vorerwähnten tertiären Amine. Geeignete Lösungsmittel sind beispielsweise aromatische Kohlenwasserstoffe, wie Benzol, Toluol und Xylol, und Aether, wie Dioxan. Die Reaktion wird in beiden Fällen vorzugsweise in einem Bereich von Raumtemperatur bis Rückflusstemperatur des Reaktionsgemisches durchgeführt.

Gemäss Verfahrensvariante f) können einerseits Verbindungen der Formel I hergestellt werden, worin Rd eine Gruppe der Formel $-(A^1)_m$-O-$A^{21}$-$R^1$ bedeutet, und $A^1$, $A^{21}$, $R^1$ und m obige Bedeutung besitzen, und andererseits Verbindungen der Formel I, welche eine in Form eines niederen Alkyläthers verätherte Hydroxygruppe besitzen.

Die Umsetzung einer Verbindung der Formel Ib' mit einer Verbindung der Formel XI bzw. die Umsetzung einer Verbindung der Formel I, welche eine freie Hydroxygruppe besitzt, mit einer Verbindung der Formel XII wird zweckmässigerweise in einem inerten organischen Lösungsmittel, wie N,N-Dimethylformamid oder dergleichen, durchgeführt, wobei man als Base zweckmässigerweise eine starke Base, z.B. ein Alkalimetallhydrid oder -hydroxyd, wie Natriumhydrid, Kaliumhydroxyd und Natriumhydroxyd, verwendet. Man arbeitet zweckmässigerweise in einem Bereich von 0°C bis Raumtemperatur. Die mit X bezeichnete Abgangsgruppe ist vorzugsweise ein Halogenatom, insbesondere ein Chlor-, Brom- oder Jodatom, oder eine Alkyl- oder Arylsulfonyloxygruppe, beispielsweise eine Methansulfonyloxy- oder p-Toluolsulfonyloxygruppe. Im Falle der Herstellung von niederen Alkyläthern kann X auch eine niedere

Alkoxysulfonyloxygruppe bedeuten, d.h. dass das Alkylierungsmittel in diesem Fall ein Di(niederes Alkyl)sulfat, wie Dimethylsulfat, ist.

Gemäss Verfahrensvariante g) können Verbindungen der Formel I hergestellt werden, worin Rd eine Gruppe der Formel $-(A^1)_m-Q^2-CO-R^1$ bedeutet, und $A^1$, $Q^2$, $R^1$ und m obige Bedeutung besitzen.

Die Umsetzung einer Verbindung der Formel Ib mit einem reaktiven Derivat einer Carbonsäure der Formel XIII, beispielsweise einem Carbonsäurechlorid, wird zweckmässigerweise in einem inerten organischen Lösungsmittel in Gegenwart eines säurebindenden Mittels, beispielsweise einem tertiären Amin, durchgeführt. Als Lösungsmittel eignen sich beispielsweise aromatische Kohlenwasserstoffe, wie Benzol, Toluol und Xylol, und halogenierte Kohlenwasserstoffe, wie Methylenchlorid. Wenn $R^1$ niederes Alkyl bedeutet, können auch entsprechende Carbonsäureanhydride verwendet werden, wobei man in diesem Fall als Lösungsmittel und als säurebindendes Mittel zweckmässigerweise Pyridin verwendet. Die Reaktion wird vorzugsweise in einem Temperaturbereich von etwa 0°C bis Siedetemperatur des Lösungsmittels durchgeführt.

Gemäss Verfahrensvariante h) können Verbindungen der Formel I hergestellt werden, worin Rd eine Gruppe der Formel $-(A^1)_m-CH_2-NR^2A^{21}-R^1$ oder $-(A^1)_m-CH_2-NR^3R^4$ bedeutet und $A^1$, $A^{21}$, $R^1$, $R^2$, $R^3$, $R^4$ und m obige Bedeutung besitzen. Die Reaktion wird vorzugsweise in einem niederen Alkohol als Lösungsmittel und mit Natriumcyanoborhydrid als Reduktionsmittel durchgeführt, wobei man zweckmässigerweise bei Raumtemperatur arbeitet und das Amin in Form seines Hydrochlorides einsetzt.

Gemäss Verfahrensvariante i) können Verbindungen der Formel I hergestellt werden, worin Rd eine Gruppe der Formel $-(A^1)_m-R^{12}$ und $R^{12}$ Amino, Aminomethyl, Hydroxymethyl oder Methyl bedeuten und $A^1$ und m obige Bedeutung besitzen. Die Wahl des geeigneten Reduktionsmittels richtet sich einerseits nach dem verwendeten Ausgangsstoff und andererseits nach dem gewünschten Produkt. Eine Verbindung der Formel Id, worin $R^{11}$ Cyano bedeutet kann beispielsweise mit Diboran in Tetrahydrofuran zur entsprechenden Aminomethylverbindung reduziert werden. Eine Verbindung der Formel Id, worin $R^{11}$ Nitro bedeutet, kann beispielsweise mit Natriumsulfid in einem niederen Alkohol, wie Methanol, zur entsprechenden Aminoverbindung reduziert werden. Eine Verbindung der Formel Id worin $R^{11}$ niederes Alkoxycarbonyl bedeutet, kann mit Lithiumborhydrid, und das Säurechlorid einer Verbindung der Formel Ia mit Natriumborhydrid in Tetrahydrofuran und/oder Dimethylformamid zur entsprechenden Hydroxymethylverbindung reduziert werden. Eine Carbonsäure der Formel Ia kann beispielsweise mit Boran/Tetrahydrofuran-Komplex oder Boran/Methylsulfid-Komplex in Tetrahydrofuran zur entsprechenden Methylverbindung reduziert werden.

Gemäss Verfahrensvariante j) können Verbindungen der Formel I hergestellt werden, worin Rd eine Gruppe der Formel $-(A^1)_m-CHO$ oder $-(A^1)_m-(CO)_n-(Q^2A^2)_q-NR^{34}R^{44}$ und $R^{34}$ und $R^{44}$ zusammen einen 3- bis 7-gliedrigen, gegebenenfalls durch eine oder zwei niedere Alkylgruppen und durch eine Oxogruppe substituierten, gesättigten N-Heterocyclus bedeuten, der als Ringglied noch ein Sauerstoff- oder Schwefelatom oder die Gruppe $>N-R^5$ enthalten kann, und $A^1$, $A^2$, $Q^2$, $R^5$, m, n und q obige Bedeutung besitzen. Die Oxidation von Alkoholen der Formeln Ib' und Ie kann nach an sich bekannten und jedem Fachmann geläufigen Methoden durchgeführt werden. Man kann die gewünschte Oxidation beispielsweise mit Mangandioxid in einem halogenierten Kohlenwasserstoff, wie Methylenchlorid bei Raumtemperatur bewerkstelligen. Die gewünschte Oxidation kann jedoch auch mit Pyridiniumchlorochromat in einem halogenierten Kohlenwasserstoff, wie Methylenchlorid, bei Raumtemperatur, oder mit Dimethylsulfoxid/Trifluoressigsäureanhydrid in einem halogenierten Kohlenwasserstoff, wie Methylenchlorid, bei Temperaturen von etwa -70°C bewerkstelligt werden.

Gemäss Verfahrensvariante k) können durch Umsetzung eines Isocyanates der Formel VIIIb mit einem niederen Alkohol oder einem Amin der Formel X, bzw. durch Umsetzen eines Isocyanates der Formel XIV mit einer Verbindung der Formel Ib Verbindungen der Formel I hergestellt werden, worin Rd eine Gruppe der Formel $-(A^1)_m-NHCO-R^{13}$, $-(A^1)_m-NHCO-NR^3R^4$ oder $-(A^1)_m-Q^2-CO-NH-R^{33}$ und $R^{13}$ niederes Alkoxy bedeuten, und $A^1$, $Q^2$, $R^3$, $R^{33}$ , $R^4$ und m obige Bedeutung besitzen. Diese Umsetzung wird zweckmässigerweise in einem inerten Lösungsmittel, beispielsweise in einem aromatischen Kohlenwasserstoff, wie Benzol, Toluol oder Xylol, in einem halogenierten Kohlenwasserstoff, wie Methylenchlorid, oder in einem Aether, wie Dioxan, durchgeführt. Die Reaktion wird vorzugsweise in einem Temperaturbereich von etwa Raumtemperatur bis zur Siedetemperatur des Reaktionsgemisches durchgeführt. Verwendet man ein Isocyanat der Formel XIV, worin $R^{33}$ Wasserstoff bedeutet, als Ausgangsstoff, so wird dieses zweckmässigerweise in geschützter Form eingesetzt. Eine besonders geeignete Schutzgruppe ist dabei die Trichloracetylgruppe, welche nach erfolgter Umsetzung beispielsweise mit Kaliumcarbonat in Wasser durch Hydrolyse wieder entfernt werden kann.

Gemäss Verfahrensvariante l) können Verbindungen der Formel I hergestellt werden, worin Rd eine Gruppe der Formel $-(A^1)_m-CO-R^{14}$ und $R^{14}$ niederes Alkyl bedeuten, und $A^1$ und m obige Bedeutung besitzen. Als Lösungsmittel verwendet man vorzugsweise Aether, wie Tetrahydrofuran. Die Reaktion wird vorzugsweise in einem Temperaturbereich von -78°C bis Raumtemperatur durchgeführt.

Gemäss Verfahrensvariante m) können Verbindungen der Formel I hergestellt werden, worin $Q^1$ zusammen mit dem Stickstoffatom eine Gruppe der Formel $>N-CH=CH-$ (c) oder $>N-CH=CH-S(O)_p-$ (g) bedeutet, und p obige Bedeutung besitzt. Diese Dehydrohalogenierung wird vorzugsweise in einem inerten organischen Lösungsmittel, z.B. in einem halogenierten niederen Kohlenwasserstoff, wie Tetrachlorkohlenstoff, oder in Dimethylsulfoxid und Dimethylformamid, und in Gegenwart eines basischen Amins, z.B. eines tertiären Amins, wie Triäthylamin, oder eines bicyclischen Amidins, wie 1,5-Diazabicyclo[4.3.O.]-non-5-en, als Base durchgeführt. Man arbeitet zweckmässigerweise in einem Temperaturbereich von Raumtemperatur bis etwa 100°C.

Gemäss Verfahrensvariante n) können Verbindungen der Formel I hergestellt werden, worin $Q^1$ zusammen mit dem Stickstoffatom eine Gruppe der Formel $>N-CH_2-S(O)_t-$, $>N-CH_2CH_2-S(O)_t-$ oder $>N-CH=CH-S(O)_t-$, und t die Zahl 1 oder 2 bedeuten. Diese S-Oxidation wird vorzugsweise mit einem Oxidationsmittel, wie m-Chlorperbenzoesäure, in einem halogenierten Kohlenwasserstoff, wie Methylenchlorid, in einem Temperaturbereich von etwa -20°C bis etwa Raumtemperatur durchgeführt.

Gemäss Verfahrensvariante o) können Verbindungen der Formel I hergestellt werden, worin $Q^1$ zusammen mit dem Stickstoffatom eine Gruppe der Formel >N-CH=CH- (a) bedeutet. Diese Reaktion wird vorzugsweise in einem hochsiedenden aromatischen Kohlenwasserstoff, wie Xylol, bei der Rückflusstemperatur durchgeführt.

Gemäss Verfahrensvariante p) können Verbindungen der Formel I hergestellt werden, worin Rb und Rc zusammen mit dem mit α bezeichneten Kohlenstoffatom eine durch Halogen substituierte Gruppe der Formel $>C_\alpha-S-CH=CH-$ (h) oder $>C_\alpha-CH=CH-S-$ (i) und die gestrichelte Linie eine zusätzliche Bindung bedeuten. Als Halogenierungsmittel verwendet man vorzugsweise elementares Halogen, beispielsweise elementares Brom. Geeignete Lösungsmittel sind beispielsweise halogenierte Kohlenwasserstoffe, wie Chloroform. Man arbeitet zweckmässigerweise in einem Temperaturbereich von 0°C bis etwa Raumtemperatur.

Gemäss Verfahrensvariante q) können Verbindungen der Formel I hergestellt werden, worin Rd eine Gruppe der Formel $-(A^1)_m-R^{15}$ und $R^{15}$ einen über ein Kohlenstoffatom gebundenen, 5-gliedrigen, gegebenenfalls durch eine oder zwei niedere Alkylgruppen substituierten, partiell ungesättigten oder aromatischen Heterocyclus bedeuten, und $A^1$ und m obige Bedeutung besitzen. Die Umsetzung einer Verbindung der Formel VIIIc mit einer Verbindung der Formel XVI, XVII oder XVIII wird zweckmässigerweise in einem inerten Lösungsmittel, beispielsweise in einem halogenierten Kohlenwasserstoff, wie Methylenchlorid, oder in einem aromatischen Kohlenwasserstoff, wie Benzol, Toluol oder Xylol, und in einem Temperaturbereich von etwa 0°C bis zur Rückflusstemperatur des Reaktionsgemisches durchgeführt. Geeignete Basen sind beispielsweise die bereits früher erwähnten tertiären Amine. Die Cyclisierung des so erhaltenen Produktes kann nach an sich bekannten und jedem Fachmann geläufigen Methoden ... geführt werden. Die Cyclisierung kann beispielsweise in Gegenwart katalytischer Mengen ei... Säure wie p-Toluolsulfonsäure, unter Entfernung des gebildeten Reaktionswassers mittels ei... pers, wie Toluol, bewerkstelligt werden. Die Cyclisierung kann aber auch mittels Azodicarb... äthylester/Triphenylphosphin in einem Aether, wie Tetrahydrofuran, bewerkstelligt werden.

Gemäss Verfahrensvariante r) können Verbindungen der Formel I hergestellt werden, worin Rd eine Gruppe der Formel $-(A')_m-CH_2-R^{16}$, A' $C_{1-6}$-Alkyl und $R^{16}$ niederes Alkoxycarbonyl bedeuten, und m obige Bedeutung besitzt. Die Reaktion eines Diazoketons der Formel VIIId mit einem niederen Alkohol wird vorzugsweise in Gegenwart eines Silberkatalysators, wie Silberoxid, durchgeführt, wobei man als Lösungsmittel vorzugsweise den niederen Alkohol verwendet. Die Reaktion wird bei erhöhter Temperatur, vorzugsweise bei der Siedetemperatur des Reaktionsgemisches durchgeführt.

Gemäss Verfahrensvariante s) können Verbindungen der Formel I hergestellt werden, worin Rd Wasserstoff bedeutet. Die Decarboxylierung einer Carbonsäure der Formel Ia wird vorzugsweise durch trockenes Erhitzen, insbesondere durch trockenes Erhitzen im Vakuum auf Temperaturen von etwa 200° bis etwa 300°C bewerkstelligt.

Gemäss Verfahrensvariante t) können Verbindungen der Formel I hergestellt werden, worin Rd Halogen bedeutet. Für die vorliegende Halogenierung geeignete Halogenierungsmittel sind N-Halogenimide und -amide, wie N-Chlorsuccinimid, N-Bromsuccinimid, N-Chloracetamid und dergleichen. Als Lösungsmittel verwendet man vorzugsweise einen halogenierten Kohlenwasserstoff, wie Methylenchlorid, Chloroform, Tetrachlorkohlenstoff und dergleichen. Die Reaktion kann in einem Temperaturbereich von etwa 0°C bis zur Siedetemperatur des Reaktionsgemisches durchgeführt werden. Vorzugsweise arbeitet man bei Raumtemperatur.

Durch Spaltung der Acetalgruppe in einer Verbindung der Formel VIIIe gemäss Verfahrensvariante u) können Verbindungen der Formel I hergestellt werden, worin Rd die Gruppe -CHO bedeutet. Die Spaltung wird vorzugsweise durch Umacetalisieren in Gegenwart einer Säure, wie p-Toluolsulfonsäure, und eines Ketons, wie Cyclohexanon, Aceton und dergleichen bewerkstelligt. Die Reaktion kann in einem Temperaturbereich von Raumtemperatur bis zur Siedetemperatur des Reaktionsgemisches durchgeführt werden.

Gemäss Verfahrensvariante v) können Verbindungen der Formel I hergestellt werden, worin $Q^1$ zusammen mit dem Stickstoffatom die Gruppe $>N-CH_2CH_2-$ bedeutet. Die Hydrierung wird zweckmässigerweise in Gegenwart eines Edelmetallkatalysators, wie Platinoxid und Palladium/Kohle, in einem für derartige Zwecke geeigneten Lösungsmittel, z.B. in einem niederen Alkohol oder in einem niederen Fettsäureester, wie Essigester, durchgeführt. Man arbeitet vorzugsweise bei Raumtemperatur.

Gemäss Verfahrensvariante w) können Verbindungen der Formel I hergestellt werden, worin $R^1$ eine Gruppe der Formel $-(A^1)_m-OCO-NR^3R^4$ bedeutet. Für den vorliegenden Zweck geeignete Lösungsmittel

sind beispielsweise Aether, wie Tetrahydrofuran, Dioxan und Diäthyläther, N,N-Dimethylformamid und Dimethylsulfoxid. Die Reaktion wird zweckmässigerweise bei Raumtemperatur durchgeführt.

Gemäss Verfahrensvariante x) können Verbindungen der Formel I, welche einen oder mehrere basische Substituenten besitzen, in pharmazeutisch annehmbare Säureadditionssalze übergeführt werden. Derartige Säureadditionssalze können nach an sich bekannten und jedem Fachmann geläufigen Methoden hergestellt werden. Es kommen dabei sowohl Salze mit anorganischen als auch Salze mit organischen Säuren in Betracht, beispielsweise Hydrochloride, Hydrobromide, Sulfate, Nitrate, Citrate, Acetate, Maleate, Succinate, Methansulfonate, p-Toluolsulfonate und dergleichen.

Die als Ausgangsstoffe verwendeten Verbindungen der Formeln II, VIIIa, VIIIb, VIIIc, VIIId, VIIIe, VIIIf, XVa und XVb sind neu und ebenfalls Gegenstand der vorliegenden Erfindung. Diese Stoffe können wie nachfolgend beschrieben hergestellt werden.

Die Verbindungen der Formel II können beispielsweise dadurch hergestellt werden, dass man eine Verbindung der allgemeinen Formel

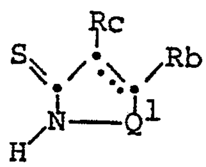

XXI

worin $Q^1$, Rb, Rc und die gestrichelte Linie obige Bedeutung besitzen, mit einer Verbindung der allgemeinen Formel

$$Ra\text{-}CHX^2\text{-}COX^1$$

XXII oder

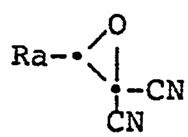

XXIII

worin $X^1$ und $X^2$ je Halogen bedeuten und $R^1$ obige Bedeutung besitzt, umsetzt. Die Reaktion einer Verbindung der Formel XXI mit einer Verbindung der Formel XXII, worin $X^1$ vorzugsweise Chlor und $X^2$ vorzugsweise Brom bedeuten, wird vorzugsweise bei Raumtemperatur in einem halogenierten Kohlenwasserstoff, wie Chloroform, durchgeführt, worauf man mit einem basischen Amin, wie Triäthylamin, behandelt. Die Reaktion einer Verbindung der Formel XXI mit einer Verbindung der Formel XXIII wird vorzugsweise in einem inerten Lösungsmittel, wie Aceton, N,N-Dimethylformamid, Dimethylsulfoxid und dergleichen, bei Raumtemperatur durchgeführt.

Die Carbonsäuren der Formel VIIIa können durch Hydrolyse der entsprechenden niederen Alkylester der Formel I hergestellt werden. Diese Hydrolyse kann nach an sich bekannten Methoden, beispielsweise in Analogie zu Verfahrensvariante c), durchgeführt werden.

Die Isocyanate der Formel VIIIb können hergestellt werden, indem man eine Verbindung der Formel Ib, worin $Q^2$ die Gruppe der Formel -NH- bedeutet, in einem inerten Lösungsmittel mit Phosgen behandelt. Geeignete Lösungsmittel sind beispielsweise halogenierte Kohlenwasserstoffe, wie Chloroform und 1,2-Dichloräthan. Die Isocyanate der Formel VIIIb können aber auch hergestellt werden, indem man ein Carbonsäurehalogenid der Formel VIIIc in einem inerten organischen Lösungsmittel mit einem Azid, wie Natriumazid oder Trimethylsilylazid, in das entsprechende Carbonsäureazid überführt und dieses durch Erwärmen zum entsprechenden Isocyanat umlagert. Geeignete Lösungsmittel sind beispielsweise Aether, wie Dioxan, Aethylenglykoldimethyläther, Diäthylenglykoldimethyläther und dergleichen, Ketone, wie Aethylmethylketon, und dergleichen. Die Umlagerung wird bei Temperaturen von 80°C und darüber bewerkstelligt.

Die Carbonsäurehalogenide der Formel VIIIc können hergestellt werden, indem man eine Carbonsäure der Formel Ia mit einem Halogenierungsmittel behandelt. Geeignete Halogenierungsmittel sind beispielsweise Thionylchlorid, Oxalylchlorid, Phosphorpentachlorid und dergleichen. In einer bevorzugten Ausführungsform verwendet man überschüssiges Thionylchlorid und arbeitet ohne zusätzliches Lösungsmittel bei Raumtemperatur.

Die Diazoketone der Formel VIIId können hergestellt werden, indem man ein Carbonsäurehalogenid der Formel VIIIc in einem inerten organischen Lösungsmittel mit Diazomethan umsetzt. Geeignete Lösungsmittel sind beispielsweise Aether, wie Tetrahydrofuran, Dioxan und Diäthyläther. Die Reaktion wird vorzugsweise in einem Temperaturbereich von etwa 0° bis etwa 10°C durchgeführt.

Die Verbindungen der Formel VIIIe können in Analogie zur Verfahrensvariante a) hergestellt werden, wobei man als Ausgangsstoff eine Verbindung der allgemeinen Formel $HC{\equiv}C\text{-}CH(OR^7)OR^8$ verwendet, worin $R^7$ und $R^8$ obige Bedeutung besitzen.

Die Verbindungen der Formel VIIIf können hergestellt werden, indem man eine Verbindung der Formel

Ib' in einem inerten Lösungsmittel, beispielsweise in einem Aether, wie Dioxan, und in Gegenwart einer Base, beispielsweise eines basischen Amins, wie Pyridin, mit Chlorameisensäurephenylester umsetzt.

Die Verbindungen der Formel XVa können hergestellt werden, indem man eine Verbindung der Formel I, worin $Q^1$ zusammen mit dem Stickstoffatom die Gruppe $>N-CH_2CH_2-$ bedeutet, mit einem N-Halogensuccinimid, wie N-Bromsuccinimid, behandelt. Die Verbindung der Formel XVa wird vorzugsweise nicht isoliert, sondern direkt dehydrohalogeniert, wobei man als Base vorzugsweise Triäthylamin verwendet.

Die Verbindungen der Formel XVb können hergestellt werden, indem man eine Verbindung der Formel I, worin $Q^1$ zusammen mit dem Stickstoffatom eine Gruppe der Formel $>NCH_2CH_2-SO-$ bedeutet, ohne zusätzliches Lösungsmittel mit einem Halogenierungsmittel, wie Thionylchlorid, behandelt.

Die übrigen als Ausgangsstoffe verwendeten Verbindungen gehören an sich bekannten Stoffklassen an. Die weiter unten folgenden Beispiele enthalten detaillierte Angaben betreffend die Herstellung sämtlicher Ausgangsstoffe. Wie bereits eingangs erwähnt besitzen die Verbindungen der Formel I wertvolle pharmakologische Eigenschaften. Sie entfalten insbesondere ausgeprägte muskelrelaxierende, sedativ-hypnotische, antikonvulsive und/oder anxiolytische Eigenschaften und weisen eine nur geringe Toxizität auf. Diese Eigenschaften können beispielsweise im nachfolgend beschriebenen und für die Erfassung derartiger Eigenschaften allgemein anerkannten Antipentetrazoltest nachgewiesen werden.

In diesem Tierversuch verabreicht man weiblichen Ratten die zu testende Verbindung oral oder intravenös und 30 Minuten später 120 mg/kg Pentetrazol intraperitoneal, das in ungeschützten Versuchstieren 1-4 Minuten nach der Injektion Emprosthotonus und tonische Streckungen der vorderen und/oder hinteren Gliedmassen bewirkt. Man verwendet 10 Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere vor den durch Pentetrazol bewirkten krampfartigen Anfällen schützt. In der nachfolgenden Tabelle werden die Resultate zusammengestellt, welche mit repräsentativen Vertretern der durch die allgemeine Formel I definierten Verbindungsklasse in dem vorstehend geschilderten Versuch erhalten worden sind. Die Tabelle enthält ausserdem Angaben über die akute Toxizität ($DL_{50}$) einiger dieser Verbindungen in mg/kg bei einmaliger oraler Verabreichung an Mäusen.

Tabelle

| Verbindung | $ED_{50}$ in mg/kg p.o. | $DL_{50}$ in mg/kg p.o. |
|---|---|---|
| A | 2,6 | >5000 |
| B | 5,4 | >5000 |
| C | 15,9 | - |
| D | 1,2 | >5000 |
| E | 3,5 | 625 |
| F | 2,8 | 5000 |
| G | 0,12 | 2500 |
| H | 1,3 | >3000 |
| I | 5,9 | >5000 |
| K | 0,56 | >5000 |
| L | 0,22 | >5000 |
| M | 5,0 | 5000 |
| N | 0,97 | 625 |
| O | 0,49 | 5000 |
| P | 0,31 | >5000 |
| Q | 3,1 | >5000 |
| R | 0,17 | 1250 |
| S | 0,87 | 5000 |
| T | 3,8 | >5000 |
| U | 2,7 | >5000 |
| V | 0,13 | >5000 |
| W | 0,51 | >3000 |
| X | 1,1 | 5000 |
| Y | 0,41 | 5000 |

A = 10-Chlor-6,7-dihydro-N-(2-methoxyäthyl)-4-oxo-3-phenyl-4H-benzo[a]chinolizin-1-carboxamid.
B = 1-[(10-Chlor-6,7-dihydro-4-oxo-3-phenyl-4H-benzo[a]chinolizin-1-yl)carbonyl]-4-piperidinol.
C = (4a,5-Dihydro-7-oxo-8-phenyl-7H-thieno[2,3-a]chinolizin-10-yl)methyl-4-morpholincarboxylat.
D = 4-[(6,7-Dihydro-4-oxo-3-phenyl-10-chlor-4H-benzo[a]chinolizin-1-yl)carbonyl]-2,6-dimethylmorpholin.
E = (S)-1-[(7-Oxo-8-phenyl-7H-thieno[2,3-a]chinolizin-10-yl)carbonyl]-2-pyrrolidinmethanol.
F = (S)-2-Methoxymethyl-1-[(7-oxo-8-phenyl-7H-thieno[2,3-a]chinolizin-10-yl)carbonyl]pyrrolidin.
G = 1-[(10-Chlor-4-oxo-3-phenyl-4H-benzo[a]chinolizin-1-yl)carbonyl]-3-methoxypyrrolidin.

H = (S)-1-[(10-Chlor-4-oxo-3-phenyl-4H-benzo[a]chinolizin-1-yl)carbonyl]-2-pyrrolidinmethanol.
I = cis-4-[(4,5-Dihydro-7-oxo-8-phenyl-7H-thieno[2,3-a]chinolizin-10-yl)carbonyl]-2,6-dimethylmorpholin.
K = 1-[(10-Chlor-6,7-dihydro-4-oxo-3-phenyl-4H-benzo[a]chinolizin-1-yl)carbonyl]-4-methoxypiperidin.
L = 1-[(10-Chlor-3-phenyl-4-oxo-4H-benzo[a]chinolizin-1-yl)carbonyl]-4-methoxypiperidin.
M = N-Äthyl-N-(2-methoxyäthyl)-7-oxo-8-phenyl-7H-thieno[2,3-a]chinolizin-10-carboxamid.
N = N-(2-Methoxyäthyl)-N-methyl-7-oxo-8-phenyl-7H-thieno[2,3-a]chinolizin-10-carboxamid.
O = (R)-2-(Methoxymethyl)-1-[(7-oxo-8-phenyl-7H-thieno[2,3-a]chinolizin-10-yl)carbonyl]pyrrolidin.
P = 1-[(10-Chlor-6,7-dihydro-4-oxo-3-phenyl-4H-benzo[a]chinolizin-1-yl)carbonyl]-3-methoxypyrrolidin.
Q = 1-[(10-Chlor-6,7-dihydro-4-oxo-3-phenyl-4H-benzo[a]chinolizin-1-yl)carbonyl]-3-äthoxypyrrolidin.
R = (R)-3-Methoxy-1-[(7-oxo-8-phenyl-7H-thieno[2,3-a]chinolizin-10-yl)carbonyl]pyrrolidin.
S = (S)-3-Methoxy-1-[(7-oxo-8-phenyl-7H-thieno[2,3-a]chinolizin-10-yl)carbonyl]pyrrolidin.
T = (S)-1-[(10-Chlor-6,7-dihydro-4-oxo-3-phenyl-4H-benzo[a]chinolizin-1-yl)carbonyl]-2-(methoxymethyl)pyrrolidin.
U = 1-[(4,5-Dihydro-7-oxo-8-phenyl-7H-thieno[2,3-a]chinolizin-10-yl)carbonyl]-3-methoxypyrrolidin.
V = 3-Methoxy-1-[(7-oxo-8-phenyl-7H-thieno[2,3-a]chinolizin-10-yl)carbonyl]azetidin.
W = (R)-1-[(10-Chlor-4-oxo-3-phenyl-4H-benzo[a]chinolizin-1-yl)carbonyl]-2-pyrrolidinmethanol.
X = (S)-1-[(10-Chlor-4-oxo-3-phenyl-4H-benzo[a]chinolizin-1-yl)carbonyl]-2-(methoxymethyl)pyrrolidin.
Y = (R)-1-[(10-Chlor-4-oxo-3-phenyl-4H-benzo[a]chinolizin-1-yl)carbonyl]-2-(methoxymethyl)pyrrolidin.

Die Verbindungen der Formel I und die pharmazeutisch annehmbaren Säureadditionssalze von Verbindungen der Formel I mit einem basischen Substituenten können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten. Arzneimittel enthaltend ein erfindungsgemäßes Produkt und einen therapeutisch inerten Träger, sowie ein Verfahren zu deren Herstellung, das dadurch gekennzeichnet ist, daß man ein erfindungsgemäßes Produkt und gegebenenfalls einen oder mehrere andere therapeutisch wertvolle Stoffe in eine galenische Darreichungsform bringt, sind ebenfalls Gegenstand der vorliegenden Erfindung.

Die erfindungsgemäßen Produkte können, wie bereits erwähnt, bei der Bekämpfung oder Verhütung von Krankheiten, insbesondere bei der Bekämpfung von Konvulsionen und Angstzuständen, sowie für die Herstellung von Arzneimitteln mit muskelrelaxierenden, sedativ-hypnotischen, antikonvulsiven und/oder anxiolytischen Eigenschaften verwendet werden. Die Dosierung kann dabei innerhalb weiter Grenzen variieren und ist natürlich in jedem einzelnen Fall den individuellen Gegebenheiten anzupassen. Bei oraler Verabreichung liegt die tägliche Dosis in einem Bereich von etwa 1 mg bis etwa 100 mg.

Die nachfolgenden Beispiele dienen der näheren Erläuterung der vorliegenden Erfindung. Sämtliche Temperaturen sind in Celsiusgraden angegeben.

Beispiel 1

aa) Methode A: Zu einer Lösung von 23,3 g 4,5-Dihydrothieno[2,3-c]pyridin-7(6H)-thion in 630 ml Chloroform gibt man unter Rühren tropfenweise 29,4 g α-Bromphenylessigsäurechlorid, wobei man dafür sorgt, daß die Temperatur nicht über 25° steigt. Nach 30 Minuten versetzt man mit 25,45g Triäthylamin und rührt noch während 3 Stunden. Man verdünnt die Mischung mit Wasser, trennt die organische

Phase ab, wäscht mit gesättigter wässriger Natriumchloridlösung, trocknet über Magnesiumsulfat, dampft ein und chromatographiert den Rückstand an Kieselgel (Laufmittel Toluol/Aethanol 95:5). Man erhält 5,6-Dihydro-3-hydroxy -2-phenylthiazolo[3,2-a]thieno[2,3-c]pyridinium-hydroxid (inneres Salz) vom Smp. 225° (Zers.) (aus Dioxan/Aceto- nitril).

Methode B: Zu einer Lösung von 26,35 g 4,5-Dihydro- thieno[2,3-c]pyridin-7(6H)-thion in 140 ml Dimethylformamid gibt man unter Rühren 28,6 g 1-Phenyl-2,2-dicyan-oxiran, wobei sich die Lösung sofort tiefrot färbt. Nach 16 Stunden wird der kristalline Niederschlag abgesaugt und mit Essigester gewaschen. Man erhält 5,6-Dihydro-3-hydroxy-2- phenylthiazolo[3,2-a]thieno[2,3-c]pyridinium-hydroxid (inneres Salz) vom Smp. 208-209°.

In analoger Weise erhält man:

ab) Aus 4,5-Dihydrothieno[2,3-c]pyridin-7(6H)-thion und 1-(o-Chlorphenyl)-2,2-dicyan-oxiran das 5,6-Dihydro-3-hydroxy -2-(o-chlorphenyl)-thiazolo[3,2-a]thieno[2,3-c]pyridinium -hydroxid (inneres Salz) vom Smp. 174-175° (aus Dioxan/Acetonitril);

ac) aus 4,5-Dihydrothieno[2,3-c]pyridin-7(6H)-thion und 1-(m-Chlorphenyl)-2,2-dicyan-oxiran das 5,6-Dihydro-3-hydroxy -2-(m-chlorphenyl)-thiazolo[3,2-a]thieno[2,3-c]pyridinium -hydroxid (inneres Salz) vom Smp. 179-181°;

ad) aus 4,5-Dihydrothieno[2,3-c]pyridin-7(6H)-thion und 1-p-Chlorphenyl)-2,2-dicyan-oxiran das 5,6-Dihydro-3-hydroxy -2-(p-chlorphenyl)-thiazolo[3,2-a]thieno[2,3-c]pyridinium -hydroxid (inneres Salz) vom Smp. 215° (Zers.);

ae) aus 4,5-Dihydrothieno[2,3-c]pyridin-7(6H)-thion und 1-(m-Fluorphenyl)-2,2-dicyan-oxiran das 5,6-Dihydro-3-hydroxy-2-(m-fluorphenyl)-thiazolo[3,2-a]thieno[2,3-c]pyridinium-hydroxid (inneres Salz);

af) aus 6,7-Dihydrothieno[3,2-c]pyridin-7(6H)-thion mit 1-Phenyl-2,2-dicyan-oxiran das 5,6-Dihydro-3-hydroxy-2-phenylthiazolo[3,2-a]thieno[3,2-c]pyridinium-hydroxid (inneres Salz) vom Smp. 198–202° (Zers.).

ba) Man versetzt eine Suspension von 31,65 g 5,6-Dihydro-3-hydroxy-2-phenylthiazolo[3,2-a]thieno[2,3-c]pyridinium-hydroxid (inneres Salz) in 770 ml Toluol mit 10,94 g Propiolsäure-methylester, erhitzt bis zur Beendigung der Reaktion unter Rückfluß, dampft das Reaktionsgemisch im Vakuum ein und chromatographiert den Rückstand an Kieselgel mit Toluol/Essigsäureäthylester (9:1). Man erhält 4,5-Dihydro-7-oxo-8-phenyl-7H-thieno[2,3-a]chinolizin-10-carbonsäure-methylester als gelbe Kristalle vom Smp. 116° (aus Essigsäureäthylester).

In analoger Weise erhält man:

bb) Aus 5,6-Dihydro-3-hydroxy-2-phenylthiazolo[3,2-a]thieno[2,3-c]pyridinium-hydroxid (inneres Salz) und Propiolsäureäthylester den 4,5-Dihydro-7-oxo-8-phenyl-7H-thieno[2,3-a]chinolizin-10-carbonsäure-äthylester vom Smp. 126–127° (aus Äthanol);

bc) aus 5,6-Dihydro-3-hydroxy-2-(o-chlorphenyl)-thiazolo[3,2-a]thieno[2,3-c]pyridinium-hydroxid (inneres Salz) und Propiolsäure-methylester den 8-(o-Chlorphenyl)-4,5-dihydro-7-oxo-7H-thieno[2,3-a]chinolizin-10-carbonsäure-methylester vom Smp. 142,5–143,5° (aus Essigsäureäthylester);

bd) aus 5,6-Dihydro-3-hydroxy-2-(m-chlorphenyl)-thiazolo[3,2-a]thieno[2,3-c]pyridinium-hydroxid (inneres Salz) und Propiolsäuremethylester den 8-(m-Chlorphenyl)-4,5-dihydro-7-oxo-7H-thieno[2,3-a]chinolizin-10-carbonsäure-methylester vom Smp. 133–134° (aus Acetonitril);

be) aus 5,6-Dihydro-3-hydroxy-2-(p-chlorphenyl)-thiazolo[3,2-a]thieno[2,3-c]pyridinium-hydroxid (inneres Salz) und Propiolsäuremethylester den 8-(p-Chlorphenyl)-4,5-dihydro-7-oxo-7H-thieno[2,3-a]chinolizin-10-carbonsäure-methylester vom Smp. 174–175,5° (aus Essigsäureäthylester);

bf) aus 5,6-Dihydro-3-hydroxy-2-(m-fluorphenyl)-thiazolo[3,2-a]thieno[2,3-c]pyridinium-hydroxid (inneres Salz) und Propiolsäuremethylester den 8-(m-Fluorphenyl)-4,5-dihydro-7-oxo-7H-thieno[2,3-a]-chinolizin-10-carbonsäure-methylester vom Smp. 132–134° (aus Essigsäureäthylester/Diisopropyläther);

bg) aus 5,6-Dihydro-3-hydroxy-2-phenylthiazolo[3,2-a]thieno[3,2-c]pyridinium-hydroxid (inneres Salz) und Propiolsäuremethylester den 4,5-Dihydro-7-oxo-8-phenyl-7H-thieno[3,2-a]chinolizin-10-carbonsäure-methylester vom Smp. 172,5–177° (aus Essigsäureäthylester).

Beispiel 2

aa) Zu einer Lösung von 0,4 g Natriumhydroxid in 70 ml Methanol gibt man 2,53 g 4,5-Dihydro-7-oxo-8-phenyl-7H -thieno[2,3-a]chinolizin-10-carbonsäure-methylester und erhitzt das Gemisch unter Rückfluss, bis die Verseifung beendet ist. Nach Abdampfen des Lösungsmittels im Vakuum, wird der Rückstand in Wasser aufgenommen und mit Chloroform extrahiert. Die wässrige Phase wird mit Aktivkohle behandelt und dann mit 1N-Salzsäure angesäuert, wobei reine 4,5-Dihydro-7-oxo-8-phenyl-7H -thieno[2,3-a]chinolizin-10-carbonsäure vom Smp. 210-210,5° (Zers.) ausfällt.

In analoger Weise erhält man:

ab) Aus 8-(m-Fluorphenyl)-4,5-dihydro-7-oxo-7H -thieno-[2,3-a]chinolizin-10-carbonsäure-methylester die 8-(m-Fluorphenyl)-4,5-dihydro-7-oxo-7H -thieno[2,3-a]chinolizin-10-carbonsäure vom Smp. 233-235°;

ac) aus Methyl 4,5-Dihydro-7-oxo-8-phenyl- 7H -thieno[3,2-a]chinolizin-10-carboxylat die 4,5-Dihydro-7-oxo-8-phenyl-7H-thieno[3,2-a]chinolizin-10-carbonsäure vom Smp. 244-247° (Zers.).

ba) Zu 7,5 ml Thionylchlorid gibt man unter Rühren portionenweise 2,5 g 4,5-Dihydro-7-oxo-8-phenyl-7H -thieno[2,3-a]chinolizin-10-carbonsäure, rührt das Gemisch während 1 Stunde bei Raumtemperatur, entfernt das überschüssige Thionylchlorid im Vakuum und löst den Rückstand in 25 ml Toluol auf. Man gibt unter Rühren 1 ml Triäthylamin hinzu und versetzt anschliessend mit 0,9 ml 2-Dimethylaminoäthylamin. Das Reaktionsgemisch wird während 2 Stunden bei Raumtemperatur gerührt, dann mit gesättigter wässriger Natriumbicarbonatlösung versetzt und dreimal mit Methylenchlorid extrahiert. Die organische Phase wird über Natriumsulfat getrocknet und eingedampft. Aus dem erhaltenen Material wird mittels methanolischer Salzsäure das Hydrochlorid hergestellt. Durch Umkristallisieren aus Methanol/Diäthyläther erhält man N-[2-(Dimethylamino)äthyl]-4,5-dihydro-7-oxo-8-phenyl-7H -thieno[2,3-a]chinolizin-10-carboxamid-hydrochlorid als gelbe Kristalle vom Smp. 260-261°.

In analoger Weise erhält man:

bb) Aus 4,5-Dihydro-7-oxo-8-phenyl-7H -thieno[2,3-a]chinolizin-10-carbonsäure und 2-Dimethylaminoäthanol das 4,5- Dihydro-7-oxo-8-phenyl-7H -thieno[2,3-a]chinolizin-10- carbonsäure -2-(dimethylamino)äthylester-hydrochlorid vom Smp. 206-207° (aus Aethanol/Diäthyläther);

bc) aus 4,5-Dihydro-7-oxo-8-phenyl-7H -thieno[2,3-a]chinolizin-10-carbonsäure und Piperidin-4-carbonsäureäthylester den 1-[[4,5-Dihydro-7-oxo-8-phenyl- 7H -thieno[2,3-a]chinolizin-10-yl]carbonyl] -4- piperidincarbonsäureäthylester als amorphes Material;

bd) aus 4,5-Dihydro-7-oxo-8-phenyl-7H -thieno[2,3-a]chinolizin-10-carbonsäure und 1-Acetylpiperazin das 1-Acetyl-4- [[4,5-dihydro-7-oxo-8-phenyl-7H -thieno[2,3-a]chinolizin-10- yl]carbonyl-piperazin vom Smp. 226-227° (aus Dioxan);

be) aus 4,5-Dihydro-7-oxo-8-phenyl-7H -thieno[2,3-a]chinolizin-10-carbonsäure und 1-Methylpiperazin das 1-[(4,5- Dihydro-7-oxo-8-phenyl-7H -thieno[2,3-a]chinolizin-10-yl]carbonyl] -4-methylpiperazin-hydrochlorid vom Smp. 280° (aus Methanol/Diäthyläther);

bf) aus 4,5-Dihydro-7-oxo-8-phenyl-7H -thieno[2,3-a]chinolizin-10-carbonsäure und Morpholin das 4-[(4,5-Dihydro-7- oxo-8-(phenyl-7H -thieno[2,3-a]chinolizin-10-yl)carbonyl]- morpholin vom Smp. 212-214° (aus Dioxan/Diäthyläther);

bg) aus 4,5-Dihydro-7-oxo-8-phenyl-7H -thieno[2,3-a]chinolizin-10-carbonsäure und 4-Hydroxypiperidin das 1-[[4,5- Dihydro-8-phenyl-7-oxo-7)H -thieno[2,3-a]chinolizin-10-yl]carbonyl]-4-piperidinol vom Smp. 140-142° (aus Acetonitril/Diäthyläther);

bh) aus 4,5-Dihydro-7-oxo-8-phenyl-7H -thieno[2,3-a] chinolizin-10-carbonsäure und Piperazin das 1-[(4,5-Dihydro-7- oxo-8-phenyl-7H -thieno[2,3-a]chinolizin-10-yl)carbonyl]piperazin -hydrochlorid vom Smp. 260-271° (aus Aethanol/Diäthyläther)

bi) aus 4,5-Dihydro-7-oxo-8-phenyl-7H -thieno[2,3-a]chinolizin-10-carbonsäure und 1,1-Diäthylaminoäthylamin das N-[2-(Diäthylamino)äthyl]-4,5-dihydro-7-oxo-8-phenyl-7H -thieno[2,3-a]chinolizin-10-carboxamid-hydrochlorid als amorphes Material;

bj) aus 4,5-Dihydro-7-oxo-8-phenyl-7H -thieno[2,3-a]chinolizin-10-carbonsäure und Thiomorpholin das 4-[(4,5-Dihydro- 7-oxo-8-phenyl-7H -thieno[2,3-a]chinolizin-10-yl)carbonyl]- tetrahydro-2H -1,4-thiazin vom Smp. 224-226° (aus Aethanol/Diäthyläther);

bk) aus 4,5-Dihydro-7-oxo-8-phenyl-7H -thieno[2,3-a]chinolizin-10-carbonsäure und 2-Methylaminoäthanol das 4,5- Dihydro-N-(2-hydroxyäthyl)-N-methyl-7-oxo-8-phenyl-7H -thieno[2,3-a]chinolizin-10-carboxamid vom Smp. 208-210° (aus Acetonitril);

bl) aus 4,5-Dihydro-7-oxo-8-phenyl-7H -thieno[2,3-a]chinolizin-10-carbonsäure und Glycinäthylester den N-[[4,5- Dihydro-7-oxo-8-phenyl-7H -thieno[2,3-a]chinolizin-10-yl]carbonyl]glycin-äthylester vom Smp. 211-213° (aus Essigsäureäthylester/Diäthyläther);

bm) aus 4,5-Dihydro-7-oxo-8-phenyl-7H -thieno[2,3-a]chinolizin-10-carbonsäure und 3-Dimethylamino-1-propylamin das N- [3-(Dimethylamino)propyl]-4,5-dihydro-8-phenyl-7H -thieno[2,3- a]chinolizin-10-carboxamid-hydrochlorid vom Smp. 261-262° (aus Methanol/Diäthyläther);

bn) aus 4,5-Dihydro-7-oxo-8-phenyl-7H -thieno[2,3-a]chinolizin-10-carbonsäure und 2-Aminoäthanol das 4,5-Dihydro-N- (2-hydroxyäthyl)-8-phenyl-7-oxo-7H -thieno[2,3-a]chinolizin-10-carboxamid vom Smp. 228-230° (aus Chloroform/Hexan);

bo) aus 4,5-Dihydro-7-oxo-8-phenyl-7H -thieno[2,3-a]chinolizin-10-carbonsäure und konzentrierter wässriger Ammoniaklösung das 4,5-Dihydro-7-oxo-8-phenyl-7H -thieno[2,3-a]chinolizin-10-carboxamid vom Smp. 281-281,5° (aus Aethanol);

bp) aus 4,5-Dihydro-7-oxo-8-(m-fluorphenyl)-7H -thieno[2,3-a]chinolizin-10-carbonsäure und Morpholin das 4-[[8-(m- Fluorphenyl)-4,5-dihydro-7-oxo-7H -thieno[2,3-a]chinolizin-10- yl]carbonyl]morpholin vom Smp. 213-214° (aus Methanol);

bq) aus 4,5-Dihydro-7-oxo-8-phenyl-7H -thieno[3,2- a]chinolizin-10-carbonsäure und 2-Aminoäthanol das 4,5-Dihydro-N- (2-hydroxyäthyl)-7-oxo-8-phenyl-7H -thieno[3,2-a]chinolizin-10-carboxamid vom Smp. 202-204° (aus Chloroform/Hexan);

br) aus 2-Chlor-4,5-dihydro-7-oxo-8-phenyl-7H-thieno[2,3- a] chinolizin-10-carbonsäure und Morpholin das 4-[(2-Chlor- 4,5-dihydro-7-oxo-8-phenyl-7H-thieno[2,3-a]chinolizin-10-yl)carbonyl]morpholin vom Smp. 190-192°;

bs) aus 4,5-Dihydro-7-oxo-8-phenyl-7H-thieno[2,3-a]chinolizin-10-carbonsäure und cis-2,6-Dime-

thylmorpholin das cis-4- [(4,5-Dihydro-7-oxo-8-phenyl-7H-thieno[2,3-a]chinolizin-10-yl)carbonyl]-2,6-dimethylmorpholin vom Smp. 221-223°.

Beispiel 3

a) Zu einer Suspension von 2,0 g 4,5-Dihydro-7-oxo-8- phenyl-7H -thieno[2,3-a]chinolizin-10-carbonsäure und 3,8 g N-Methyl-2-chlorpyridiniumjodid in 12,5 ml Methylenchlorid gibt man 7,1 ml Tri-n-butylamin und 6,1 ml Isopropanol, rührt während 24 Stunden unter Rückfluss, dampft das Reaktionsgemisch im Vakuum ein und chromatographiert den Rückstand mit Toluol/Essigsäureäthylester (9:1) an Kieselgel. Nach Kristallisieren aus Methanol erhält man reinen 4,5-Dihydro-7-oxo-8-phenyl-7H -thieno[2,3-a]chinolizin- 10-carbonsäure-isopropylester vom Smp. 165,5-166°.

In analoger Weise erhält man:

b) Aus 4,5-Dihydro-7-oxo-8-phenyl-7H -thieno[2,3- a]chinolizin-10-carbonsäure, N-Methyl-2-chlorpyridiniumjodid, Tri-n- butylamin und Benzylalkohol den 4,5-Dihydro-8-phenyl-7-oxo-7H -thieno[2,3-a]chinolizin-10-carbonsäure-benzylester vom Smp. 159-160° (aus Acetonitril);

c) aus 4,5-Dihydro-7-oxo-8-phenyl-7H -thieno[2,3-a]chinolizin-10-carbonsäure, N-Methyl-2-chlorpyridiniumjodid, Tri-n- butylamin und Diäthylamin das N,N-Diäthyl-4,5-dihydro-7- oxo-8-phenyl-7H -thieno[2,3-a]chinolizin-10-carboxamid vom Smp. 175-175,5° (aus Isopropanol);

d) aus 4,5-Dihydro-7-oxo-8-phenyl-7H -thieno[2,3- a]chinolizin-10-carbonsäure, N-Methyl-2-chlorpyridiniumjodid, Tri-n- butylamin und Cyclohexylamin das N-Cyclohexyl-4,5-dihydro-7- oxo-8-phenyl-7H -thieno[2,3-a]chinolizin-10-carboxamid vom Smp. 284-284,5° (aus Dioxan).

Beispiel 4

a) Man gibt 10,82 g 4,5-Dihydro-7-oxo-8-phenyl-7H -thieno[2,3-a]chinolizin-10-carbonsäure zu 67 ml Thionylchlorid, rührt während 1 Stunde bei Raumtemperatur, destilliert das überschüssige Thionylchlorid im Vakuum ab, nimmt das erhaltene Säurechlorid in 67 ml Tetrahydrofuran auf und tropft diese Lösung innerhalb von etwa 30 Minuten zu einer Suspension von 2,55 g Natriumborhydrid in 67 ml Dimethylformamid. Nach 3 Stunden wird zunächst unter Kühlen mit 2N-Salzsäure angesäuert und dann kurz zum Sieden erhitzt. Man stellt mit 2N-Natronlauge alkalisch und extrahiert mit Chloroform/Methanol (9:1). Die organische Phase wird mit gesättigter wässriger Kochsalzlösung gewaschen, über Magnesiumsulfat getrocknet und im Vakuum eingedampft. Man erhält nach Umkristallisieren aus Methanol reines 4,5-Dihydro- 10-(hydroxymethyl)-8-phenyl-7H -thieno[2,3-a]chinolizin-7-on vom Smp. 199-200°.

In analoger Weise erhält man:

b) Aus 10-Chlor-6,7-dihydro-4-oxo-3-phenyl -4H- benzo[a]chinolizin-1-carbonsäure das 6,7-Dihydro-1-(hydroxymethyl)- 3-phenyl-10-chlor -4H-benzo[a]chinolizin-4-on vom Smp. 248-249°;

c) aus 6,7-Dihydro-4-oxo-3-phenyl -4H-benzo[a]chinolizin- 1-carbonsäure das 6,7-Dihydro-1-(hydroxymethyl)-3-phenyl -4H- benzo[a]chinolizin-4-on vom Smp. 175-177°;

d) aus 1-[(10-Chlor-6,7-dihydro-4-oxo-3-phenyl-4H- benzo[a]chinolizin-1-yl)carbonyl]-4-piperidincarbonsäure das 1-[(10-Chlor-6,7-dihydro-4-oxo-3-phenyl-4H-benzo[a]chinolizin-1-yl)carbonyl]-4-piperidinmethanol vom Smp. 217-218°;

e) aus 7-Oxo-8-phenyl-7H-thieno[2,3-a]chinolizin -10- carbonsäure das 10-(Hydroxymethyl)-8-phenyl-7H-thieno[2,3- a]chinolizin-7-on vom Smp. 209-216°.

Beispiel 5

a) Eine Suspension von 1,55 g 4,5-Dihydro-10-(hydroxymethyl)-8-phenyl-7H -thieno[2,3-a]chinolizin-7-on in 6,7 ml Pyridin wird unter Rühren mit 3,35 ml Acetanhydrid versetzt, wobei man nach etwa 15 Minuten eine klare Lösung erhält. Nach Rühren über Nacht werden die ausgefallenen gelben Kristalle abgesaugt und mit Aether gewaschen. Das Filtrat wird im Vakuum eingedampft, und der Rückstand wird zusammen mit den obigen gelben Kristallen aus Methanol umkristallisiert. Man erhält reines (4,5-Dihydro-7-oxo-8- phenyl -thieno[2,3-a]chinolizin-1-yl)methylacetat vom Smp. 145-146°.

In analoger Weise erhält man:

b) Aus 6,7-Dihydro-1-(hydroxymethyl)-3-phenyl-10-chlor- 4H-benzo[a]chinolizin-4-on das (10-Chlor-6,7-dihydro-4-oxo-3- phenyl -4H-benzo[a]chinolizin-1-yl)methylacetat vom Smp. 124-125°;

c) aus (S)-1-[(10-Chlor-6,7-dihydro-4-oxo -3-phenyl-4H- benzo[a]chinolizin-1-yl)carbonyl] -2-pyrrolidinmethanol das [(S)- 1-[(10-Chlor-6,7-dihydro-4-oxo-3-phenyl -4H-benzo- [a]chinolizin- 1-yl)carbonyl] -2-pyrrolidinyl]methylacetat vom Smp. 134-136°;

d) aus 1-[(10-Chlor-6,7-dihydro-4-oxo -3-phenyl-4H- benzo[a]chinolizin-1-yl)carbonyl]-4-piperidinol das 1-[(10-Chlor-6,7-dihydro-4-oxo -3-phenyl-4H-benzo[a]chinolizin-1-yl)carbonyl] -4-piperidinylacetat vom Smp. 179-181°.

Beispiel 6

Man suspendiert 6,8 g 4,5-Dihydro-10-(hydroxymethyl)-8- phenyl-7H -thieno[2,3-a]chinolizin-7-on in 220 ml Methylenchlorid, versetzt mit 68 g Braunstein und rührt über Nacht bei Raumtemperatur. Das anorganische Material wird abfiltriert, und das Filtrat wird eingedampft. Der Rückstand wird aus Dioxan kristallisiert, wobei man reinen 4,5-Dihydro-7-oxo-8-phenyl-7H -thieno[2,3-a]chinolizin-10- carboxaldehyd vom Smp. 212-212,5° erhält.

Beispiel 7

a) Eine Suspension von 275 mg Natriumhydrid in Mineralöl (55-proz.) wird zweimal mit n-Pentan gewaschen. Man gibt dann 6,3 ml Dimethylformamid und anschliessend innerhalb von 15 Minuten tropfenweise eine Lösung von 1,3 g 4,5-Dihydro-10-(hydroxymethyl)-8-phenyl-7H -thieno[2,3-a]chinolizin-7-on in 20 ml Tetrahydrofuran dazu. Sobald die Wasserstoff-Entwicklung beendet ist, gibt man 0,47 ml Methyljodid dazu, rührt während 1 Stunde bei Raumtemperatur und versetzt dann unter Kühlung mit Wasser. Der ausgefallene gelbe Niederschlag wird mit Wasser gut gewaschen. Man erhält nach Umkristallisieren aus Acetonitril reines 4,5-Dihydro-10-(methoxymethyl)-8-phenyl-7H -thieno[2,3-a]chinolizin-7-on vom Smp. 119,5-120,5°.

In analoger Weise erhält man:

b) Aus 4,5-Dihydro-10-(hydroxymethyl)-8-phenyl-7H -thieno[2,3-a]chinolizin-7-on und Aethyljodid das 4,5-Dihydro-10-(äthoxymethyl)-8-phenyl-7H -thieno[2,3-a]chinolizin-7-on vom Smp. 140-141° (aus Aethanol);

c) aus 10-(Hydroxymethyl)-8-phenyl -7H-thieno[2,3- a]chinolizin-7-on und 2-Chloräthyl-methyläther in Gegenwart ca. eines Aequivalentes Kaliumjodid das 10-[(2-Methoxyäthoxy)methyl] -8-phenyl-7H-thieno[2,3-a]chinolizin -7-on vom Smp. 106-107°;

d) aus 6,7-Dihydro-1-(hydroxymethyl)-3-phenyl-10-chlor-4H-benzo[a]chinolizin-4-on und Aethyljodid das 10-Chlor- 6,7-dihydro-1-(äthoxymethyl)-3-phenyl -4H-benzo[a]chinolizin-4-on vom Smp. 121-123°.

Beispiel 8

Zu einer Suspension von 1,02 g 4,5-Dihydro-10- (hydroxymethyl)-8-phenyl-7H -thieno[2,3-a]chinolizin-7-on in 11,5 ml Toluol werden einige Tropfen Triäthylamin und dreimal je 0,24 ml Aethylisocyanat dazugegeben, wobei dazwischen jeweils etwa während 2 Stunden bei 85° gerührt wird. Nach Beendigung der Reaktion dampft man im Vakuum ein. Durch Umkristallisieren des Rückstandes aus Aethanol erhält man reines (4,5-Dihydro-7-oxo-8-phenyl-7H -thieno[2,3- a]chinolizin-10-yl)methyl-äthylcarbamat vom Smp. 136-137°.

Beispiel 9

Zu einer Suspension von 2,17 g 4,5-Dihydro-10- (hydroxymethyl)-8-phenyl-7H -thieno[2,3-a]chinolizin-7-on in 56 ml Methylenchlorid wird innerhalb 15 Minuten eine Lösung von 0,92 ml Trichloracetylisocyanat in 14 ml Methylenchlorid getropft. Nach 1,5 Stunden wird im Vakuum eingedampft. Der Rückstand wird in Aether suspendiert, worauf man absaugt und mit Aether gut auswäscht. Nach kurzem Trocknen werden die erhaltenen Kristalle in 60 ml Tetrahydrofuran/Methanol (1:1) suspendiert, worauf man mit 1,91 g Kaliumcarbonat in 14 ml Wasser versetzt und über Nacht bei Raumtemperatur rührt. Nach Absaugen des Produktes, Waschen mit Wasser und Umkristallisieren aus Dioxan erhält man reines (4,5-Dihydro-7-oxo-8-phenyl-7H -thieno[2,3-a]chinolizin-10-yl)methylcarbamat vom Smp. 237-238°.

Beispiel 10

a) Zu einer siedenden Lösung von 0,34 g 4,5-Dihydro-7- oxo-8-(m-fluorphenyl)-7H- thieno[2,3-a]chinolizin-10- carbonsäure in 5 ml Toluol tropft man innert 20 Minuten 0,96 ml N,N-Dimethylformamid-di-t-butylacetal. Man erhitzt weitere 30 Minuten unter Rückfluss, lässt über Nacht bei Raumtemperatur stehen und wäscht dann einmal mit Wasser, zweimal mit gesättigter wässriger Natriumbicarbonatlösung und einmal mit gesättigter wässriger Natriumchloridlösung. Die organische Phase wird über Natriumsulfat getrocknet und im Vakuum eingedampft. Den Rückstand kristallisiert man aus Essigsäureäthylester um und erhält 8-(m-Fluorphenyl)- 4,5-dihydro-7-oxo-7H -thieno[2,3-a]chinolizin-10- carbonsäure-tert-butylester vom Smp. 135-137°.

In analoger Weise erhält man:

b) Aus 4,5-Dihydro-7-oxo-8-phenyl-7H -thieno[2,3-a]chinolizin- 10-carbonsäure den 4,5-Dihydro-7-oxo-8-phenyl-7H -thieno[2,3-a]chinolizin-10-carbonsäure-tert-butylester vom Smp. 166,5-167,5° (aus Cyclohexan);

c) aus 4,5-Dihydro-7-oxo-8-phenyl-7H -thieno[3,2-a]chinolizin- 10-carbonsäure den 4,5-Dihydro-7-oxo-8-phenyl-7H -thieno[3,2-a]chinolizin-10-carbonsäure-tert-butylester vom Smp. 200-202° (aus Essigsäureäthylester);

d) aus 10-Chlor-6,7-dihydro-4-oxo-3-phenyl -4H-benzo[a]chinolizin-1-carbonsäure den 2-Chlor-5,6-dihydro-8-oxo-9- phenyl-8H-benzo[a]chinolizin-11-carbonsäure-tert- butylester.

Beispiel 11

2,0 g 4,5-Dihydro-7-oxo-8-phenyl-7H -thieno[2,3-a]chinolizin-10-carbonsäure und 6,2 ml Thionylchlorid werden bei Raumtemperatur während 2 Stunden gerührt, worauf das überschüssige Thionylchlorid im Vakuum abdestilliert wird. Der im Hochvakuum getrocknete Rückstand wird in 20 ml Tetrahydrofuran gelöst. Diese Lösung tropft man unter Rühren bei -78° langsam zu dem aus 168 mg Magnesium und 0,9 ml einer 65,5-proz. Lösung von Methylbromid in Tetrahydrofuran hergestellten Methylmagnesiumbromidlösung. Nachdem sich das Reaktionsgemisch auf Raumtemperatur erwärmt hat, wird es über Nacht bei dieser Temperatur gerührt, dann unter Kühlen mit 0,1N-Salzsäure angesäuert und mit Aether extrahiert. Die Aetherphase wird mit 10-proz. wässriger Kaliumbicbarbonatlösung und gesättigter wässriger Kochsalzlösung gewaschen. Man trocknet über Magnesiumsulfat, dampft im Vakuum ein und chromatographiert mit Cyclohexan/Dioxan (2:1) an Kieselgel. Durch Umkristallisieren aus Isopropanol erhält man reines 10-Acetyl-4,5-dihydro-8-phenyl-7H -thieno[2,3-a]chinolizin-7-on vom Smp. 149-149,5°.

Beispiel 12

Eine Lösung von 0,303 g 5,6-Dihydro-3-hydroxy-2- (m-fluorphenyl)-thiazolo[3,2-a]thieno[2,3-c]-pyridiniumhydroxid (inneres Salz) und 0,145 ml 3-Butyn-2-on in 5 ml Toluol wird während 1 Stunde unter Rückfluss erhitzt. Das Lösungsmittel wird im Vakuum eingedampft, und der Rückstand wird über Kieselgel mit Toluol/Essigsäureäthylester (1:1) chromatographiert. Man erhält nach Umkristallisieren aus Aethanol 10-Acetyl-4,5-dihydro-8-(m-fluorphenyl)-7H-thieno[2,3-a]chinolizin-7-on vom Smp. 109-110°.

Beispiel 13

a) Eine Lösung von 0,1 g 1-[[4,5-Dihydro-7-oxo-8-phenyl-7H-thieno[2,3-a]chinolizin-10-yl]carbonyl] -4-piperidin-carbonsäure-äthylester und 0,016 g Natriumhydroxid in 2 ml Aethanol wird während 3 Stunden unter Rückfluss erhitzt. Dann dampft man im Vakuum ein, nimmt den Rückstand in Wasser auf und extrahiert mit Chloroform. Die wässrige Phase stellt man mit 3N-Salzsäure sauer und extrahiert mit Essigsäureäthylester. Man trocknet die organische Phase über Natriumsulfat, dampft im Vakuum ein und erhält rohe 1-[[4,5-Dihydro-7-oxo-8-phenyl-7H -thieno[2,3-a]chinolizin- 10-yl]carbonyl] -4-piperidincarbonsäure.

b) Zu 9 ml Thionylchlorid werden unter Rühren 3 g 1-[[4,5-Dihydro-7-oxo-8-phenyl-7H -thieno[2,3-a]chinolizin-10-yl]carbonyl] -4-piperidincarbonsäure portionenweise zugegeben. Das Gemisch wird bei Raumtemperatur während 1 Stunde gerührt, worauf das überschüssige Thionylchlorid im Vakuum entfernt wird. Der Rückstand wird in 30 ml Toluol gelöst. Man gibt unter Rühren 4,8 ml Triäthylamin hinzu und versetzt anschliessend mit 4,13 ml einer 15-proz. Lösung von Aethylamin in Toluol. Das Reaktionsgemisch wird während 1,5 Stunden bei Raumtemperatur gerührt, dann mit Wasser versetzt und mit Methylenchlorid dreimal extrahiert. Die organische Phase wird über Natriumsulfat getrocknet und eingedampft. Der Rückstand wird an Kieselgel chromatographiert, und das erhaltene Produkt wird aus Methylenchlorid/Diäthyläther umkristallisiert. Man erhält N-Aethyl-1-[[4,5- dihydro-7-oxo-8-phenyl-7H -thieno[2,3-a]chinolizin-10-yl]carbonyl] -4-piperidincarboxamid vom Smp. 134-135°.

Beispiel 14

Zu einer Lösung von 1,0 g 4,5-Dihydro-N-(2- hydroxyäthyl)-8-phenyl-7-oxo-7H -thieno[2,3-a]chinolizin-10- carboxamid in 27 ml Tetrahydrofuran werden 0,94 g Azodicarbonsäure-diäthylester und 0,71 g Triphenylphosphin gegeben, worauf das Reaktionsgemisch während 24 Stunden gerührt wird. Dann dampft man im Vakuum ein, versetzt den Rückstand mit Wasser und extrahiert dreimal mit Methylenchlorid. Die organische Phase wird über Natriumsulfat getrocknet und im Vakuum eingedampft. Den Rückstand chromatographiert man an Kieselgel mit Acetonitril/Diäthyläther (2:3). Durch Umkristallisieren aus Dioxan erhält man 4,5-Dihydro-10-(2- oxazolin-2-yl)-8-phenyl-7H -thieno[2,3-a]chinolizin-7-on vom Smp. 211-212°.

Beispiel 15

a) 6,4 g 5,6-Dihydro-3-hydroxy -2-phenylthiazolo[3,2- a]thieno[2,3-c]pyridinium-hydroxid (inneres Salz) werden in 225 ml Toluol suspendiert, worauf man mit 1,75 ml Acrylnitril versetzt und über Nacht unter Rückfluss erhitzt. Nach dem Abkühlen wird das ausgefallene Produkt abgesaugt und mit Toluol gewaschen. Umkristallisieren aus Acetonitril liefert reines 4,5,7,8,9,10-Hexahydro-7-oxo-8-phenyl-8,10a-epithio- 10aH -thieno[2,3-a]chinolizin-10-carbonitril vom Smp. 228-229°.

b) 2,35 g 4,5,7,8,9,10-Hexahydro-7-oxo-8-phenyl-8,10a- epithio-10aH -thieno[2,3-a]chinolizin-10-carbonitril werden zu einer frisch bereiteten Natriummethylatlösung (hergesttellt aus 178 mg Natrium und 10 ml Methanol) gegeben. Es wird während 2 Stunden unter Rückfluss erhitzt, wobei zunächst eine klare Lösung entsteht, aus welcher dann ein gelbes Produkt ausfällt. Nach dem Abkühlen wird das ausgefallene 4,5-Dihydro-7-oxo-8-phenyl-7H -thieno[2,3- a]chinolizin-carbonitril abgesaugt, mit Methanol gewaschen und aus Aethanol/Dioxan umkristallisiert. Das Produkt hat einen Smp. von 189-189,5°.

Beispiel 16

Eine Lösung von 1,5 g 4,5-Dihydro-7-oxo-8-phenyl-7H -thieno[2,3-a]chinolizin-10-carbonitril in 15 ml Tetrahydrofuran wird mit 20 ml einer 1M-$BH_3$/Tetrahydrofuranlösung versetzt und bei Raumtemperatur bis zur vollständigen Umsetzung gerührt. Dann wird unter Eiskühlung mit 2N- Salzsäure vorsichtig angesäuert, kurz zum Sieden erhitzt und mit 2N-Natronlauge alkalisch gestellt. Das Tetrahydrofuran wird im Vakuum entfernt, und der wässrige Rückstand wird mit Chloroform extrahiert. Die organische Phase wird mit gesättigter Kochsalzlösung gewaschen, über Magnesiumsulfat getrocknet und eingedampft. Der Rückstand wird mit Chloroform/Methanol (19:1) an Kieselgel chromatographiert. Man erhält nach Behandlung mit methanolischer Salzsäure reines 10-(Aminomethyl)-4,5-dihydro- 8-phenyl-7H -thieno[2,3-a]chinolizin-7-on-hydrochlorid vom Smp. 254-255°.

Beispiel 17

a) 4,27 g 5,6-Dihydro-3-hydroxy -2-phenylthiazolo[3,2- a]thieno[2,3-c]pyridinium-hydroxid (inneres Salz) werden in 50 ml Toluol suspendiert. Man versetzt mit 1,32 g Nitroäthylen, rührt zunächst bei Raumtemperatur, erwärmt nach 1 Stunde kurz auf 60°, dampft im Vakuum ein und chromatographiert den Rückstand mit Toluol/Essigsäureäthylester (9:1) an Kieselgel. Durch Kristallisieren aus Acetonitril erhält man reines 4,5,9,10-Tetrahydro-10-nitro-8-phenyl-8,10a-epithio-10aH -thieno[2,3-a]chinolizin-7(8H)-on vom Smp. 192-196°.

b) 1,58 g 4,5,9,10-Tetrahydro-10-nitro-8-phenyl-8,10a- epithio-10aH -thieno[!2,3-a]chinolizin-7(8H)-on werden zusammen mit 2 Aequivalenten Natriummethylat in 30 ml Methanol langsam erwärmt. Nach Abklingen der Reaktion wird noch während 2 Stunden unter Rückfluss erhitzt, worauf man das Lösungsmittel abdestilliert und den Rückstand mit Toluol/Aceton (9:1) an Kieselgel chromatographiert. Man erhält 4,5- Dihydro-10-nitro-8-phenyl-7H -thieno[2,3-a]chinolizin-7-on vom Smp. 186-189° (aus Acetonitril) und in einer späteren Fraktion 10-Amino-4,5-dihydro-8-phenyl-7H -thieno[2,3-a]chinolizin-7-on vom Smp. 180,5-181,5° (aus Toluol).

Beispiel 18

3,2 g 4,5,9,10-Tetrahydro-10-nitro-8-phenyl-8,10a- epithio-10aH -thieno[2,3-a]chinolizin-7(8H)-on werden in 300 ml Methanol suspendiert. Man versetzt unter Rühren mit einer Lösung von Natriummethylat in Methanol (hergestellt aus 4,46 g Natrium und 5 ml Methanol) und anschliessend mit einer Lösung von 14 g Natriumsulfid in 40 ml Methanol, wobei eine dunkle Lösung entsteht, die 4 Stunden lang bei 60° gehalten wird. Nach Abdestillieren des Methanols im Vakuum wird der Rückstand in Wasser aufgenommen. Man extrahiert mit Chloroform und wäscht die organische Phase mit gesättigter wässriger Natriumchloridlösung. Man trocknet die organische Phase über Magnesiumsulfat, dampft im Vakuum ein und chromatographiert das Produkt mit Toluol/Aceton (9:1) an Kieselgel. Man erhält 10-Amino-4,5-dihydro-8-phenyl-7H -thieno[2,3-a]chinolizin-7-on vom Smp. 180,5-181,5° (aus Toluol).

Beispiel 19

Eine Lösung von 0,78 g 10-Amino-4,5-dihydro-8-phenyl- 7H -thieno[2,3-a]chinolizin-7-on in 16 ml Aceton wird mit 1,84 g gepulvertem Kaliumcarbonat und 1,26 ml Chlorameisensäure- äthylester versetzt und während 3 Stunden unter Rückfluss erhitzt. Das ungelöste anorganische Material wird abgesaugt und mit Aceton gewaschen. Das Filtrat wird im Vakuum eingedampft. Man nimmt den Rückstand in Chloroform auf, wäscht mit Wasser und gesättigter wässriger Kochsalzlösung, trocknet über Magnesiumsulfat, dampft ein und chromatographiert mit Toluol/Aceton (19:1) an Kieselgel. Man erhält 4,5-Dihydro-7-oxo-8-phenyl-7H -thieno[2,3- a]chinolizin-10-di(äthylcarbamat) vom Smp. 180,5-181,5° (aus Aethanol) und in einer späteren Fraktion 4,5-Dihydro-7-oxo- 8-phenyl-7H -thieno[2,3-a]chinolizin-10-äthylcarbamat vom Smp. 212-213° (aus Toluol).

Beispiel 20

0,48 g 4,5-Dihydro-7-oxo-8-phenyl-7H -thieno[2,3- a]chinolizin-10-carbonsäure wird zusammen mit 1,5 ml Thionylchlorid während 1 Stunde bei Raumtemperatur gerührt, worauf überschüssiges Thionylchlorid im Vakuum entfernt wird. Das erhaltene gelbe, kristalline Säurechlorid wird über Nacht im Hochvakuum getrocknet. Dieses wird dann in 1,5 ml Dioxan gelöst. Man versetzt mit 0,3 ml Trimethylsilylazid und rührt während 3 Stunden bei 80°. Dann werden 2,5 ml Aethanol zugegeben, worauf man bei 85° während 4 Stunden rührt. Das Reaktionsgemisch wird im Vakuum eingedampft, und der verbleibende Rückstand wird mit Toluol/Aceton (9:1) an Kieselgel chromatographiert. Kristallisieren aus Toluol liefert 4,5-Dihydro-7-oxo-8-phenyl-7H -thieno[2,3-a]chinolizin-10- äthylcarbamat vom Smp. 212-213°.

Beispiel 21

Man stellt aus 1,61 g 4,5-Dihydro-7-oxo-8-phenyl-7H -thieno[2,3-a]chinolizin-10-carbonsäure und 5 ml Thionylchlorid das entsprechende Säurechlorid her, und zwar in Analogie zu den Angaben in Beispiel 20. Man löst dieses in 10 ml Tetrahydrofuran, tropft bei 0-5° ätherische Diazomethanlösung im Ueberschuss dazu und rührt das Reaktionsgemisch während 2 Stunden bei Raumtemperatur. Das ausgefallene Diazoketon wird abgesäugt, mit Aether gut gewaschen und in 33 ml Methanol suspendiert. Man versetzt mit 2 Spatelspitzen frisch bereitetem Silberoxid und erhitzt während 3 Stunden unter Rückfluss, wobei das gesamte Diazoketon in Lösung geht. Nach Abtrennen des Silberoxids wird eingedampft, und der Rückstand wird mit Toluol/Essigsäureäthylester (9:1) an Kieselgel chromatographiert. Kristallisieren aus Methanol liefert reinen 4,5-Dihydro-7-oxo-8-phenyl-7H- thieno[2,3- a]chinolizin-10-essigsäuremethylester vom Smp. 147,5-148°.

Beispiel 22

a) 0,646 g 4,5-Dihydro-7-oxo-8-phenyl-7H-thieno[2,3- a]chinolizin-10-carbonsäure werden in 20 ml Tetrahydrofuran gelöst. Man versetzt bei -15° mit 0,4 ml einer 10M-Lösung von Boran/Methylsulfid in Tetrahydrofuran, lässt auf Raumtemperatur erwärmen und erhitzt anschliessend während 2 Stunden unter Rückfluss. Nach Abkühlen auf Raumtemperatur wird das Reaktionsgemisch mit Methanol versetzt und im Vakuum eingedampft. Man erhitzt den Rückstand in 2N- Salzsäure kurz zum Sieden und extrahiert mit Chloroform. Die organischen Extrakte werden mit gesättigter wässriger Natriumbicarbonatlösung gewaschen, über Magnesiumsulfat getrocknet und im Vakuum eingedampft. Man erhält nach Umkristallisieren aus Essigsäureäthylester/Diäthyläther reines 4,5- Dihydro-10-methyl-8-phenyl-7H -thieno[2,3-a]chinolizin-7-on vom Smp. 80,5-81°.

In analoger Weise erhält man:

b) Aus 4,5-Dihydro-7-oxo-8-phenyl-7H -thieno[3,2- a]chinolizin-10-carbonsäure und Boran/Methylsulfid das 4,5- Dihydro-10-methyl-8-phenyl-7H-thieno[3,2-a]chinolizin-7-on vom Smp. 130-136°;

c) aus 10-Chlor-6,7-dihydro-4-oxo -3-phenyl-4H- benzo[a]chinolizin -1-carbonsäure und Boran/Methylsulfid das 10-Chlor- 6,7-dihydro-1-methyl-3-phenyl-4H-benzo[a]chinolizin-4-on vom Smp. 151-153°.

Beispiel 23

2,85 g 5,6-Dihydro-3-hydroxy-2-phenylthiazolo[3,2- a]thieno[2,3-c]pyridinium-hydroxid (inneres Salz) werden zusammen mit 1,45 ml Phenylvinylsulfoxid in 100 ml Xylol über Nacht unter Rückfluss erhitzt, worauf das Reaktionsgemisch im Vakuum eingedampft und der erhaltene Rückstand mit Toluol/Essigsäureäthylester (9:1) an Kieselgel chromatographiert werden. Man erhält 4,5-Dihydro-8-phenyl-7H-thieno[2,3-a]chinolizin-7-on vom Smp. 133-134° (aus Essigsäureäthylester).

Beispiel 24

a) Eine Lösung von 0,71 ml 2-(Diäthylamino) äthylamin in 5 ml Diäthyläther wird unter Eiskühlung mit ätherischer Salzsäure angesäuert. Man dampft im Vakuum ein, löst das erhaltene Hydrochlorid in 5 ml Methanol und versetzt mit 307,4 mg 4,5-Dihydro-7-oxo-8-phenyl-7H -thieno[2,3-a]chinolizin-10-carboxaldehyd und 62,8 mg Natriumcyanoborhydrid, wobei nach einiger Zeit eine klare Lösung entsteht. Nach Rühren über Nacht wird im Vakuum eingedampft, der Rückstand wird in 2N-Salzsäure kurz erhitzt. Nach dem Abkühlen stellt man die Reaktionslösung mit 2N-Natronlauge alkalisch und extrahiert dreimal mit Chloroform. Der organische Extrakt wird mit gesättigter wässriger Kochsalzlösung gewaschen, über Magnesiumsulfat getrocknet und im Vakuum eingedampft. Den Rückstand nimmt man in Aethanol auf und fällt dann mit äthanolischer Salzsäure das Dihydrochlorid aus. Umkristallisieren aus Eisessig liefert 10-[[[2- (Diäthylamino)äthyl]amino]methyl]-4,5-dihydro -8-phenyl-7H-thieno[2,3- a]chinolizin-7-on-dihydrochlorid vom Smp. 230-231°.

In analoger Weise erhält man:

b) Aus 4,5-Dihydro-7-oxo-8-phenyl-7H-thieno[2,3- a]chinolizin-10-carboxaldehyd und 2-Aminoäthanol-hydrochlorid das 4,5-Dihydro-10-[[(2-hydroxyäthyl)amino]methyl]-8-phenyl-7H- thieno[2,3-a]chinolizin-7-on-hydrochlorid vom Smp. 229- 229,5° (aus Methanol);

c) aus 4,5-Dihydro-7-oxo-8-phenyl-7H -thieno[2,3- a]chinolizin-10-carboxaldehyd und Glycinäthylester-hydrochlorid das N-[(4,5-Dihydro-7-oxo-8-phenyl-7H -thieno[2,3-a]chinolizin-10- yl)methyl] glycin-äthylester-hydrochlorid vom Smp. 158,5-160° (aus Dioxan);

d) aus 4,5-Dihydro-7-oxo-8-phenyl -7H-thieno[2,3- a]chinolizin-10-carboxaldehyd und Morpholin das 4,5-Dihydro-10- (morpholinomethyl)-8-phenyl -7H-thieno[2,3-a]chinolizin-7- on-hydrochlorid vom Smp. 220-226° (aus Isopropanol).

Beispiel 25

1,8 g 4,5-Dihydro-7-oxo-8-phenyl-7H -thieno[2,3- a]chinolizin-10-carbonsäure-methylester werden in 32 ml Chloroform gelöst, worauf man bei 0° mit 0,55 ml Brom in 1 ml Chloroform versetzt und über Nacht

bei Raumtemperatur rührt. Nach Waschen mit 2N-Natronlauge und gesättigter Kochsalzlösung, Trocknen über Magnesiumsulfat und Eindampfen wird der Rückstand in Toluol/Essigsäureäthylester (4:1) an Kieselgel chromatographiert. Man erhält reinen 2-Brom-4,5-dihydro-7-oxo- 8-phenyl-7H -thieno[2,3-a]chinolizin-10-carbonsäure- methylester vom Smp. 170,5-171° (aus Isopropanol).

Beispiel 26

Eine Lösung von 1 g 5,6-Dihydro-3-hydroxy -2- phenylthiazolo[3,2-a]thieno[3,2-c]pyridinium-hydroxid (inneres Salz) und 0,23 g 3-Butyn-2-on in 10 ml Toluol wird während 48 Stunden bei Zimmertemperatur gerührt. Man dampft im Vakuum ein und chromatographiert den Rückstand mit Toluol/Essigsäureäthylester (9:1) an Kieselgel. Man erhält reines 10-Acetyl-4,5-dihydro-8-phenyl-7H -thieno[3,2- a]chinolizin-7-on vom Smp. 181-182° (aus Essigsäureäthylester)

Beispiel 27

a) Ein Gemisch aus 20 g 6,7-Dihydro-4-methyl-thieno[3,2- c]pyridin und 19,8 g 2-Methylenphenylessigsäure-methylester wird unter einer Argonatmosphäre während 2 Stunden auf 80° und anschliessend während 48 Stunden auf 110° erhitzt. Zu dem abgekühlten Reaktionsgemisch gibt man Methanol und lässt das Produkt auskristallisieren. Man erhält 4,5,8,9-Tetrahydro- 8-phenyl-7H-thieno[3,2-a]chinolizin-7-on vom Smp. 159-161°.

b) Eine Lösung von 3 g 4,5,8,9-Tetrahydro-8-phenyl-7H- thieno[3,2-a]chinolizin-7-on in 30 ml Toluol wird mit 1,85 g Mangandioxid versetzt und während 3 Tagen unter Rückfluss erhitzt. Man gibt dann noch 1 g Mangandioxid hinzu und erhitzt über Nacht unter Rückfluss. Dann wird filtriert und im Vakuum eingedampft. Man chromatographiert den Rückstand mit Acetonitril an Kieselgel. Das erhaltene Produkt wird aus Toluol umkristallisiert. Man erhält 4,5- Dihydro-8-phenyl-7H-thieno[3,2-a]chinolizin-7-on vom Smp. 187-189°.

Beispiel 28

aa) Methode A: 23,65 g 3,4-Dihydroisochinolin-1(2H)-thion werden in 750 ml Chloroform gelöst, worauf man bei Raumtemperatur unter Kühlen zunächst mit 54 g α-Bromphenylacetylchlorid und 90 Minuten später mit 53 ml Triäthylamin versetzt und über Nacht bei Raumtemperatur rührt. Das Reaktionsgemisch wird mit Wasser gewaschen, über Magnesiumsulfat getrocknet und im Vakuum eingedampft. Man erhält 5,6- Dihydro-3-hydroxy -2-phenylthiazolo[2,3-a]isochinolinium- hydroxid (inneres Salz) vom Smp. 210° (Zers.) (aus Acetonitril/Dioxan).

ab) Methode B: 2,96 g 7-Chlor-3,4-dihydroisochinolin- 1(2H)-thion und 3,7 g 1-(p-Chlorphenyl)-2,2-dicyanoxiran werden in 60 ml Aceton über Nacht gerührt. Der ausgefallene rote kristalline Niederschlag wird abgesaugt und aus Dioxan umkristallisiert. Man erhält 9-Chlor-2-(p-chlorphenyl)-5,6- dihydro -3-hydroxythiazolo[2,3-a]isochinolinium-hydroxid (inneres Salz) vom Smp. 276° (Zers.).

In analoger Weise erhält man:

ac) Aus 7-Chlor-3,4-dihydroisochinolin-1(2H)-thion und α- Bromphenylacetylchlorid (Methode A) oder 1-Phenyl-2,2- dicyanoxiran (Methode B) das 9-Chlor-2-phenyl-5,6-dihydro -3- hydroxythiazolo-[2,3-a]isochinolinium-hydroxid (inneres Salz) vom Smp. 296° (Zers.);

ad) aus 7-Chlor-3,4-dihydroisochinolin-1(2H)-thion und 1- (o-Chlorphenyl)-2,2-dicyanoxiran das 9-Chlor-2-(o-chlorphenyl)- 5,6-dihydro -3-hydroxythiazolo[2,3-a]isochinolinium-hydroxid (inneres Salz) vom Smp. 260-262° (Methode B, Dimethylformamid als Lösungsmittel);

ba) 4,2 g 9-Chlor-2-phenyl-5,6-dihydro -3-hydroxythiazolo[2,3- a]isochinolinium-hydroxid (inneres Salz) werden zusammen mit 1,32 ml Propiolsäure-methylester in 100 ml Toluol während 4 Stunden unter Rückfluss erhitzt. Nach Einengen im Vakuum wird der Rückstand mit Toluol/Aceton (9:1) an Kieselgel chromatographiert. Man erhält 10-Chlor-6,7- dihydro-4-oxo-3-phenyl-4H -benzo[a]chinolizin-1-carbonsäure- methylester vom Smp. 139-141° (aus Essigsäureäthylester).

In analoger Weise erhält man:

bb) Aus 9-Chlor-2-(p-chlorphenyl)-5,6-dihydro -3- hydroxythiazolo[2,3-a]isochinolinium-hydroxid (inneres Salz) und Propiolsäuremethylester den 10-Chlor-3-(p-chlorphenyl)-6,7- dihydro-4-oxo-4H -benzo[a]chinolizin-1-carbonsäure- methylester vom Smp. 138,5-140,5° (aus Essigsäureäthylester);

bc) aus 9-Chlor-2-(o-chlorphenyl)-5,6-dihydro -3- hydroxythiazolo[2,3-a]isochinolinium-hydroxid (inneres Salz) und Propiolsäuremethylester den 10-Chlor-3-(o-chlorphenyl)-6,7- dihydro-4-oxo-4H -benzo[a]chinolizin-1-carbonsäure- methylester vom Smp. 87,5-89,5° (aus Aethanol);

bd) aus 5,6-Dihydro-3-hydroxy -2-phenylthiazolo[2,3- a]isochinolinium-hydroxid (inneres Salz) und Propiolsäuremethylester den 6,7-Dihydro-3-phenyl-4-oxo-4H -benzo[a]chinolizin-1- carbonsäure-methylester vom Smp. 196,5-197,5° (aus Aethanol);

be) aus 5,6-Dihydro-3-hydroxy -2-phenylthiazolo[2,3- a]isochinolinium-hydroxid (inneres Salz) und Phenylvinylsulfoxid in Xylol das 6,7-Dihydro-3-phenyl-4H-benzo[a]chinolizin-4-on vom Smp. 139,5-140,5° (aus Acetonitril);

bf) aus 9-Chlor-2-phenyl-5,6-dihydro-3-hydroxythiazolo[2,3- a]isochinolinium-hydroxid (inneres Salz) und Propiolsäure- äthylester den 10-Chlor-6,7-dihydro-4-oxo-3-phenyl-4H- benzochinolizin -1-carbonsäureäthylester vom Smp. 114-116° (aus Aethanol).

Beispiel 29

a) 4,08 g 5,6-Dihydro-3-hydroxy -2-phenylthiazolo[2,3- a]isochinolinium-hydroxid (inneres Salz) und 1,95 ml Acrylnitril werden zusammen in 250 ml Toluol über Nacht unter Rückfluss erhitzt. Nach Abdampfen des Lösungsmittels im Vakuum wird der Rückstand aus Acetonitril umkristallisiert. Man erhält 1,2,3,4,6,7-Hexahydro-4-oxo-3-phenyl-3,11b- epithio -11bH-benzo[a]chinolizin-1-carbonitril vom Smp. 225-226°.

b) 1,7 g 1,2,3,4,6,7-Hexahydro-4-oxo-3-phenyl-3,11b- epithio -11bH-benzo[a]chinolizin-1-carbonitril werden zusammen mit einer Natriummethylatlösung (bereitet aus 156 mg Natrium in 40 ml Methanol während 2 Stunden unter Rückfluss erhitzt. Nach dem Abkühlen wird das auskristallisierte Produkt abgesaugt und aus Isopropanol umkristallisiert. Man erhält reines 6,7-Dihydro-4-oxo-3-phenyl-4H -benzo[a]chinolizin-1- carbonitril vom Smp. 204-205°.

Beispiel 30

aa) Eine Lösung von 24,51 g 10-Chlor-6,7-dihydro-4-oxo- 3-phenyl-4H -benzo[a]chinolizin-1-carbonsäuremethylester und 3,91 g Natriumhydroxid in 267 ml Aethanol wird über Nacht unter Rückfluss erhitzt. Man dampft das Lösungsmittel im Vakuum ab, nimmt den Rückstand in Wasser auf, extrahiert mit Chloroform, stellt die wässrige Phase mit 2N-Salzsäure sauer und filtriert die ausgefallene 10-Chlor-6,7-dihydro-4- oxo-3-phenyl-4H -benzo[a]chinolizin-1-carbonsäure vom Smp. 280-283° ab.

In analoger Weise erhält man:

ab) Aus 6,7-Dihydro-4-oxo-3-phenyl-4H-benzo[a]chinolizin- 1-carbonsäuremethylester und Natriumhydroxid die 6,7- Dihydro-4-oxo-3-phenyl -4H-benzo[a]chinolizin-1- carbonsäure;

ac) aus 10-Chlor-4-oxo-3-phenyl-4H-benzo[a]chinolizin-1- carbonsäure-methylester die 10-Chlor-4-oxo-3-phenyl -4H- benzo[a]chinolizin-1-carbonsäure vom Smp. 221-222°.

ba) Zu 5,7 ml Thionylchlorid werden unter Rühren 2 g 10- Chlor-6,7-dihydro-4-oxo-3-phenyl -4H-benzo[a]chinolizin-1- carbonsäure portionenweise zugegeben. Das Gemisch wird bei Raumtemperatur während 1 Stunde gerührt, worauf das überschüssige Thionylchlorid im Vakuum entfernt und der Rückstand in 100 ml Toluol gelöst wird. Man gibt unter Rühren 0,58 ml Triäthylamin hinzu und versetzt anschliessend mit 0,67 ml 2-Dimethylaminoäthylamin. Das Reaktionsgemisch wird während 2 Stunden bei Raumtemperatur gerührt, mit gesättigter wässriger Natriumbicarbonatlösung versetzt und mit Methylenchlorid dreimal extrahiert. Die organische Phase wird über Natriumsulfat getrocknet, filtriert und eingedampft. Aus dem erhaltenen Material wird das Hydrochlorid mittels methanolischer Salzsäure hergestellt. Durch Umkristallisieren aus Methanol/Diäthyläther erhält man. 10-Chlor-N-[2- (dimethylamino)äthyl]-6,7-dihydro-4-oxo-3-phenyl-4H- benzo[a]chinolizin-1-carboxamid-hydrochlorid vom Smp. 160-162°.

In analoger Weise erhält man:

bb) Aus 10-Chlor-6,7-dihydro-4-oxo-3-phenyl -4H-benzo[a]chinolizin-1-carbonsäure und 2-Dimethylaminoäthanol das 2-(Dimethylamino)äthyl 10-chlor-6,7-dihydro-4-oxo-3-phenyl -4H-benzo[a]chinolizin-1-carboxylat-hydrochlorid vom Smp. 223-225° (aus Methanol/Diäthyläther);

bc) aus 10-Chlor-6,7-dihydro-4-oxo-3-phenyl -4H-benzo[a]chinolizin-1-carbonsäure und N-Methylpiperazin das 1-[(10-Chlor-6,7-dihydro-4-oxo-3-phenyl -4H-benzo[a]chinolizin- 1-yl)carbonyl] -4-methylpiperazin-hydrochlorid vom Smp. 275-278° (aus Methanol/Diäthyläther);

bd) aus 10-Chlor-6,7-dihydro-4-oxo-3-phenyl -4H- benzo[a]chinolizin-1-carbonsäure und Morpholin das 4-[(10-Chlor-6,7- dihydro-4-oxo-3-phenyl -4H-benzo[a]chinolizin-1- yl)carbonyl]morpholin vom Smp. 245-248° (aus Dioxan/Diäthyläther);

be) aus 10-Chlor-6,7-dihydro-4-oxo-3-phenyl -4H-benzo[a]chinolizin-1-carbonsäure und cis-2,6-Dimethylmorpholin das cis-4-[(6,7-Dihydro-4-oxo-3-phenyl-10-chlor -4H-benzo[a]chinolizin-1-yl)carbonyl]-2,6-dimethylmorpholin vom Smp. 137-140°;

bf) aus 10-Chlor-6,7-dihydro-4-oxo-3-phenyl-4H- benzo[a]chinolizin-1-carbonsäure und 4-Piperidinol das 4-[(10- Chlor-6,7-dihydro-4-oxo-3-phenyl -4H-benzo[a]chinolizin-1-yl)carbonyl]-4-piperidinol vom Smp. 130-134°;

bg) aus 10-Chlor-6,7-dihydro-4-oxo-3-phenyl-4H- benzo[a]chinolizin -1-carbonsäure und 2-Methoxyäthylamin das 10-Chlor-6,7-dihydro-N-(2-methoxyäthyl)-4-oxo-3-phenyl -4H- benzo[a]chinolizin-1-carboxamid vom Smp. 157-158°;

bh) aus 6,7-Dihydro-4-oxo-3-phenyl-4H-benzo[a]chinolizin-1-carbonsäure und 2-Methoxyäthylamin das 6,7-Dihydro-N- (2-methoxyäthyl)-4-oxo-3-phenyl -4H-benzo[a]chinolizin-1- carboxamid vom Smp. 166-167°;

bi) aus 6,7-Dihydro-4-oxo-3-phenyl-4H-benzo[a]chinolizin-1-carbonsäure und 4-Piperidinol den 1-[(6,7-Dihydro-4-oxo-3- phenyl-4H-benzo[a]chinolizin -1-yl)carbonyl]-4-piperidinol vom Smp. 220-222°;

bj) aus 6,7-Dihydro-4-oxo-3-phenyl-4H-benzo[a]chinolizin-1-carbonsäure und cis-2,6-Dimethylmor-

pholin das cis-4-[(6,7- Dihydro-4-oxo-3-phenyl-4H-benzo[a]chinolizin -1-yl)carbonyl]-2,6-dimethylmorpholin vom Smp. 221-223°;

bk) aus 6,7-Dihydro-4-oxo-3-phenyl-4H-benzo[a]chinolizin-1-carbonsäure und Morpholin das 4-[(6,7-Dihydro-4-oxo-3- phenyl-4H-benzo[a]chinolizin -1-yl)carbonyl]morpholin vom Smp. 242-244°;

bl) aus 6,7-Dihydro-4-oxo-3-phenyl-4H-benzo[a]chinolizin-1-carbonsäure und N-Methylpiperazin das 1-[(6,7-Dihydro-4- oxo-3-phenyl-4H-benzo[a]chinolizin -1-yl)carbonyl]-4- methylpiperazin-hydrochlorid vom Smp. 212-214°;

bm) aus 10-Chlor-6,7-dihydro-4-oxo -3-phenyl-4H- benzo[a]chinolizin-1-carbonsäure und Aethanolamin das 10-Chlor-6,7- dihydro -N-(2-hydroxyäthyl)-4-oxo-3-phenyl-4H- benzo[a]chinolizin -1-carboxamid vom Smp. 145-147°;

bn) aus 10-Chlor-6,7-dihydro-4-oxo-3-phenyl-4H- benzo[a]chinolizin-1-carbonsäure und 3-Pyrrolidinol das 1-[(10- Chlor-6,7-dihydro-4-oxo-3-phenyl-4H-benzo[a]chinolizin-1-yl)carbonyl]-3-pyrrolidinol vom Smp. 215-217°;

bo) aus 10-Chlor-6,7-dihydro-4-oxo-3-phenyl -4H-benzo[a]chinolizin-1-carbonsäure und 2-(S)-Pyrrolidinmethanol das (S)-1-[[10-Chlor-6,7-dihydro -4-oxo-3-phenyl-4H- benzo[a]chinolizin-1-yl]carbonyl] -2-pyrrolidinmethanol vom Smp. 164-166°;

bp) aus 10-Chlor-6,7-dihydro-4-oxo-3-phenyl -4H-benzo[a]chinolizin-1-carbonsäure und Tetrahydro-4H-1,4-thiazin das 4-[(10-Chlor-6,7-dihydro-4-oxo-3-phenyl-4H-benzo[a]chinolizin-1-yl)carbonyl]-tetrahydro -4H-1,4-thiazin vom Smp. 262-263;

bq) aus 10-Chlor-6,7-dihydro-4-oxo-3-phenyl-4H-benzo[a]chinolizin-1-carbonsäure und 2-(R)-Pyrrolidinmethanol das (R)-1-[(10-chlor -6,7-dihydro-4-oxo-3-phenyl-4H-benzo[a]chinolizin-1-yl)carbonyl]-2-pyrrolidinmethanol vom Smp. 175-177°;

br) aus 10-Chlor-6,7-dihydro-4-oxo-3-phenyl -4H-benzo[a]chinolizin-1-carbonsäure und 2-Piperidinmethanol das 1-[(10-Chlor-6,7-dihydro-4-oxo-3-phenyl-4H-benzo[a]chinolizin-1-yl)carbonyl]-2-piperidinmethanol vom Smp. 214-216°;

bs) aus 10-Chlor-6,7-dihydro -4-oxo-3-phenyl-4H- benzo[a]chinolizin-1-carbonsäure und 3-Methoxypropylamin das 10-Chlor-6,7-dihydro-N-(3-methoxypropyl)-4-oxo -3-phenyl-4H- benzo[a]chinolizin-1-carboxamid vom Smp. 149°;

bt) aus 10-Chlor-6,7-dihydro-4-oxo-3-phenyl -4H-benzo[a]chinolizin-1-carbonsäure und 1-Amino-2-thiazolin das 10-Chlor- N-(4,5-dihydro-2-thiazolinyl -6,7-dihydro-4-oxo-3-phenyl-4H- benzo[a]chinolizin -1-carboxamid vom Smp. 190-191°.

Beispiel 31

aaa) 38,9 g 3,3'-Dithiobis-thiophen-2-carbonsäure- methylester werden in 1500 ml 1N-Natronlauge und 700 ml Aethanol suspendiert, worauf man bis zur Beendigung der Reaktion unter Rückfluss erhitzt. Nach Abkühlen im Eisbad wird mit 25- proz. Salzsäure angesäuert, wobei das Produkt auskristallisiert. Durch Umkristallisieren aus Wasser erhält man 3,3'-Dithio-bis-thiophen-2-carbonsäure als farblose Kristalle vom Smp. 256-257°.

aab) 41,3 g 3,3'-Dithiobis-thiophen-2-carbonsäure werden in 340 ml Thionylchlorid suspendiert, worauf man während 2,5 Stunden unter Rückfluss erhitzt. Das überschüssige Thionylchlorid wird im Vakuum entfernt, und der Rückstand wird in 700 ml Dioxan suspendiert. Es wird nun Ammoniak in die Lösung eingeleitet. Nach Kühlen im Eisbad wird filtriert, und die Kristalle werden in 1500 ml Wasser während 30 Minuten gerührt. Die Kristalle werden abfiltriert. Durch Umkristallisieren aus n-Butanol/Dioxan erhält man 3,3'-Dithiobis-thiophen-2-carboxamid als farblose Kristalle vom Smp. 222-223°.

aac) 77,5 g 3,3'-Dithio-bis-thiophen-2-carboxamid werden in 1200 ml Dioxan suspendiert, worauf man unter Inertgas auf 45° erwärmt. Zur Suspension gibt man bei 45-48° portionenweise 46,4 g Natriumborhydrid. Nach Beendigung der Wasserstoffentwicklung wird bis zum Verschwinden des Disulfides unter Rückfluss erhitzt. Unter Kühlen werden nun 450 ml Wasser zugetropft und anschliessend wird mit 2N-Salzsäure angesäuert. Nach Verdünnen der Lösung mit 1800 ml Wasser und Sättigung mit Kochsalz wird erschöpfend mit Aether extrahiert. Die vereinigten Extrakte werden über Natriumsulfat getrocknet, filtriert und eingedampft. Nach Trocknen erhält man das rohe 3-Mercaptothiophen-2-carboxamid vom Smp. 116-120°.

aad) 11,1 g 3-Mercapto-thiophen-2-carboxamid, 2,2 g 95-proz. Paraformaldehyd und 13,3 g p-Toluolsulfonsäure-monohydrat werden zusammen mit 300 ml Mesitylen unter Rückfluss erhitzt. Nach beendeter Reaktion lässt man abkühlen und entfernt das Lösungsmittel im Vakuum. Nach Chromatographieren des Rückstandes an Kieselgel und Umkristallisieren aus Essigsäureäthylester erhält man 2,3-Dihydro-4H-thieno[2,3-e][1,3]thiazin-4-on als gelbliche Kristalle vom Smp. 153-155°.

aae) 19,6 g 2,3-Dihydro-4H-thieno[2,3-e][1,3]-thiazin-4-on werden zusammen mit 23,1 g Lawesson-Reagens in 200 ml Acetonitril unter Rückfluss solange erhitzt, bis alles Ausgangsmaterial umgesetzt ist. Das Reaktionsgemisch wird abgekühlt, und das auskristallisierte Produkt wird abfiltriert. Durch Umkristallisieren aus Essigsäureäthylester erhält man reines 2,3-Dihydro-4H-thieno[2,3-e][1,3]thiazin- 4-thion vom Smp. 136-138°. Durch Eindampfen der Mutterlauge und Umkristallisieren kann weiteres Material gewonnen werden.

aba) In analoger Weise erhält man aus 4,5,6,7-Tetrahydro-8H- thieno[2,3-c]azepin-8-on das 4,5,6,7-Tetrahydro-8H-thieno[2,3- c]azepin-8-thion vom Smp. 103-104° (aus Essigsäureäthylester).

aca) Methode A: 17,4 g 3-Mercapto-thiophen-2-carbonsäure- methylester werden unter einer Inertgasatmosphäre in 350 ml Toluol gelöst, worauf man mit 20,5 g 2-Aminoäthylbromid- hydrobromid und dann mit 100 ml 3N-Natriummethylatlösung in Methanol versetzt. Es wird während 30 Minuten bei Raumtemperatur gerührt und nachher unter Rückfluss erhitzt, bis die Reaktion beendet ist. Man dampft im Vakuum ein, nimmt den Rückstand in 300 ml Wasser auf, säuert mit 2N- Salzsäure an und filtriert die Kristalle ab. Durch Umkristallisieren aus Chloroform erhält man 3,4-Dihydro- thieno[2,3-f][1,4]thiazepin-5(2H)-on als weisse Kristalle vom Smp. 186-188°.

Methode B: 65,4 g 3-Mercapto-thiophen-2-carbonsäure- methylester werden in 800 ml Aethanol gelöst, worauf man mit 19,4 ml Aethylenimin versetzt. Nach beendeter Reaktion wird das Reaktionsgemisch im Eisbad abgekühlt, und durch Einleiten von trockenem Chlorwasserstoff wird das Hydrochlorid ausgefällt. Die Kristalle werden abfiltriert, und das Filtrat wird eingedampft, wobei noch mehr Rohprodukt gewonnen wird. Durch Umkristallisieren aus Methanol/Essigsäureäthylester erhält man 3-[(2-Aminoäthyl)thio]-2-thiophen- carbonsäure-methylester -hydrochlorid als weisse Kristalle vom Smp. 174-176°.

1,0 g 3-[(2-Aminoäthyl)thio]-2-thiophen-carbonsäure- methylester -hydrochlorid werden unter Argon in 20 ml Toluol suspendiert, worauf man 9,4 ml 1N-Natriummethylatlösung in Methanol zugibt. Nach beendeter Reaktion wird im Vakuum eingedampft, und der Rückstand wird mit 20 ml Wasser behandelt. Die Kristalle werden abfiltriert, mit Wasser gewaschen und getrocknet. Durch Umkristallisieren erhält man 3,4-Dihydro-thieno[2,3-f][1,4]thiazepin-5(2H)-on als weisse Kristalle vom Smp. 186-188°. Die Reaktion kann auch mit 1,1 Moläquivalent Kalium-tert-butylatlösung in Toluol ausgeführt werden.

acb) 1,0 g 3,4-Dihydro-thieno[2,3-f][1,4]thiazepin-5(2H)-on werden zusammen mit 15 ml Pyridin und 1,35 g Phosphorpentasulfid während 5 Stunden unter Rückfluss erhitzt. Nach Abkühlen giesst man auf 90 ml Wasser und filtriert die Kristalle ab. Nach Chromatographieren an Kieselgel wird aus Essigsäureäthylester umkristallisiert. Man erhält 3,4- Dihydro-thieno[2,3-f][1,4]thiazepin-5(2H)-thion als gelbe Kristalle vom Smp. 138-139°.

ada) 288 g Thieno[2,3-c]pyridin werden in 5,3 l Methylenchlorid gelöst und bei -5° bis 0° wird portionenweise eine äquivalente Menge m-Chlorperbenzoesäure zugesetzt. Es wird bis zur Beendigung der Reaktion weitergerührt. Zugabe von 800 ml gesättigter ätherischer Chlorwasserstofflösung fällt das Produkt als weisse Kristalle aus, die mit Aether gewaschen werden. Das so erhaltene Thieno[2,3-c]pyridin 6-oxid- hydrochlorid ist genügend rein, um für die nächste Stufe eingesetzt zu werden, Smp. 202°.

adb) 671,5 g Thieno[2,3-c]pyridin 6-oxid-hydrochlorid werden unter Argon in 4000 ml Dioxan suspendiert und 655 ml Phosphoroxychlorid werden zugesetzt. Es wird auf dem Oelbad bis zum Einsetzen der exothermen Reaktion erwärmt. Nach dem Abklingen der exothermen Reaktion wird noch 10 Minuten zum Rückfluss erhitzt. Man engt im Vakuum ein und nimmt den Rückstand in 2000 ml Toluol auf. Dann wird unter Kühlung langsam mit 3000 ml Wasser versetzt. Durch portionenweise Zugabe von Soda wird neutralisiert. Die organische Phase wird abgetrennt und die wässrige Phase nochmals mit 1000 ml Toluol extrahiert. Nach Waschen mit Wasser wird über Natriumsulfat getrocknet, filtriert und eingedampft, wobei rohes 7-Chlor- thieno[2,3-c]pyridin als braunes Oel erhalten wird.

adc) 4,85 g 7-Chlor-thieno[2,3-c]pyridin werden in 15 ml Dimethylformamid gelöst und unter Argon werden 3,18 g Natriumhydrogensulfid-monohydrat zugesetzt. Es wird eine Stunde auf 110-115° erhitzt, nochmals 1,06 g Natriumhydrogensulfid- monohydrat zusetzt und während einer weiteren Stunde bei der oben angegebenen Temperatur hält. Es wird auf 150 ml Eiswasser gegossen und mit 1N wässeriger Salzsäurelösung angesäuert. Nach kurzem Rühren bei 2° werden die gelblichen Kristalle abfiltriert. Nach Umkristallisation aus einem Toluol/Essigsäureäthylestergemisch erhält man reines Thieno[2,3-c]pyridin-7(6H)-thion, Smp. 187-189°.

ba) 16,7 g Thieno[2,3-c]pyridin-7(6H)-thion werden in 1000 ml Methylenchlorid unter einer Inertgasatmosphäre suspendiert, worauf man 15,3 ml ca. 90-proz. α-Bromphenylessigsäurechlorid zutropft. Nach beendeter Zugabe wird noch etwa eine halbe Stunde bei Raumtemperatur gerührt und dann 27,8 ml Triäthylamin zugetropft. Anschliessend wird während 30 Minuten bei Raumtemperatur gerührt. Die Lösung wird zweimal mit je 750 ml Wasser gewaschen, über Natriumsulfat getrocknet, filtriert und im Vakuum eingedampft. Die erhaltenen roten Kristalle werden durch Chromatographieren und Umkristallisieren aus Chloroform/Aether/Hexan gereinigt. Man erhält 3-Hydroxy-2-phenylthiazolo[3,2-a]thieno[2,3-c]pyridinium-hydroxid (inneres Salz) als rote Kristalle vom Smp. 195-200° (Zers.).

In analoger Weise erhält man:

bb) Aus 2,3-Dihydro-4H-thieno[2,3-e][1.3]thiazin-4-thion und α-Bromphenacetylchlorid das 7-Hydroxy-8-phenyl-5H- thiazolo[3,2-c]thieno[2,3-e][1,3] thiazinium-hydroxid (inneres Salz) vom Smp. 194-197° (aus Methanol);

bc) aus 4,5,6,7-Tetrahydro-8H-thieno[2,3-c]azepin-8-thion und α-Bromphenacetylchlorid das 5,6-Dihydro-8-hydroxy-9- phenyl -4H-thiazolo[3,2-a]thieno[2,3-c]azepinium-hydroxid (inneres Salz) vom Smp. 205-208° (aus Chloroform/Diäthyläther);

bd) aus 3,4-Dihydrothieno[2,3-f][1,4]thiazepin-5(2H)-thion und α-Bromphenacetylchlorid das 5,6-Dihydro-8-hydroxy-9- phenyl -thiazolo[3,2-d]thieno[2,3-f][1,4]thiazepinium-hydroxid (inneres Salz) vom Smp. 196-198° (aus Chloroform/Diäthyläther).

ca) 22,9 g 3-Hydroxy-2-phenylthiazolo[3,2-a]thieno[2,3-c] pyridinium-hydroxid (inneres Salz) und 13,6 ml Propiolsäuremethylester werden unter Argon zusammen in 1000 ml Toluol bis zum Verschwinden des Ausgangsstoffes unter Rückfluss erhitzt. Das Lösungsmittel wird im Vakuum entfernt, und der Rückstand wird in 500 ml Aether/Methanol (9:1) während 1 Stunde gerührt. Die erhaltenen gelben Kristalle werden abgenutscht. Man erhält den 7-Oxo-8-phenyl-7H-thieno[2,3- a]chinolizin -10-carbonsäuremethylester vom Smp. 153-154° (Zers.). Durch Chromatographieren des Filtrates an Kieselgel kann man eine weitere Portion des Produktes vom Smp. 151-152° (Zers.) gewinnen.

In analoger Weise erhält man:

cb) Aus 7-Hydroxy-8-phenyl-5H-thiazolo[3,2-c]thieno[2,3- e][1,3] thiazinium-hydroxid (inneres Salz) und Propiolsäure- methylester den 7-Oxo-8-phenyl-5H-pyrido[1,2- c]thieno-[2,3-c][1,3] thiazin-10-carbonsäure-methylester vom Smp. 141-143° (aus Methanol);

cc) aus 5,6-Dihydro-8-hydroxy-9-phenyl -4H-thiazolo[3,2- a]thieno[2,3-c]azepinium-hydroxid (inneres Salz) und Propiolsäuremethylester den 8-Oxo-9-phenyl-4,5,6,8 -tetrahydro- pyrido[1,2-a]thieno-[2,3-c]azepin-11-carbonsäure -methylester vom Smp. 142-144° (aus Essigsäureäthylester);

cd) aus 5,6-Dihydro-8-hydroxy-9-phenyl -thiazolo[3,2- d]thieno[2,3-f][1,4]thiazepinium-hydroxid (inneres Salz) und Propiolsäure-methylester den 5,6-Dihydro-8-oxo-9-phenyl -8H-pyrido[1,2-d]thieno[2,3-f][1,4]thiazepin -11-carbonsäuremethylester vom Smp. 158-160° (aus Aethanol).

Beispiel 32

aa) 1,34 g 7-Oxo-8-phenyl-7H-thieno[2,3- a]chinolizin -10-carbonsäure-methylester werden zusammen mit 0,45 g Kaliumhydroxid unter Argon in 25 ml Wasser/Methanol unter Rückfluss erhitzt. Nach beendeter Reaktion säuert man mit 2N-Salzsäure an und filtriert die gelben Kristalle ab. Nach Waschen mit Wasser wird die Säure im Vakuum während mehreren Stunden getrocknet. Man erhält 7-Oxo-8-phenyl-7H- thieno[2,3-a]chinolizin-10-carbonsäure vom Smp. 185-186° (Zers.).

In analoger Weise erhält man:

ab) Aus 8-Oxo-9-phenyl-4,5,6,8-tetrahydro -pyrido[1,2-a]thieno[2,3-c]azepin-11-carbonsäure-methylester die 8-Oxo-9-phenyl-4,5,6,8- tetrahydro -pyrido[1,2-a]thieno[2,3-c]azepin-11-carbonsäure vom Smp. 226-229° (Zers.);

ac) aus 5,6-Dihydro-8-oxo-9-phenyl -8H-pyrido[1,2- d]thieno[2,3-f][1,4]thiazepin-11-carbonsäure - methylester die 5,6-Dihydro-8-oxo-9-phenyl -8H-pyrido[1,2-d]thieno[2,3- f][1,4]thiazepin-11-carbonsäure vom Smp. 265-266° (Zers.; aus Methanol/Dimethylformamid).

ba) 0,64 g 7-Oxo-8-phenyl-7H-thieno[2,3-a]chinolizin-10- carbonsäure werden in 12 ml Toluol suspendiert, worauf man bei Raumtemperatur und unter Feuchtigkeitsausschluss 0,9 ml Thionylchlorid und katalytische Mengen an Dimethylformamid zugibt. Es wird während 1 Stunde bei Raumtemperatur gerührt und dann im Vakuum eingedampft. Der Rückstand wird in 15 ml Dioxan aufgenommen. Nach Zugabe von 0,7 ml Morpholin wird während 1 Stunde bei Raumtemperatur gerührt. Man verdünnt mit Wasser und filtriert die Kristalle ab. Durch Umkristallisieren aus Aethanol/Dimethylformamid erhält man 4-[(7-Oxo-8-phenyl -7H-thieno[2,3- a]chinolizin-10-yl)carbonyl]morpholin als gelbe Kristalle vom Smp. 271-272° (Zers.).

In analoger Weise erhält man:

bb) Aus 8-Oxo-9-phenyl-4,5,6,8-tetrahydro -pyrido[1,2- a]thieno[2,3-c]azepin-11-carbonsäure und Morpholin das 4-[(8-Oxo-9-phenyl-4,5,6,8-tetrahydro -pyrido[1,2-a]thieno[2,3- c]azepin -11-yl)carbonyl]morpholin vom Smp. 238-240° (aus Aethanol);

bc) aus 5,6-Dihydro-8-oxo-9-phenyl -8H-pyrido[1,2- d]thieno[2,3-f][1,4]thiazepin-11-carbonsäure und Morpholin das 4-[(5,6-Dihydro-8-oxo-9-phenyl -8H-pyrido[1,2-d]thieno[2,3- f][1,4]thiazepin -11-yl)-carbonyl]morpholin vom Smp. 253-254° (aus Aethanol);

bd) aus 5,6-Dihydro-8-oxo-9-phenyl -8H-pyrido[1,2- d]thieno[2,3-f][1,4]thiazepin-11-carbonsäure und 2- (Dimethylamino)-äthylamin das N-[2-(Dimethylamino)äthyl] 5,6-dihydro-8-oxo-9-phenyl -8H-pyrido[1,2-d]thieno[2,3-f][1,4]thiazepin-11-carboxamid vom Smp. 155-157° (aus Essigsäureäthylester);

be) aus 5,6-Dihydro-8-oxo-9-phenyl -8H-pyrido[1,2- d]thieno[2,3-f][1,4]thiazepin-11-carbonsäure und 2- Dimethylaminoäthanol das 2-(Dimethylamino)äthyl 5,6-dihydro- 8-oxo-9-phenyl -8H-pyrido[1,2-d]-thieno[2,3-f][1,4]thiazepin-11- carboxylat vom Smp. 115-117° (aus Cyclohexan).

Beispiel 33

a) 1,22 g 7-Oxo-8-phenyl-7H-thieno[2,3-a]chinolizin-10- carbonsäure werden in 40 ml Benzol suspendiert, worauf man 1,8 ml N,N-Dimethylformamid-di-tert-butylacetal zugibt, während 15 Minuten unter Rückfluss erhitzt, nochmals 1,8 ml des obigen Reagenses zugibt, erneut während 15 Minuten unter Rückfluss erhitzt und dieses Prozedere erneut wiederholt. Nach beendeter Reaktion wird im Vakuum eingedampft. Nach Chromatographieren des Rückstandes an Kieselgel und Umkristallisieren aus Toluol erhält man 7-Oxo-8-phenyl-7H- thieno[2,3-a]chinolizin-10-carbonsäure-tert-butylester als gelbe Kristalle vom Smp. 155-157°.

b) In analoger Weise erhält man aus 5,6-Dihydro-8-oxo-9- phenyl -8H-pyrido[1,2-d]thieno[2,3-f][1,4-thiazepin-11- carbonsäure und N,N-Dimethylformamid-di-tert-butylacetal den 5,6-Dihydro-8-oxo-9-phenyl -8H-pyrido[1,2-d]thieno[2,3-f][1,4]thiazepin -11-carbonsäure-tert-butylester vom Smp. 185-187° (aus Toluol).

Beispiel 34

a) 1,93 g 7-Oxo-8-phenyl-7H-thieno[2,3-a]chinolizin- 10-carbonsäure werden wie beschrieben mit 2,65 ml Thionylchlorid in das Säurechlorid übergeführt. Man nimmt dieses in 80 ml Dioxan auf, versetzt mit 2,7 ml N- Methylpiperazin, rührt dann bis zur Beendigung der Reaktion bei Raumtemperatur und giesst auf 200 ml Wasser. Die Wasserphase wird mit Kochsalz gesättigt und mehrmals mit Essigester extrahiert. Die vereinigten organischen Phasen werden über Natriumsulfat getrocknet, filtriert und eingedampft. Durch Umkristallisieren aus Toluol erhält man 1-Methyl-4-[(7-oxo-8-phenyl -7H-thieno[2,3-a]-chinolizin-10-yl)carbonyl]piperazin als gelbe Kristalle vom Smp. 233-234° (Zers.).

In analoger Weise erhält man:

b) Aus 8-Oxo-9-phenyl-4,5,6,8-tetrahydro -pyrido[1,2- a]thieno[2,3-c]azepin-11-carbonsäure und 4-Piperidinol das 1- [(8-Oxo-9-phenyl-4,5,6,8-tetrahydro -pyrido[1,2-a]thieno[2,3- c]azepin-11-yl)carbonyl] -4-piperidinol vom Smp. 196-198° (aus Acetonitril).

Beispiel 35

3,45 g 5,6-Dihydro-8-oxo-9-phenyl -8H-pyrido[1,2- d]thieno[2,3-f][1,4]thiazepin-11-carbonsäure werden bei 0,5 Torr auf 285° erhitzt, worauf man die Temperatur noch während 40 Minuten bei 258-262° hält. Das Rohprodukt wird an Kieselgel chromatographiert. Nach Umkristallisieren aus Acetonitril erhält man 5,6-Dihydro-9- phenyl -8H-pyrido[1,2-d]thieno[2,3-f][1,4]thiazepin-8-on als gelbe Kristalle vom Smp. 139-141°.

Beispiel 36

4,95 g 5,6-Dihydro-9-phenyl -8H-pyrido[1,2- d]thieno[2,3-f][1,4]thiazepin-8-on werden unter Argon zu 2,35 g N-Chlorsuccinimid in 50 ml Tetrachlorkohlenstoff gegeben. Man rührt während 2,5 Stunden bei Raumtemperatur, versetzt mit weiteren 0,21 g N-Chlorsuccinimid und rührt noch während 1 Stunde. Nach Eindampfen im Vakuum wird an Kieselgel chromatographiert. Durch Umkristallisieren aus Acetonitril erhält man 11-Chlor-5,6-dihydro-9-phenyl -8H- pyrido[1,2-d]thieno[2,3-f][1,4]thiazepin-8-on als gelbe Kristalle vom Smp. 179°.

Beispiel 37

a) 3,55 g 5,6-Dihydro-8-oxo-9-phenyl -8H-pyrido[1,2- d]thieno[2,3-f][1,4]thiazepin-11-carbonsäure werden in Gegenwart katalytischer Mengen an Dimethylformamid mit 20 ml Thionylchlorid umgesetzt. Nach beendeter Reaktion wird das Lösungsmittel im Vakuum entfernt. Man erhält das rohe Säurechlorid als gelbe Kristalle vom Smp. 201-204°.

b) 0,89 g Acetamidoxim in 50 ml Methylenchlorid werden mit 1,67 ml Triäthylamin versetzt. Unter Kühlung wird bei 21-24° das obige Säurechlorid in 100 ml Dichlormethan zugetropft, worauf man bis zur Beendigung der Reaktion rührt. Nach Waschen mit Wasser wird über Natriumsulfat getrocknet, filtriert und im Vakuum eingedampft. Das Rohmaterial wird in Gegenwart von katalytischen Mengen an p-Toluolsulfonsäure zusammen mit 110 ml Toluol unter Rückfluss erhitzt, wobei gebildetes Reaktionswasser über einen Wasserabscheider entfernt wird. Das Produkt wird nach Chromatographieren an Kieselgel aus essigsaäureäthylester umkristallisiert, wobei man 5,6-Dihydro-11-(3-methyl-1,2,4- oxadiazol-5-yl)-9-phenyl-8H-pyrido[1,2-d]thieno[2,3-f][1,4]thiazepin-8-on als gelbe Kristalle vom smp. 183-185° erhält.

Beispiel 38

3,8 g 2-(Dimethylamino)äthyl 5,6-Dihydro-8-oxo-9-phenyl -8H-pyrido[1,2-d]thieno[2,3-f][1,4]thiazepin-11-carboxylat in 90 ml Methylenchlorid werden mit 9 ml 3N-äthanolischer Salzsäure behandelt, worauf man auf -10° abkühlt. Man versetz tropfenweise mit 1,72 g ca. 90-proz. m-Chlorperbenzoesäure in 45 ml Methylenchlorid. Nach Aufwärmen auf Raumtemperatur wird mit 100 ml 5-proz. Natriumhydrogencarbonatlösung gewaschen, über Natriumsulfat getrocknet, filtriert und im Vakuum eingedampft. Der Rüchstand wird durch Chromatographieren an Kieselgel gereinigt und dann in 34 ml Methylenchlorid gelöst. Nach Zugabe von 3N-äthanolischer Salzsäure bis zur Reaktion wird noch während 15 Minuten bei Raumtemperatur gerührt. Nach Entfernen der Lösungsmittel im Vakuum und Umkristallisieren aus Methanol erhält man 2-(Dimethylamino)äthyl 5,6-dihydro-8- oxo-9-phenyl -8H-pyrido[1,2-d]thieno[2,3-f][1,4]thiazepin-11- carboxylat -4-oxid-hydrochlorid als gelbe Kristalle vom Smp. 148-152° (Zers.).

Beispiel 39

a) 4 g 7-Oxo-8-phenyl-pyrido[1,2-c]thieno[2,3- e][1,3]thiazin -10-carbonsäure-methylester werden in 100 ml Methylenchlorid gelöst, worauf man auf 0° abkühlt und eine Lösung von 2,43 g m-Chlorperbenzoesäure in 40 ml Methylenchlorid zutropft. Nach beendeter Reaktion wird die ausgefallene m-Chlorbenzoesäure durch Filtration entfernt, und das Filtrat mit gesättigter Natriumhydrogencarbonatlösung und Wasser gewaschen, über Natriumsulfat getrocknet, filtriert und eingedampft. Nach Chromatographieren an Kieselgel wird aus Acetonitril umkristallisiert. Man erhält 7-Oxo-8-phenyl- pyrido[1,2-c]-thieno[2,3-e][1,3]thiazin -10-carbonsäure- methylester 4-oxid als gelbe Kristalle vom Smp. 183-186°.

In analoger Weise erhält man aus m- Chlorperbenzoesäure und:

b) 5,6-Dihydro-8-oxo-9-phenyl -8H-pyrido[1,2- d]thieno- [2,3-f][1,4]thiazepin -11-carbonsäure-tert-butylester den 5,6-Dihydro-8-oxo-9-phenyl -8H-pyrido[1,2- d]thieno[2,3-f][1,4]thiazepin -11-carbonsäure-tert- butylester 4-oxid vom Smp. 185-187° (aus Toluol);

c) 5,6-Dihydro-9-phenyl -8H-pyrido[1,2-d]thieno[2,3-f][1,4]thiazepin-8-on das 5,6-Dihydro-9-phenyl -8H-pyrido[1,2-d]thieno[2,3-f][1,4]thiazepin-8-on 4-oxid vom Smp. 199-200° (aus Methanol).

d) 11-Chlor-5,6-dihydro-9-phenyl -8H-pyrido[1,2- d]thieno[2,3-f][1,4]thiazepin-8-on das 11-Chlor-5,6-dihydro-9- phenyl-8H-pyrido[1,2-d]thieno[2,3-f][1,4]thiazepin-8-on- 4-oxid vom Smp. 209-211° (aus Acetonitril).

e) 5,6-Dihydro-11-(3-methyl-1,2,4-oxadiazol-5-yl)- 9-phenyl-8H-pyrido[1,2-d]thieno[2,3-f][1,4]thiazepin-8-on- das 5,6-Dihydro-11-(3-methyl-1,2,4-oxadiazol-5-yl)- 9-phenyl-8H-pyrido[1,2-d]thieno[2,3-f][1,4]thiazepin-8-on- 4-oxid vom Smp. 220° (aus Acetonitril).

f) 4-(5,6-Dihydro-8-oxo-9-phenyl -8H-pyrido[1,2- d]thieno[2,3-f][1,4]thiazepin-11-yl] carbonyl] morpholin das 4-[(5,6-Dihydro-8-oxo-9-phenyl -8H-pyrido[1,2-d]thieno[2,3- f][1,4]thiazepin-11-yl) carbonyl]morpholin 4-oxid vom Smp. 170° (aus Aethanol);

g) 5,6-Dihydro-8-oxo-9-phenyl -8H-pyrido[1,2- d]thieno[2,3-f][1,4]thiazepin -11-carbonsäure-methylester das 5,6-Dihydro-8-oxo-9-phenyl -8H-pyrido[1,2-d]thieno[2,3- f][1,4]thiazepin-11-carbonsäure -methylester 4-oxid vom Smp. 135-137° (aus Aether/n-Hexan/Essigsäureäthylester).

Beispiel 40

aa) Zu 70 ml Thionylchlorid werden unter Feuchtigkeitsausschluss 15,7 g 5,6-Dihydro-8-oxo-9-phenyl -8H-pyrido[1,2-d]thieno[2,3-f][1,4]thiazepin-11-carbonsäuremethylester 4-oxid portionenweise so zugegeben, dass die Temperatur der Reaktionslösung bei ca. 35° liegt. Nach beendeter Zugabe rührt man noch, bis die Reaktion beendet ist. Das überschüssige Thionylchlorid wird im Vakuum entfernt, und der Rückstand wird mit 100 ml Wasser behandelt. Die Kristalle werden abfiltriert und getrocknet, worauf an Kieselgel chromatographiert wird. Durch Umkristallisieren aus Toluol erhält man 5-Chlor-5,6-dihydro-9-phenyl -8H-pyrido[1,2-d]thieno[2,3-f][1,4]thiazepin-11-carbonsäure -methylester als gelbe Kristalle vom Smp. 192-196°.

ab) In analoger Weise erhält man aus 11-Chlor-5,6-dihydro-9-phenyl -8H-pyrido[1,2-d]thieno[2,3-f][1,4]thiazepin-8-on 4-oxid das 5,11-Dichlor-5,6-dihydro-9-phenyl -8H-pyrido- [1,2-d]thieno[2,3-f][1,4]-thiazepin-8-on vom Smp. 162- 164° (aus Essigsäureäthylester).

ba) 6,05 g 5-Chlor-5,6-dihydro-8-oxo-9-phenyl -8H- pyrido[1,2-d]thieno[2,3-f][1,4]thiazepin-11-carbonsäure - methylester werden in 60 ml Dimethylsulfoxid suspendiert. Es werden 3,6 ml 1,5-Diazabicyclo[4.3.0]-non-5-en zugegeben, worauf man bis zur Beendigung der Reaktion auf 70-75° erhitzt. Man kühlt dann auf Raumtemperatur ab, wobei Kristalle ausfallen. Das Gemisch wird auf 300 ml Wasser gegossen, und die Kristalle werden abfiltriert und mit Wasser gewaschen. Die noch feuchten Kristalle werden in Chloroform gelöst. Man trocknet über Natriumsulfat, filtriert und dampft im Vakuum ein. Nach Chromatographieren an Kieselgel erhält man 8- Oxo-9-phenyl-8H-pyrido[1,2-d]thieno[2,3-f][1,4] thiazepin-11- carbonsäure-methylester als gelbe Kristalle, welche aus Toluol umkristallisiert werden. Das Produkt hat dann einen Smp. von 207-210°.

bb) In analoger Weise erhält man aus 5,11-Dichlor-5,6- dihydro-9-phenyl -8H-pyrido[1,2-d]thieno[2,3-f]thiazepin-8-on das 11-Chlor-9-phenyl-8H-pyrido[1,2-d]thieno[2,3-f][1,4] thiazepin-8-on vom Smp. 149-151° (aus Essigsäureäthylester).

Beispiel 41

Zu 5,5 g 8-Oxo-9-phenyl-8H-pyrido[1,2-d]thieno [2,3- f][1,4]thiazepin-11-carbonsäure-methylester in 100 ml Methylenchlorid werden bei 0° 6,2 g m-Chlorperbenzoesäure (Gehalt etwa 80%) in 130 ml Methylenchlorid zugetropft. Man lässt auf Raumtemperatur aufwärmen und rührt, bis die Reaktion beendet ist. Es wird mit gesättigter Natriumhydrogencarbonatlösung und Wasser gewaschen, über Natriumsulfat getrocknet, filtriert und eingedampft. Nach Chromatographieren an Kieselgel und Umkristallisieren aus Toluol erhält man 8-Oxo-9-phenyl-8H-pyrido[1,2-d]thieno[2,3- f][1,4]thiazepin -11-carbonsäure-methylester 4,4-dioxid als gelbe Kristalle vom Smp. 241-243°.

Beispiel 42

a) 4,4 g 8-Oxo-9-phenyl-8H-pyrido[1,2-d]thieno[2.3-f][1,4]thiazepin -11-carbonsäure-methylester werden unter Argon zusammen mit 90 ml Xylol bis zur Beendigung der Reaktion unter Rückfluss erhitzt. Die Lösung wird im Vakuum eingedampft, und der Rückstand wird mit 100 ml n- Hexan behandelt, worauf

man abfiltriert. Durch Umkristallisieren aus Essigsäureäthylester erhält man 7-Oxo-8-phenyl-7H-thieno[2,3-a]chinolizin-10-carbonsäure -methylester als gelbe Kristalle vom Smp. 153-156°.

b) In analoger Weise erhält man aus 11-Chlor-9-phenyl -8H-pyrido[1,2-d]thieno[2,3-f][1,4]thiazepin-8-on das 10-Chlor-8- phenyl-7H-thieno[2,3-a]chinolizin-7-on vom Smp. 178-180° (aus Essigsäureäthylester).

Beispiel 43

a) Eine Suspension von 0,59 g 4,5-Dihydro-10- (hydroxymethyl)-8-phenyl -7H-thieno[2,3-a]chinolizin-7-on in 15 ml Dioxan wird mit 0,38 ml Chlorameisensäure-phenylester und 0,27 ml Pyridin versetzt und 2,5 Stunden bei Raumtemperatur gerührt. Dann werden 3 ml Morpholin zugegeben und bis zur vollständigen Umsetzung weitergerührt. Man verdünnt mit Chloroform, wäscht mit 1N-Salzsäure, 10-proz. Kaliumbicarbonatlösung und gesättigter Kochsalzlösung, trocknet über Magnesiumsulfat und engt ein. Durch Kristallisieren aus Aethanol erhält man reines (4,5-Dihydro-7-oxo-8-phenyl-7H- thieno[2,3-a]chinolizin-10-yl)methyl 4-morpholincarboxylat vom Smp. 163-164°.

In analoger Weise erhält man:

b) Aus 4,5-Dihydro-10-(hydroxymethyl)-8-phenyl-7H- thieno[2,3-a]chinolizin-7-on und N-Methylpiperazin (4,5- Dihydro-7-oxo-8-phenyl -7H-thieno[2,3-a]chinolizin-10-yl)methyl-4-methyl-1-piperazincarboxylat;

c) aus 4,5-Dihydro-10-(hydroxymethyl) -8-phenyl-7H- thieno[2,3-a]chinolizin-7-on und 2,6-Dimethylmorpholin das 4,5-Dihydro-7-oxo-8-phenyl-7H-thieno[2,3-a]chinolizin-10-yl)methyl-2,6-dimethyl-4-morpholincarboxylat vom Smp. 156-157°;

d) aus 4,5-Dihydro-10-(hydroxymethyl) -8-phenyl-7H- thieno[2,3-a]chinolizin-7-on und 3-Hydroxypyrrolidin das 4,5-Dihydro-7-oxo-8-phenyl -7H-thieno[2,3-a]chinolizin-10-yl)methyl-3-hydroxy -1-pyrrolidincarboxylat vom Smp. 161-162°;

e) aus 6,7-Dihydro-1-(hydroxymethyl)-3-phenyl -10-chlor- 4H-benzo[a]chinolizin-4-on und 2,6-Dimethylmorpholin das (10-Chlor-6,7-dihydro-4-oxo -3-phenyl-4H-benzo[a]chinolizin-1- yl)methyl -2,6-dimethyl-4-morpholincarboxylat vom Smp. 159,5-161,5°;

f) aus 6,7-Dihydro-1-(hydroxymethyl) -3-phenyl-10-chlor- 4H-benzo[a]chinolizin-4-on und Aminoacetaldehyd- dimethylacetal das [10-Chlor-6,7-dihydro -4-oxo-3-phenyl-4H- benzo[a]chinolizin -1-yl]methyl-(2,2-dimethoxyäthyl)carbamat, M.S.: 468 (M);

g) aus 6,7-Dihydro-1-(hydroxymethyl) -3-phenyl-10-chlor- 4H-benzo[a]chinolizin-4-on und Aethyl-4-piperidincarboxylat das 1-[10-Chlor-6,7-dihydro -4-oxo-4-phenyl-4H-benzo- [a]chinolizin-1-yl]methyl - 4-äthyl-1,4-piperidindicarboxylat vom Smp. 122,5-123,5°;

h) aus 6,7-Dihydro-1-(hydroxymethyl) -3-phenyl-10- chlor-4H-benzo[a]chinolizin-4-on und 3-pyrrolidinol das (10- Chlor-6,7-dihydro-4-oxo-3-phenyl -4H-benzo[a]chinolizin-1-yl)methyl-3-hydroxy-1-pyrrolidincarboxylat vom Smp. 224-225°;

i) aus 6,7-Dihydro-1-hydroxymethyl) -3-phenyl-10-chlor- 4H-benzo[a]chinolizin-4-on und Aminoacetonitril das (10- Chlor-6,7-dihydro-4-oxo-3-phenyl -4H-benzo[a]chinolizin-1-yl)methyl-(cyanomethyl)-carbamat vom Smp. 166-167°;

j) aus 6,7-Dihydro-1-(hydroxymethyl) -3-phenyl-10-chlor- 4H-benzo[a]chinolizin -4-on und (S)-2-(hydroxymethyl)- pyrrolidin das (10-Chlor-6,7-dihydro -4-oxo-3-phenyl-4H- benzo[a]chinolizin-1-yl)-methyl-(S)-2-(hydroxymethyl)- pyrrolidincarboxylat, M.S.: 464 (M);

k) aus 6,7-Dihydro-1-(hydroxymethyl) -3-phenyl-10-chlor- 4H-benzo[a]chinolizin-4-on und 1,4-Dioxa-8-azaspiro[4,5]decan das (10-Chlor-6,7-dihydro -4-oxo-3-phenyl-4H- benzo[a]chinolizin -1-yl)methyl-1,4-dioxa-8-azaspiro[4,5]decan -8-carboxylat vom Smp. 142-143.

l) aus 10-Chlor-6,7-dihydro-1-(hydroxymethyl)-3- phenyl -4H-benzo[a]chinolizin-4-on und (R)-Prolinmethylester das 1-[(10-Chlor-6,7-dihydro-4-oxo-3-phenyl-4H- benzo[a]chinolizin-1-yl)methyl]-2-methyl-(R) -1,2- pyrrolidindicarboxylat M.S.: 492 ($M^+$).

Beispiel 44

Ein Gemisch aus 0,855 g 5,6-Dihydro-3-hydroxy-2- phenylthiazolo[3,2-a]thieno[2,3-c] pyridinium-hydroxid (inneres Salz) und 0,65 ml 3,3-Diäthoxypropin in 30 ml Cyclohexanon wird mit einigen Kristallen p-Toluolsulfonsäure 3 Stunden lang bei 130° unter Inertgas gerührt und dann im Vakuum eingeengt. Der Rückstand wird in Essigsäure- äthylester aufgenommen, mit 10-proz. Kaliumbicarbonatlösung und gesättigter Kochsalzlösung gewaschen, über Magnesiumsulfat getrocknet, eingeengt und an Kieselgel mit Toluol/Acetonitril (19:1) chromatographiert. Durch Kristallisieren aus Dioxan erhält man 4,5- Dihydro-7-oxo-8-phenyl -7H-thieno[2,3-a]chinolizin-10- carboxaldehyd vom Smp. 213-215°.

Beispiel 45

a) Eine Lösung von 100 mg 1-[(4,5-Dihydro-7-oxo-8- phenyl-7H-thieno[2,3-a]chinolizin-10-yl)-carbonyl]-4- piperidinol in 5 ml Methylenchlorid wird mit 51 mg Pyridiniumchlorochromat versetzt. Nach beendeter Reaktion wird das Lösungsmittel im Vakuum eingedampft und der Rückstand über Kieselgel

chromatographiert. Man kristallisiert das Produkt aus Diäthyläther um und erhält 1-[(4,5-Dihydro-7-oxo-8-phenyl-7H-thieno[2,3- a]chinolizin-10-yl)carbonyl]-4-piperidinon vom Smp. 218-220°.

In analoger Weise erhält man:

b) Aus 1-[[10-Chlor-6,7-dihydro -4-oxo-3-phenyl-4H- benzo[a]chinolizin-1-yl]carbonyl] -4-piperidinol das 1-[[10- Chlor-6,7-dihydro-4-oxo -3-phenyl-4H-benzo[a]chinolizin-1- yl]carbonyl] -4-piperidinon vom Smp. 164-166°;

c) aus 1-[[10-Chlor-6,7-dihydro -4-oxo-3-phenyl-4H- benzo[a]chinolizin -1-yl]carbonyl]-3-pyrrolidinol das 1-[(10- Chlor-4-oxo-3-phenyl-4H-benzo[a]chinolizin-1-yl)carbonyl]-3- pyrrolidinon vom Smp. 196-198°.

Beispiel 46

In Analogie zu den Angaben in Beispiel 7a) erhält man:

a) Aus 3-Hydroxy-1-[(7-oxo-8-phenyl-7H-thieno[2,3- a]chinolizin -10-yl)carbonyl]pyrrolidin und Methyljodid das 3- Methoxy-1-[(7-oxo-8-phenyl-7H-thieno[2,3-a]chinolizin -10-yl)carbonyl]pyrrolidin vom Smp. 179-181°;

b) aus (S)-1-[7-Oxo-8-phenyl-7H-thieno[2,3-a]chinolizin -10-yl)carbonyl]-2-pyrrolidin-methanol und Methyljodid das (S)-2- Methoxymethyl-1-[(7-oxo-8-phenyl -7H-thieno[2,3- a]chinolizin-10-yl)carbonyl]-pyrrolidin vom Smp. 153-155°;

c) aus 1-[(7-Oxo-8-phenyl-7H-thieno[2,3-a]chinolizin -10- yl)carbonyl]-4-piperidinol und Methyljodid das 4-Methoxy-1-[(7- oxo-8-phenyl -7H-thieno[2,3-a]chinolizin-10-yl)carbonyl]piperidin vom Smp. 200-203°;

d) aus N-(3-Hydroxypropyl)-7-oxo-8-phenyl-7H-thieno[2,3- a]chinolizin-10-carboxamid und ungefähr 2 Moläquivalenten NaH und Methyljodid das N-(3-Methoxypropyl)-N-methyl-7- oxo-8-phenyl-7H-thieno[2,3-a]chinolizin-10-carboxamid vom Smp. 161-163°;

e) aus 6,7-Dihydro-1-(hydroxymethyl)-3-phenyl-10-chlor-4H- benzo[a]chinolizin-4-on und Methyljodid das 10-Chlor-6,7- dihydro-1-(methoxymethyl)-3-phenyl -4H-benzo[a]chinolizin-4-on vom Smp. 154-155°;

f) aus (R)-1-[(7-Oxo -8-phenyl-7H-thieno[2,3- a]chinolizin-10-yl)carbonyl] -2-pyrrolidin-methanol und Methyljodid das (R)-2-Methoxymethyl-1-[(7-oxo-8-phenyl -7H- thieno[2,3-a]chinolizin-10-yl)carbonyl]-pyrrolidin vom Smp. 153-155°;

g) aus (R)-1-[(7-Oxo-8-phenyl-7H-thieno[2,3- a]chinolizin -10-yl)carbonyl]-3-pyrrolidinol und Methyljodid das (R)-3- Methoxy-1-[(7-oxo-8-phenyl -7H-thieno[2,3-a]chinolizin-10-yl)carbonyl]pyrrolidin vom Smp. 162-165°;

h) aus (S)-1-[(7-Oxo-8-phenyl-7H-thieno[2,3- a]chinolizin-10-yl)carbonyl]-3-pyrrolidinol und Methyljodid das (S)-3-Methoxy-1-[(7-oxo-8-phenyl -7H-thieno[2,3-a]chinolizin- 10-yl)carbonyl]pyrrolidin vom Smp. 163-165°;

i) aus 1-[(10-Chlor-4-oxo-3-phenyl-4H- benzo[a]chinolizin-1-yl)carbonyl] -3-pyrrolidinol und Methyliodid das 1-[(10-Chlor-4-oxo-3-phenyl-4H- benzo[a]chinolizin-1-yl)carbonyl]-3-methoxypyrrolidin vom Smp. 176-178°;

j) aus (S)-1-[(10-Chlor-4-oxo-3-phenyl -4H- benzo[a]chinolizin-1-yl)carbonyl] -2-pyrrolidin-methanol und Methyljodid das (S)-1-[(10-Chlor-4-oxo-3-phenyl-4H- benzo[a]chinolizin -1-yl)carbonyl]-2-(methoxymethyl)pyrrolidin vom Smp. 137-139°;

k) aus (R)-1-[(10-Chlor-4-oxo-3-phenyl-4H-benzo[a]chinolizin-1-yl)carbonyl]-2-pyrrolidin-methanol und Methyljodid das (R)-1-[(10-Chlor-4-oxo-3-phenyl -4H- benzo[a]chinolizin-1-yl)carbonyl] -2-(methoxymethyl)pyrrolidin vom Smp. 137-140°;

l) aus 1-[(7-Oxo-8-phenyl-7H-thieno[2,3-a]chinolizin -10- yl)carbonyl]-3-azetidinol und Methyljodid das 3-Methoxy- 1-[(7-oxo-8-phenyl -7H-thieno[2,3-a]chinolizin-10-yl)carbonyl]azetidin vom Smp. 212-213°;

m) aus 1-[(7-Oxo-8-phenyl-7H-thieno[2,3-a]chinolizin -10- yl)carbonyl]-3-piperidinol und Methyljodid das 3-Methoxy-1-[(7- oxo-8-phenyl -7H-thieno[2,3-a]chinolizin-10-yl)carbonyl]piperidin vom Smp. 180-181°;

n) aus 7-Oxo-8-phenyl-N-(tetrahydro -2-furfuryl)-7H-thieno[2,3-a]chinolizin-10-carboxamid und Methyljodid das N-Methyl-7-oxo-8-phenyl-N-(tetrahydro-2-furfuryl)-7H-thieno[2,3-a]chinolizin-10-carboxamid vom Smp. 168–169°;

o) aus N-(2-Methoxyäthyl)-7-oxo-8-phenyl-7H-thieno[2,3-a]chinolizin-10-carboxamid und Äthyljodid das N-Äthyl-N-(2-methoxyäthyl)-7-oxo-8-phenyl-7H-thieno[2,3-a]chinolizin-10-carboxamid vom Smp. 141–143°;

p) aus N-(2-Hydroxyäthyl)-7-oxo-8-phenyl-7H-thieno[2,3-a]chinolizin-10-carboxamid und ca. 3 Äquivalenten Methyljodid das N-(2-Methoxyäthyl)-N-Methyl-7-oxo-8-phenyl-7H-thieno[2,3-a]chinolizin-10-carboxamid vom Smp. 126–128°;

q) aus 1-[(10-Chlor-1-oxo-3-phenyl-4H-benzo[a]chinolizin-1-yl)carbonyl]-3-azetidinol und Methyljodid das 1-[(10-Chlor-4-oxo-3-phenyl-4H-benzo[a]chinolizin-1-yl)-carbonyl]-3-methoxyazetidin vom Smp. 175–177°;

r) aus (S)-1-[(7-Oxo-8-phenyl-7H-thieno[2,3-a]chinolizin-10-yl)carbonyl]-2-azetidinmethanol und Methyljodid das (S)-2-(Methoxymethyl)-1-[(7-oxo-8-phenyl-7H-thieno[2,3-a]chinolizin-10-yl)carbonyl]azetidin vom Smp. 95–100°.

Beispiel 47

In Analogie zu den Angaben in Beispiel 32ba) erhält man:

a) Aus 7-Oxo-8-phenyl-7H-thieno[2,3-a]chinolizin-10-carbonsäure und N-Methylpiperazin das 1-Methyl-4-[(7-oxo-8-phenyl-7H-thieno[2,3-a]chinolizin -10-yl)carbonyl]piperazin vom Smp. 233-234°;

b) aus 7-Oxo-8-phenyl-7H-thieno[2,3-a]chinolizin-10- carbonsäure und 3-Hydroxypyrrolidin das 3-Hydroxy-1-[(7- oxo-8-phenyl-7H-thieno[2,3-a]chinolizin -10-yl)carbonyl]pyrrolidin vom Smp. 254-257°;

c) aus 7-Oxo-8-phenyl-7H-thieno[2,3-a]chinolizin-10- carbonsäure und (S)-2-Pyrrolidinmethanol das (S)-1-[7-Oxo-8- phenyl-7H-thieno[2,3-a]chinolizin -10l-yl)carbonyl]-2- pyrrolidin-methanol vom Smp. 140-150° (Zers.);

d) aus 7-Oxo-8-phenyl-7H-thieno[2,3-a]chinolizin-10- carbonsäure und 2-Methoxyäthylamin das N-(2-Methoxyäthyl)- 7-oxo-8-phenyl-7H-thieno[2,3-a]chinolizin -10-carboxamid vom Smp. 201-203°;

e) aus 7-Oxo-8-phenyl-7H-thieno[2,3-a]chinolizin-10- carbonsäure und cis-2,6-Dimethylmorpholin das cis-2,6-Dimethyl- 4-[(7-oxo-8-phenyl -7H-thieno[2,3-a]chinolizin-10- yl)carbonyl]morpholin vom Smp. >280°;

f) aus 7-Oxo-8-phenyl-7H-thieno[2,3-a]chinolizin- 10-carbonsäure und 4-Piperidinol das 1-[(7-Oxo-8-phenyl-7H- thieno[2,3-a]chinolizin -10-yl)carbonyl]-4-piperidinol vom Smp. 241-245° (Zers.);

g) aus 7-Oxo-8-phenyl-7H-thieno[2,3-a]chinolizin-10- carbonsäure und 2-Aminoäthanol das N-(2-Hydroxyäthyl)-7-oxo-8-phenyl -7H-thieno[2,3-a]chinolizin-10- carboxamid vom Smp. 219-220°;

h) aus 7-Oxo-8-phenyl-7H-thieno[2,3-a]chinolizin-10- carbonsäure und 2-(Aethylamino)äthanol das N-Aethyl-N-(2- hydroxyäthyl)-7-oxo-8-phenyl -7H-thieno[2,3- a]chinolizin-10-carboxamid vom Smp. 208-210°;

i) aus 7-Oxo-8-phenyl-7H-thieno[2,3-a]chinolizin-10-carbonsäure und 3-Amino-2-propanol das N-(3-Hydroxypropyl)-7-oxo-8-phenyl-7H-thieno[2,3-a]chinolizin-10-carboxamid vom Smp. 228–230°;

j) aus 7-Oxo-8-phenyl-7H-thieno[2,3-a]chinolizin-10-carbonsäure und 2-(Methylamino)äthanol das N-(2-Hydroxyäthyl)-N-methyl-7-oxo-8-phenyl-7H-thieno[2,3-a]chinolizin-10-carboxamid vom Smp. 231–233°;

k) aus 7-Oxo-8-phenyl-7H-thieno[2,3-a]chinolizin-10-carbonsäure und N-Äthylpiperazin das 1-Äthyl-4-[(7-oxo-8-phenyl-7H-thieno[2,3-a]chinolizin-10-yl)carbonyl]piperazin vom Smp. 214–215° (Zers.);

m) aus 7-Oxo-8-phenyl-7H-thieno[2,3-a]chinolizin-10-carbonsäure und (2S,4R)-4-Hydroxy-pyrrolidinmethanol das (2S,4R)-1-[(7-Oxo-8-phenyl-7H-thieno[2,3-a]chinolizin-10-yl)carbonyl]-4-hydroxy-2-pyrrolidinmethanol vom Smp. 154–161°.

Beispiel 48

0,64 ml Dimethylsulfoxid werden in 12 ml Methylenchlorid gelöst und unter Argon auf –70° abgekühlt. Es werden 0,94 ml Trifluoressigsäureanhydrid in 2,2 ml Methylenchlorid tropfenweise zugesetzt. Nach 10 Minuten werden portionenweise 1,75 g 3-Hydroxy-1-[(7-oxo-8-phenyl-7H-thieno[2,3-a]chinolizin-10-yl)carbonyl]pyrrolidin zugegeben. Nach 10 Minuten läßt man auf Raumtemperatur aufwärmen. Nach Zusatz von 10 ml Methylenchlorid wird tropfenweise mit 1,87 ml Triäthylamin versetzt. Wenig unlösliches Material wird durch Filtration entfernt, und das Filtrat wird mehrmals mit Wasser gewaschen. Nach Trocknen mit Natriumsulfat wird filtriert und eingedampft. Nach Chromatographieren an Kieselgel wird aus Aethanol/N,N-Dimethylformamid umkristallisiert. Es werden 1-[(7-Oxo-8-phenyl-7H-thieno[2,3-a]chinazolin -10-yl)carbonyl]pyrrolidin-3-on als gelbe Kristalle vom Smp. 256-261° (Zers.) erhalten.

Beispiel 49

a) 6,3 g 1-[(7-Oxo-8-phenyl-7H-thieno[2,3- a]chinolizin -10-yl)carbonyl]-3-pyrrolidinol werden in 80 ml N,N- Dimethylformamid suspendiert und unter Argon auf 4° abgekühlt. Dann gibt man hintereinander 2,6 ml Aethyljodid und 1,52 g pulverisiertes Kaliumhydroxid (Gehalt ca. 90%) zu, entfernt das Kühlbad und rührt 3 1/2 Stunden bei Raumtemperatur. Nach erneutem Zugeben der gleichen Mengen an Reagenzien wird bis zur Beendigung der Reaktion weitergerührt und auf 800 ml 10 proz. Kochsalzlösung, welche 25 ml 1N- Salzsäure enthält, gegossen. Die gelben Kristalle werden abfiltriert und getrocknet. Nach Chromatographieren an Kieselgel und Umkristallisieren erhält man 3-Aethoxy-1-[(7- oxo-8-phenyl-7H-thieno[2,3-a]chinolizin -10-yl)carbonyl]pyrrolidin vom Smp. 167-170°.

In analoger Weise erhält man:

b) Aus 1-[(7-Oxo-8-phenyl-7H-thieno[2,3-a]chinolizin-10- yl)carbonyl]-3-pyrrolidinol und Propyljodid das 1-[(7-Oxo-8- phenyl-7H-thieno[2,3-a]chinolizin -10-yl)carbonyl]-3-propoxy- pyrrolidin vom Smp. 145-147°;

c) aus 2S,4R)-2-(Hydroxymethyl) -1-[(7-oxo-8- phenyl-7H-thieno[2,3-a]chinolizin -10-yl)carbonyl]-4-pyrrolidinol und Methyljodid das (2S,4R)-1-[(7-Oxo-8-phenyl-7H- thieno[2,3-a]chinolizin -10-yl)carbonyl]-4-methoxy-2- (methoxymethyl)pyrrolidin vom Smp. 166-174°;

d) aus 1-[(7-Oxo-8-phenyl-7H-thieno[2,3-a]chinolizin-10- yl)carbonyl]-3-pyrrolidinol und Isopropyljodid das 3-Isopropoxy- 1-[(7-oxo-8-phenyl -7H-thieno[2,3-a]chinolizin-10-yl)carbonyl]pyrrolidin vom Smp. 116-119°;

e) aus -N-(trans-2-Hydroxycyclohexyl)-7-oxo -8-phenyl-7H- -thieno[2,3-a]chinolizin-10-carboxamid und Methyljodid das N- (trans-2-Methoxycyclohexyl)-N-methyl-7-oxo -8-phenyl-7H- thieno[2,3-a]chinolizin-10-carboxamid vom Smp. 204-205°;

f) aus 2-(Hydroxymethyl)-1-[(7-oxo-8-phenyl -7H-thieno[2,3-a]chinolizin-10-yl)carbonyl]piperidin und Methyljodid das 2- (Methoxymethyl)-1-[(7-oxo-8-phenyl -7H-thieno[2,3-a]chinolizin- 10-yl)carbonyl]-piperidin vom Smp. 204-207°;

g) aus (S)-1-[(7-Oxo-8-phenyl-7H-thieno[2,3- a]chinolizin -10-yl)carbonyl]-2-azetidinmethanol und Methyljodid das (S)-2-(Methoxymethyl)-1-[(7-oxo-8-phenyl -7H-thieno[2,3- a]chinolizin-10-yl)carbonyl]-azetidin vom Smp. 95-100°.

Beispiel 50

a) 32 g 10-Chlor-6,7-dihydro-4-oxo-3-phenyl -4H- benzo[a]chinolizin-1-carbonsäure-methylester werden in 350 ml Tetrachlorkohlenstoff mit 16 g N-Bromsuccinimid in Gegenwart von 280 mg Dibenzoylperoxid während 16 Stunden unter Rückfluss erhitzt. Man lässt abkühlen und gibt 24,3 ml Triäthylamin dazu. Dann wird erneut während 3 Stunden unter Rückfluss erhitzt. Nach Abkühlen wird mit ca. 900 ml Methylenchlorid versetzt, und die organische Phase wird einmal mit 550 ml 1N-Salzsäure gewaschen und danach über Natriumsulfat getrocknet, filtriert und eingedampft. Der Rückstand wird durch Chromatographieren an Kieselgel gereinigt und aus Essigester umkristallisiert. Man erhält 10- Chlor-4-oxo-3-phenyl -4H-benzo[a]chinolizin-1-carbonsäure- methylester als gelbe Kristalle vom Smp. 169-170°.

b) In analoger Weise erhält man aus 1-(10-Chlor-6,7- dihydro-4-oxo-3-phenyl -4H-benzo[a]chinolizin-1-yl)-4- methoxypiperidin das 1-[(10-Chlor-3-phenyl-4-oxo-4H- benzo[a]chinolizin -1-yl)carbonyl]-4-methoxypiperidin vom Smp. 158-162°.

Beispiel 51

In Analogie zu den Angaben in Beispiel 49 erhält man:

a) Aus 1-[(10-Chlor-6,7-dihydro-4-oxo-3-phenyl -4H- benzo[a]chinolizin-1-yl)carbonyl]-4-piperidinol und Methyljodid das 1-[(10-Chlor-6,7-dihydro-4-oxo-3-phenyl -4H- benzo[a]chinolizin-1-yl)carbonyl]-4-methoxypiperidin vom Smp. 152-153°;

b) aus 1-[(10-Chlor-6,7-dihydro-4-oxo-3-phenyl -4H- benzo[a]chinolizin-1-yl)carbonyl]-3-pyrrolidinol und Methyljodid das 1-[(10-Chlor-6,7-dihydro-4-oxo-3-phenyl -4H- benzo[a]chinolizin-1-yl)carbonyl]-3-methoxypyrrolidin vom Smp. 96-98°;

c) aus 1-[(10-Chlor-6,7-dihydro-4-oxo-3-phenyl -4H- benzo[a]chinolizin-1-yl)carbonyl]-3-pyrrolidinol und Aethyljodid das 1-[(10-Chlor-6,7-dihydro-4-oxo-3-phenyl -4H- benzo[a]chinolizin-1-yl)carbonyl]-3-äthoxypyrrolidin vom Smp. 133-136°;

d) aus (S)-1-[[10-Chlor-6,7-dihydro-4-oxo-3-phenyl -4H- benzo[a]chinolizin-1-yl]carbonyl]-2-pyrrolidinmethanol und Methyljodid das (S)-1-[(10-Chlor-6,7-dihydro-4-oxo-3- phenyl -4H-benzo[a]chinolizin-1-yl)carbonyl] -2-(methoxymethyl)pyrrolidin vom Smp. 133-135°;

e) aus 1-[(4,5-Dihydro-7-oxo-8-phenyl -7H-thieno[2,3- a]chinolizin-10-yl)carbonyl]-3-pyrrolidinol und Methyljodid das 1- [(4,5-Dihydro-7-oxo-8-phenyl -7H-thieno[2,3- a]chinolizin-10-yl)carbonyl] -3-methoxypyrrolidin vom Smp. 155-156°;

f) aus (S)-1-[(4,5-Dihydro-7-oxo-8-phenyl -7H-thieno[2,3- a]chinolizin-10-yl)carbonyl] -2-pyrrolidinmethanol und Methyljodid das (S)-2-(Methoxymethyl)-1-[(4,5-dihydro-7-oxo- 8-phenyl -7H-thieno[2,3-a]chinolizin-10- yl)carbonyl]pyrrolidin vom Smp. 129-131°;

g) aus (R)-1-[(10-Chlor-6,7-dihydro-4-oxo-3-phenyl -4H- benzo[a]chinolizin-1-yl)carbonyl]-2-pyrrolidinmethanol und Methyljodid das (R)-1-[(10-Chlor-6,7-dihydro-4-oxo-3- phenyl -4H-benzo[a]chinolizin-1-yl)carbonyl] -2-(methoxymethyl)pyrrolidin vom Smp. 136-138°;

h) aus 1-[(10-Chlor-6,7-dihydro-4-oxo-3-phenyl -4H- benzo[a]chinolizin-1-yl)carbonyl]-4-piperidinol und Aethyljodid das 1-(10-Chlor-6,7-dihydro-4-oxo-3-phenyl -4H- benzo[a]chinolizin-1-yl)-4-äthoxypiperidin vom Smp. 142-144°;

i) aus [(4,5-Dihydro-7-oxo-8-phenyl -7H-thieno[2,3- a]chinolizin-10-yl)methyl]-3-hydroxy -1-pyrrolidincarboxylat und Methyljodid das (4,5-Dihydro-7-oxo-8-phenyl -7H- thieno[2,3-a]chinolizin-10-yl)-methyl-3-methoxy -1-pyrrolidincarboxylat. M.S.: 436 (M).

Beispiel 52

Die in Beispiel 31bd beschriebene Verbindung kann auch wie folgt hergestellt werden:

0,6 g 3,4-Dihydro-thieno[2,3-f][1,4]thiazepin-5(2H)- thion und 1,0 g α-(Tosylhydrazono)phenylacetylchlorid werden in 50 ml Methylenchlorid längere Zeit bei Raumtemperatur und in Gegenwart von 0,84 ml

Triäthylamin gerührt. Das Lösungsmittel wird im Vakuum entfernt, und der Rückstand wird an Kieselgel chromatographiert. Man erhält 5,6-Dihydro-8-hydroxy-9-phenyl -thiazolo[3,2-d]thieno[2,3-f][1,4]thiazepinium-hydroxyd (inneres Salz) vom Smp. 190-195°.

Beispiel 53

70,8 mg 7-Hydroxy-8-phenylthiazolo[3;2-a]thieno[2,3-c]pyridinium-hydroxid (inneres Salz) und 38,2 mg N-(2- Methoxyäthyl)-2-propinamid werden längere Zeit unter Argon in Toluol erhitzt. Das Lösungsmittel wird im Vakuum entfernt, und der Rückstand wird an Kieselgel chromtographiert. Nach Umkristallisieren aus Essigsäureäthylester erhält man N-(2-Methoxyäthyl)-7-oxo-8-phenyl -7H-thieno[2,3-a]-chinolizin-10-carboxamid als gelbe Kristalle vom Smp. 201-202°.

Beispiel 54

363 mg 10-Chlor-4-oxo-3-phenyl-4H-benzo[a]chinolizin-1- carbonsäure-methylester werden in Gegenwart von 36 mg 10-proz. Palladium/Kohle in 50 ml Essigester bei Raumtemperatur hydriert. Der Katalysator wird abfiltriert, und das Lösungsmittel wird im Vakuum entfernt. Der Rückstand wird durch Chromatographieren an Kieselgel gereinigt und das Produkt mit Methanol kristallisiert. Man erhält 10-Chlor-6,7-dihydro-4-oxo-3-phenyl-4H-benzo[a]chinolizin -1-carbonsäure-methylester vom Smp. 138-139°.

Beispiel 55

432 mg (R)-1-[(10-Chlor-4-oxo-3-phenyl-4H- benzo[a]chinolizin -1-yl)carbonyl]-2-pyrrolidinmethanol, 4 mg Tetrabutylammoniumjodid in 2 ml Methylenchlorid werden mit 104 mg Natriumhydroxid in 0,1 ml Wasser während 30 Minuten im Ultraschallbad gehalten. Dann werden 0,19 ml Dimethylsulfat zugegeben. Nach ca. 1 Stunde werden nochmals 0,19 ml Dimethylsulfat zugegeben, und man lässt weitere 90 Minuten reagieren. Es wird mit Methylenchlorid verdünnt, mit Wasser gewaschen, über Natriumsulfat getrocknet, filtriert und eingedampft. Der Rückstand wird an Kieselgel chromatographiert, und das Produkt wird aus Cyclohexan/Aethanol umkristallisiert. M an erhält (R)-1-[(10-Chlor-4-oxo-3- phenyl-4H-benzo-[a]chinolizin -1-yl)carbonyl]-2-methoxymethyl- pyrrolidin als gelbe Kristalle vom Smp. 137-140°.

Beispiel 56

aa) 26, 2 g 3-Trifluormethylphenylessigsäure-methylester werden in 100 ml Diäthyläther gelöst und nach Zugabe von 8,2 ml Ameisensäure-methylester mit 2,76 g Natrium versetzt. Es wird bis zur Beendigung der Reaktion bei Raumtemperatur gerührt und unter Kühlung mit 1N Salzsäure versetzt. Die organische Phase wird abgetrennt und die Wasserphase noch zweimal mit Aether extrahiert. Die vereinigten organischen Phasen werden mit gesättigter Kochsalzlösung gewaschen, über Natriumsulfat getrocknet, filtriert und eingedampft. Destillation liefert 3-Hydroxy-2- (α,α,α-trifluor -m-tolyl)acrylsäure-methylester als farbloses Oel vom Sdp. 59-62°/0,2 mm.

In analoger Weise erhält man:

ab) Aus m-Fluorphenylessigsäure-methylester und Ameisensäure-methylester den 2-(m-Fluorphenyl)-3-hydroxy- acrylsäure-methylester vom Sdp. 75-81°/0,1 mm.

ac) aus p-Fluorphenylessigsäure-methylester und Ameisensäure-methylester den 2-(p-Fluorphenyl)-3-hydroxy- acrylsäure-methylester vom Sdp. 65-70°/0,1 mm;

ad) aus p-Chlorphenylessigsäure-methylester und Ameisensäure-methylester den 2-(p-Chlorphenyl)-3- hydroxy-acrylsäure-methylester vom Smp. 89-97°.

ba) 26 g 3-Hydroxy-2-(α,α,α-trifluor -m-tolyl)acrylsäure- methylester werden unter Kühlung im Eisbad in 1N Natronlauge gelöst und mit 10,6 ml Dimethylsulfat versetzt. Es wird ca. 40 Stunden bei etwa 0° gerührt und nochmals 40 ml 1N Natronlauge und 3,8 ml Dimethylsulfat zugesetzt. Nach beendeter Reaktion wird mit Wasser verdünnt und mit Methylenchlorid extrahiert. Nach Trocknen mit Natriumsulfat wird destilliert, wobei 3-Methoxy-2- (α,α,α-trifluor -m-tolyl)acrylsäure-methylester als farbloses Oel vom Sdp. 75-90°/0,2 mm erhalten wird.

In analoger Weise erhält man:

bb) aus 2-(m-Fluorphenyl)-3-hydroxy-acrylsäure- methylester und Dimethylsulfat den 2-(m-Fluorphenyl)-3- methoxy-acrylsäure-methylester vom Sdp. 80-100°/0,1 mm;

bc) aus 2-(p-Fluorphenyl)-3-hydroxy-acrylsäure- methylester und Dimethylsulfat den 2-(p-Fluorphenyl)-3-methoxy- acrylsäure-methylester vom Smp. 70-76°;

bd) aus 2-(p-Chlorphenyl)-3-hydroxy-acrylsäure- methylester und Dimethylsulfat den 2-(p-Chlorphenyl)-3-methoxy- acrylsäure-methylester vom Smp. 53-58°.

ca) 8,05 g 3,4-Dihydro-thieno[2,3-f][1,4]thiazepin-5(2H)- thion werden in 40 ml Methylenchlorid suspendiert und unter Argon mit einer Lösung von 7,1 ml Brommalonsäure- diäthylester in 40 ml Methylenchlorid versetzt. Nach 2,5- stündigem Rühren werden langsam 50 ml 10-proz. Kaliumhydrogencarbonatlösung hinzugegeben und 30 Minuten gerührt. Die organische Phase wird abgetrennt und die Wasserphase noch zweimal mit Methylenchlorid extrahiert. Nach Trocknen mit Natriumsulfat wird an Kieselgel

chromatographiert, wobei Diäthyl [3,4-Dihydrothieno[2,3-f][1,4]thiazepin -8(5H)- yliden]malonat in Form von schwach gelblichen Kristallen vom Smp. 87-89°(Cyclohexan) erhalten werden.

cb) 2,56 g Diäthyl [3,4-Dihydrothieno[2,3-f][1,4]thiazepin-8(5H)-yliden]malonat werden in 15,6 ml Aethanol suspendiert und nach Zugabe von 4,3 ml 2N äthanolischer Natriummethylatlösung bis zur Beendigung der Reaktion zum Rückfluss erhitzt. Das Lösungsmittel wird im Vakuum entfernt und der Rückstand in 60 ml Wasser aufgenommen und mehrmals mit Methylenchlorid extrahiert. Nach Waschen der organischen Phasen mit Wasser und Trocknung mit Natriumsulfat wird an Kieselgel chromatographiert. Nach Umkristallisation erhält man Aethyl [6,7-Dihydrothieno[2,3- f][1,4]thiazepin-8(5H)-yliden]acetat in Form von gelblichen Kristallen, Smp. 86-87° (Aethanol).

d) 255 mg Aethyl [6,7-Dihydrothieno[2,3-f][1,4]thiazepin-8(5H)-yliden]acetat werden unter Argon in 3 ml Tetrahydrofuran bei 2-3° mit 44 mg ca. 55-proz. Natriumhydrid- Dispersion portionenweise versetzt und während 20 Minuten gerührt. Dazu gibt man eine Lösung von 3-Methoxy- 2-(α,α,α-trifluor -m-tolyl)acrylsäure-methylester in 2 ml Tetrahydrofuran. Das Kühlbad wird entfernt und die Lösung 17 Stunden bei Raumtemperatur gerührt, dann 2 Stunden auf 45-50° und schliesslich 6 Stunden zum Rückfluss erhitzt. Die Lösung wird mit ätherischer Salzsäure-Lösung angesäuert und nach Eindampfen an Kieselgel chromatographiert. Das Gemisch von Methyl- und Aethylester wird nun in 6 ml Methanol gelöst und 0,14 ml 1N Natriummethylatlösung werden zugesetzt. Es wird ca. 20 Stunden bei Raumtemperatur gerührt, mit methanolischer Salzsäure angesäuert und eingedampft. Der Rückstand wird durch Umkristallisation gereinigt, wobei man 5,6-Dihydro- 8-oxo-9-(α,α,α-trifluor-m-tolyl)-8H -pyrido[1,2- d]thieno[2,3-f][1,4]thiazepin-11- carbonsäuremethylester als gelbliche Kristalle erhält, Smp. 190-191° (Essigester).

Beispiel 57

In Analogie zu den Angaben in Beispiel 32ba erhält man:

a) Aus 7-Oxo-8-phenyl-7H-thieno[2,3-a]chinolizin -10- carbonsäure und (R)-2-Pyrrolidinmethanol das (R)-1-[(7-Oxo-8- phenyl-7H-thieno[2,3-a]chinolizin -10-yl)carbonyl]-2- pyrrolidin-methanol vom Smp. 146-154°;

b) aus 7-Oxo-8-phenyl-7H-thieno[2,3-a]chinolizin -10- carbonsäure und 1-(2-Methoxyäthyl)-piperazin das 4-(2- Methoxyäthyl)-1-[(7-oxo-8-phenyl -7H-thieno[2,3-a]chinolizin-10- yl)carbonyl]piperazin vom Smp. 166-168°;

c) aus 7-Oxo-8-phenyl-7H-thieno[2,3-a]chinolizin -10-carbonsäure und (R)-3-Hydroxypyrrolidin das (R)-1-[(7- Oxo-8-phenyl-7H-thieno[2,3-a]chinolizin -10-yl)carbonyl]-3- pyrrolidinol vom Smp. 237-239°;

d) aus 7-Oxo-8-phenyl-7H-thieno[2,3-a]chinolizin -10-carbonsäure und (S)-3-Hydroxypyrrolidin das (S)-1-[(7- Oxo-8-phenyl-7H-thieno[2,3-a]chinolizin -10-yl)carbonyl]-3- pyrrolidinol vom Smp. 237-239°;

e) aus 7-Oxo-8-phenyl-7H-thieno[2,3-a]chinolizin -10- carbonsäure und 3-Hydroxyazetidin das 1-[(7-Oxo-8-phenyl- 7H-thieno[2,3-a]chinolizin -10-yl)carbonyl]-3-azetidinol vom Smp. 240-241°;

f) aus 7-Oxo-8-phenyl-7H-thieno[2,3-a]chinolizin -10- carbonsäure und 3-Hydroxypiperidin das 1-[(7-Oxo-8-phenyl-7H- thieno[2,3-a]chinolizin -10-yl)carbonyl]-3-piperidinol vom Smp. 279-280°;

g) aus 7-Oxo-8-phenyl-7H-thieno[2,3-a]chinolizin -10- carbonsäure und 2-(Hydroxymethyl)-piperidin das 2-(Hydroxymethyl)- 1-[(7-oxo-8-phenyl -7H-thieno[2,3-a]chinolizin-10-yl)carbonyl]piperidin vom Smp. 256-258°;

h) aus 7-Oxo-8-phenyl-7H-thieno[2,3-a]chinolizin -10-carbonsäure und trans-2-Amino-cyclohexanol das N-(trans-2- Hydroxycyclohexyl)-7-oxo-8-phenyl -7H-thieno[2,3-a]chinolizin- 10-carboxamid vom Smp. 245-246°;

i) aus 10-Chlor-4-oxo-3-phenyl-4H-benzo[a]chinolizin -1- carbonsäure und (S)-2-Pyrrolidinmethanol das (S)-1-[(10- Chlor-4-oxo-3-phenyl-4H-benzo[a]chinolizin -1-yl)carbonyl]-2- pyrrolidinmethanol vom Smp. 178-180°;

j) aus 10-Chlor-4-oxo-3-phenyl-4H- benzo[a]chinolizin -1-carbonsäure und (R)-2- Pyrrolidinmethanol das (R)-1-[(10-Chlor-4-oxo-3-phenyl-4H- benzo[a]chinolizin -1-yl)carbonyl]-2-pyrrolidinmethanol vom Smp. 178-181°;

k) aus 7-Oxo-8-phenyl-7H-thieno[2,3-a]chinolizin -10-carbonsäure und Furfurylamin das 7-Oxo-8-phenyl-N-(2- furfuryl) -7H-thieno[2,3-a]chinolizin-10-carboxamid vom Smp. 187-188°;

l) aus 7-Oxo-8-phenyl-7H-thieno[2,3-a]chinolizin -10- carbonsäure und 2-Amino-2-thiazolin das 7-Oxo-8-phenyl-N- 2-thiazolin-2-yl -7H-thieno[2,3-a]chinolizin-10-carboxamid vom Smp. 172-174°;

m) aus 7-Oxo-8-phenyl-7H-thieno[2,3-a]chinolizin -10- carbonsäure und 2-Aminothiazol das 7-Oxo-8-phenyl-N-(2- thiazolyl)-7H-thieno[2,3-a]chinolizin -10-carboxamid vom Smp. 286-289°;

n) aus 7-Oxo-8-phenyl-7H-thieno[2,3-a]chinolizin -10- carbonsäure und 3-Chlorpropylamin-Hydrochlorid in Gegenwart eines zusätzlichen Aequivalentes Triäthylamin das N-(3- Chlorpropyl)-7-oxo-8-phenyl-7H-thieno[2,3-a]chinolizin -10-carboxamid vom Smp. 196-198°;

o) aus 7-Oxo-8-phenyl-7H-thieno[2,3-a]chinolizin -10- carbonsäure und 2-Fluoräthylamin-hydrochlorid in Gegenwart eines Ueberschusses von Triäthylamin das N-(2-Fluoräthyl)- 7-oxo-8-phenyl-7H-thieno[2,3-a]chinolizin -10-carboxamid vom Smp. 230-232°;

p) aus 7-Oxo-8-phenyl-7H-thieno[2,3-a]chinolizin -10-carbonsäure und 3-Methoxypropylamin das N-(3- Methoxypropyl)-7-oxo-8-phenyl -7H-thieno[2,3-a]chinolizin -10- carboxamid vom Smp. 188-189°;

q) aus 7-Oxo-8-phenyl-7H-thieno[2,3-a]chinolizin -10- carbonsäure und Bis-(2-Methoxäthyl)-amin das N,N-Bis- (2-methoxyäthyl)-7-oxo-8-phenyl -7H-thieno[2,3-a]chinolizin- 10-carboxamid vom Smp. 152°;

r) aus 7-Oxo-8-phenyl-7H-thieno[2,3-a]chinolizin -10- carbonsäure und N-(2-Methoxyäthyl)-anilin das N-(2- Methoxyäthyl)-7-oxo-8-phenyl -7H-thieno[2,3-a]chinolizin-10- carboxanilid vom Smp. 143-147°;

s) aus 10-Chlor-4-oxo-3-phenyl-4H-benzo[a]chinolizin -1- carbonsäure und 3-Hydroxy-azetidin das 1-[(10-Chlor-4-oxo-3- phenyl-4H-benzo[a]chinolizin -1-yl)carbonyl]-3-azetidinol vom Smp. 246-247°;

Beispiel 58

Eine Suspension von 0,676 g 6,7-Dihydro-1-(hydroxymethyl)-3-phenyl-10-chlor -4H-benzo[a]chinolizin-4-on in 18 ml Dioxan wird mit 0,6 ml Chlorameisensäurephenylester und 0,42 ml Pyridin versetzt und 3 Stunden bei Raumtemperatur gerührt. Dann werden 1,03 g Aminoacetonitril zugegeben und bei 100° weitergerührt, bis nach Dünnschichtchromatographie kein Carbonat mehr vorhanden ist. Nach Eindampfen wird in Chloroform aufgenommen, zweimal mit Wasser und gesättigter Kochsalzlösung gewaschen, über Magnesiumsulfat getrocknet und eingeengt. Chromatographie über Kieselgel [Laufmittel Toluol/Dioxan (9:1)] ergibt [[(10-Chlor-6,7- dihydro-4-oxo-3-phenyl -4H-benzo[a]chinolizin-1- yl)-methyl]amino]acetonitril vom Smp. 181-183° (aus Methanol) sowie in einer zweiten Fraktion (10-Chlor-6,7-dihydro-4-oxo-3-phenyl -4H-benzo[a]chinolizin-1-yl)methyl-(cyanomethyl)carbamat vom Smp. 166-167° (aus Toluol).

Beispiel 59

Eine Suspension von 0,376 g [[(10-Chlor-6,7-dihydro-4- oxo-3-phenyl -4H-benzo[a]chinolizin-1-yl)methyl]amino]ecetonitril in 10 ml Tetrahydrofuran wird mit 0,1 ml Ameisensäure-essigsäureanhydrid versetzt, und die nach kurzer Zeit gebildete klare gelbe Lösung wird 45 Minuten bei Raumtemperatur gerührt. Nach Eindampfen wird in Chloroform aufgenommen, mit 10-proz. Kaliumbicarbonat- und gesättigter Kochsalz-Lösung gewaschen, über Magnesiumsulfat getrocknet, eingeengt und durch Chromatographie an Kieselgel mit Toluol/Dioxan (9:1) gereinigt. N-[(10-Chlor-6,7-dihydro-4-oxo-3-phenyl -4H-benzo-[a]chinolizin-1-yl)-methyl]- N-(cyanomethyl)formamid bildet ein gelbes Harz.

Beispiel 60

a) 13,5 g Lithiumaluminiumhydrid werden bei ca. 0° in 550 ml absolutem Tetrahydofuran unter Argon suspendiert, und darauf wird innerhalb von etwa einer halben Stunde (S)-Azetidin-2- carbonsäuremethylester-hydrochlorid portionenweise zugegeben. Es wird 30 Minuten bei ca. 5°, dann 3 Stunden bei ca. 20° gerührt. Danach wird erneut abgekühlt und tropfenweise 60 ml Wasser zugefügt. Nach 3-tägigem Rühren bei Raumtemperatur wird der weisse Niederschlag abfiltriert und mit Chloroform erschöpfend extrahiert. Das Filtrat wird eingedampft und zusammen mit dem oben erhaltenen Extrakt im Vakuum destilliert. Man erhält (S)-2-Azetidinmethanol als farbloses Oel vom Sdp. 44-46°/0,06 Torr.

b) In Analogie zu den Angaben in Beispiel 32ba erhält man aus 7-Oxo-8-phenyl-7H-thieno[2,3-a]chinolizin-10-carbonsäure und (S)-2-Azetidinmethanol das (S)-1-[(7-Oxo-8-phenyl-7H-thieno[2,3-a]chinolizin-10-yl)carbonyl]-2-azetidinmethanol vom Smp. 158–161°.

<u>Beispiel A</u>

Die Verbindung A (10-Chlor-6,7-dihydro-N-(2-methoxyäthyl)-4-oxo-3-phenyl-4H-benzo[a]chinolizin-1-carboxamid) kann in an sich bekannter Weise als Wirkstoff zur Herstellung von pharmazeutischen Präparaten nachfolgender Zusammensetzung verwendet werden:

a) Tabletten

| | mg/Tablette |
|---|---|
| Verbindung A | 5 |
| Milchzucker | 135 |
| Maisstärke | 51 |
| Polyvinylpyrrolidin | 8 |
| Magnesiumstearat | 1 |
| Tablettengewicht | 200 |

b) Kapseln

| | mg/Kapsel |
|---|---|
| Verbindung A | 10 |
| Milchzucker | 30 |
| Maisstärke | 8,5 |
| Talk | 1 |
| Magnesiumstearat | 0,5 |
| Kapselfüllgewicht | 50 |

Als Wirkstoffe können auch die nachfolgend aufgeführten Verbindungen B-Y verwendet werden:

B = 1-[(10-Chlor-6,7-dihydro-4-oxo-3-phenyl-4H- benzo[a]chinolizin-1-yl)carbonyl]-4-piperidinol.
C = (4,5-Dihydro-7-oxo-8-phenyl-7H-thieno[2,3- a]chinolizin-10-yl)methyl-4-morpholincarboxylat.
D = 4-[(6,7-Dihydro-4-oxo-3-phenyl-10-chlor-4H-benzo[a]chinolizin-1-yl)carbonyl]-2,6-dimethylmorpholin.
E = (S)-1-[(7-Oxo-8-phenyl-7H-thieno[2,3-a]chinolizin- 10-yl)carbonyl]-2-pyrrolidinmethanol.
F = (S)-2-Methoxymethyl-1-[(7-oxo-8-phenyl-7H- thieno[2,3-a]chinolizin-10-yl)carbonyl]pyrrolidin.
G = 1-[(10-Chlor-4-oxo-3-phenyl-4H-benzo[a]chinolizin- 1-yl)carbonyl]-3-methoxypyrrolidin.
H = (S)-1-[(10-Chlor-4-oxo-3-phenyl-4H-benzo[a]chinolizin-1-yl)carbonyl]-2-pyrrolidinmethanol.
I = cis-4-[(4,5-Dihydro-7-oxo-8-phenyl-7H-thieno[2,3-a]chinolizin-10-yl)carbonyl]-2,6-dimethylmorpholin.
K = 1-[(10-Chlor-6,7-dihydro-4-oxo-3-phenyl-4H-benzo[a]chinolizin-1-yl)carbonyl]-4-methoxypiperidin.
L = 1-[(10-Chlor-3-phenyl-4-oxo-4H-benzo[a]chinolizin-1-yl)carbonyl]-4-methoxypiperidin.
M = N-Äthyl-N-(2-methoxyäthyl)-7-oxo-8-phenyl-7H-thieno[2,3-a]chinolizin-10-carboxamid.
N = N-(2-Methoxyäthyl)-N-methyl-7-oxo-8-phenyl-7H-thieno[2,3-a]chinolizin-10-carboxamid.
O = (R)-2-(Methoxymethyl)-1-[(7-oxo-8-phenyl-7H-thieno[2,3-a]chinolizin-10-yl)carbonyl]pyrrolidin.
P = 1-[(10-Chlor-6,7-dihydro-4-oxo-3-phenyl-4H-benzo[a]chinolizin-1-yl)carbonyl]-3-methoxypyrrolidin.
Q = 1-[(10-Chlor-6,7-dihydro-4-oxo-3-phenyl-4H-benzo[a]chinolizin-1-yl)carbonyl]-3-äthoxypyrrolidin.
R = (R)-3-Methoxy-1-[(7-oxo-8-phenyl-7H-thieno[2,3-a]chinolizin-10-yl)carbonyl]pyrrolidin.
S = (S)-3-Methoxy-1-[(7-oxo-8-phenyl-7H-thieno[2,3-a]chinolizin-10-yl)carbonyl]pyrrolidin.
T = (S)-1-[(10-Chlor-6,7-dihydro-4-oxo-3-phenyl-4H-benzo[a]chinolizin-1-yl)carbonyl]-2-(methoxymethyl)pyrrolidin.
U = 1-[(4,5-Dihydro-7-oxo-8-phenyl-7H-thieno[2,3-a]chinolizin-10-yl)carbonyl]-3-methoxypyrrolidin.
V = 3-Methoxy-1-[(7-oxo-8-phenyl-7H-thieno[2,3-a]chinolizin-10-yl)carbonyl]azetidin.
W = (R)-1-[(10-Chlor-4-oxo-3-phenyl-4H-benzo[a]chinolizin-1-yl)carbonyl]-2-pyrrolidinmethanol.
X = (S)-1-[(10-Chlor-4-oxo-3-phenyl-4H-benzo[a]chinolizin-1-yl)carbonyl]-2-(methoxymethyl)pyrrolidin.
Y = (R)-1-[(10-Chlor-4-oxo-3-phenyl-4H-benzo[a]chinolizin-1-yl)carbonyl]-2-(methoxymethyl)pyrrolidin.

**Patentansprüche für die Vertragsstaaten: BE, CH, DE, FR, IT, LI, LU, NL, SE**

1. Verbindungen der allgemeinen Formel

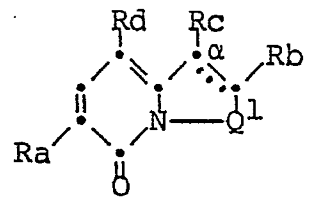

I

worin $Q^1$ zusammen mit dem Stickstoffatom eine Gruppe der Formel >N–$CH_2CH_2$– (a), >N–$CH_2CH_2CH_2$– (b), >N–CH=CH– (c), >N–$CH_2$–CH=CH– (d), >N–$CH_2$–$S(O)_p$– (e), >N–$CH_2CH_2$–$S(O)_p$– (f) oder >N–CH=CH–$S(O)_p$– (g), p die Zahl 0, 1 oder 2 und Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >$C_\alpha$–S–CH=CH– (h), >$C_\alpha$–CH=CH–S– (i) oder >$C_\alpha$–CH=CH–CH=CH– (j), die gestrichelte Linie eine zusätzliche Bindung, Rd die Gruppe der Formel –$(A^1)_m$–$(CO)_n$–$(Q^2A^2)_q$–$R^1$, m, n und q je die Zahl 0 oder 1, $A^1$ niederes Alkylen, $A^2$ niederes Alkylen, eine direkte Bindung oder die Gruppe –CO–, $Q^2$ ein Sauerstoffatom oder die Gruppe –$NR^2$–, $R^1$ Wasserstoff, Hydroxy, Cyano, Nitro, Halogen, niederes Alkoxy, niederes Alkyl, niederes Alkoxycarbonyl, Aryl, eine Gruppe der Formel –$NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe >N-$R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Massgabe, dass n die Zahl 0 bedeutet, wenn q die Zahl 1 und $A^2$ die Gruppe -CO- bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe -CO- bedeuten, und dass $R^1$ eine von Hydroxy, Cyano, Nitro, Halogen, niederes Alkoxycarbonyl, niederes Alkoxy und –$NR^3R^4$ verschiedene Bedeutung hat, wenn q die Zahl 1 und $A^2$ eine direkte Bindung bedeuten, und die mit nieder bezeichneten Reste höchstens 7 Kohlenstoffatome besitzen,
und pharmazeutisch annehmbare Säureadditionssalze von Verbindungen der Formel I mit einem oder mehreren basischen Substituenten.

2. Verbindungen nach Anspruch 1, worin Ra eine gegebenenfalls durch Halogen, Trifluormethyl, Nitro, niederes Alkyl oder niederes Alkoxy substituierte Phenylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >$C_\alpha$–S–CH=CH– (h), >$C_\alpha$–CH=CH–S– (i) oder >$C_\alpha$–CH=CH–CH=CH– (j), und die gestrichelte Linie eine zusätzliche Bindung, $A^2$ niederes Alkylen oder die Gruppe –CO–, $R^1$ Wasserstoff, Hydroxy, Cyano, Nitro, Halogen, niederes Alkoxy, niederes Alkyl, niederes Alkoxycarbonyl, Aryl, eine Gruppe der Formel –$NR^3R^4$ oder einen über ein Kohlenstoffatom gebundenen, 5-gliedrigen, gegebenenfalls durch eine oder zwei niedere Alkylgruppen substituierten, partiell ungesättigten oder aromatischen Heterocyclus, $R^3$ und $R^4$ je Wasserstoff, niederes Alkyl, $(C_{5-6})$-Cycloalkyl oder niederes Alkoxycarbonyl oder zusammen mit dem Stickstoffatom einen 5- oder 6-gliedrigen, gegebenenfalls durch eine oder zwei niedere Alkylgruppen substituierten, gesättigten N-Heterocyclus, der entweder noch an einem Kohlenstoffatom durch Oxo, Hydroxy, niederes Alkoxy, niederes Alkoxyalkyl, niederes Hydroxyalkyl, niederes Alkoxycarbonyl, Carbamoyl oder mono- oder di(niederes Alkyl)carbamoyl substituiert sein kann oder der als Ringglied noch ein Sauerstoff- oder Schwefelatom oder die Gruppe >N–$R^5$ enthalten kann, und $R^5$ Wasserstoff, niederes Alkyl, niederes Hydroxyalkyl, niederes Alkoxyalkyl, niederes Alkanoyl, niederes Alkoxycarbonyl, Carbamoyl oder mono- oder di(niederes Alkyl)carbamoyl bedeuten.

3. Verbindungen nach Anspruch 1 oder 2, worin $Q^1$ zusammen mit dem Stickstoffatom die Gruppe der Formel >N-$CH_2CH_2$- (a) oder >N-CH=CH- (c) bedeutet.

4. Verbindungen nach einem der Ansprüche 1-3, worin Ra eine gegebenenfalls durch m-Halogen oder m-Trifluormethyl substituierte Phenylgruppe bedeutet.

5. Verbindungen nach Anspruch 4, worin Ra Phenyl bedeutet.

6. Verbindungen nach einem der Ansprüche 1-5, worin Rb und Rc zusammen mit dem mit α bezeichneten Kohlenstoffatom eine gegebenenfalls durch Halogen substituierte Gruppe der Formel >$C_\alpha$-S-CH=CH- oder >$C_\alpha$-CH=CH-CH=CH- und die gestrichelte Linie eine zusätzliche Bindung bedeuten.

7. Verbindungen nach Anspruch 6, worin Rb und Rc zusammen mit dem mit α bezeichneten Kohlenstoffatom die Gruppe der Formel >$C_\alpha$-S-CH=CH- oder >$C_\alpha$-CH=CCl-CH=CH- bedeuten.

8. Verbindungen nach einem der Ansprüche 1 und 3-7, worin $Q^2$ ein Sauerstoffatom, $A^2$ die Gruppe -CO-, $R^1$ die Gruppe -$NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann, und entweder m und q die Zahl 0 und n die Zahl 1 oder m und q die Zahl 1 und n die Zahl 0 bedeuten.

9. Verbindungen nach Anspruch 8, worin $A^1$ Methylen und $R^3$ niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4- Morpholinylgruppe bedeuten.

10. Verbindungen nach Anspruch 9, worin $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl, 2-(niederes Hydroxyalkyl)- 1-pyrrolidinyl, 4-Hydroxy-1-piperidinyl, 4-(niederes Alkoxy)-1- piperidinyl, 4-Morpholinyl oder 2,6-di(niederes Alkyl)-4- morpholinyl bedeuten.

11. Verbindungen nach einem der Ansprüche 1-7, worin m und q die Zahl 0, n die Zahl 1 und $R^1$ Hydroxy oder niederes Alkoxy bedeuten.

12. 10-Chlor-6,7-dihydro-N-(2-methoxyäthyl)-4-oxo-3- phenyl-4H-benzo[a]chinolizin-1-carboxamid.

13. 1-[(10-Chlor-6,7-dihydro-4-oxo-3-phenyl-4H- benzo[a]chinolizin-1-yl)carbonyl]-4-piperidinol.

14. (4,5-Dihydro-7-oxo-8-phenyl-7H-thieno[2,3-a]chinolizin-10-yl)methyl-4-morpholincarboxylat.

15. 4-[(6,7-Dihydro-4-oxo-3-phenyl-10-chlor-4H-benzo[a]chinolizin-1-yl)carbonyl]-2,6-dimethylmorpholin.

16. (S)-1-[(7-Oxo-8-phenyl-7H-thieno[2,3-a]chinolizin- 10-yl)carbonyl]-2-pyrrolidinmethanol.

17. (S)-2-Methoxymethyl-1-[(7-oxo-8-phenyl-7H-thieno[2,3-a]chinolizin-10-yl)carbonyl]pyrrolidin.

18. 1-[(10-Chlor-4-oxo-3-phenyl-4H-benzo[a]chinolizin-1-yl)carbonyl]-3-methoxypyrrolidin.

19. (S)-1-[(10-Chlor-4-oxo-3-phenyl-4H-benzo[a]chinolizin-1-yl)carbonyl]-2-pyrrolidinmethanol.

20. cis-4-[(4,5-Dihydro-7-oxo-8-phenyl-7H-thieno[2,3-a]chinolizin-10-yl)carbonyl]-2,6-dimethylmorpholin.

21. 1-[(10-Chlor-6,7-dihydro-4-oxo-3-phenyl-4H-benzo[a]chinolizin-1-yl)carbonyl]-4-methoxypiperidin.

22. 1-[(10-Chlor-3-phenyl-4-oxo-4H-benzo[a]chinolizin-1-yl)carbonyl]-4-methoxypiperidin.

23. N-Äthyl-N-(2-methoxyäthyl)-7-oxo-8-phenyl-7H-thieno[2,3-a]chinolizin-10-carboxamid.

24. N-(2-Methoxyäthyl)-N-methyl-7-oxo-8-phenyl-7H-thieno[2,3-a]chinolizin-10-carboxamid.

25. (R)-2-(Methoxymethyl)-1-[(7-oxo-8-phenyl-7H-thieno[2,3-a]chinolizin-10-yl)carbonyl]pyrrolidin.

26. 1-[(10-Chlor-6,7-dihydro-4-oxo-3-phenyl-4H-benzo[a]chinolizin-1-yl)carbonyl]-3-methoxypyrrolidin.

27. 1-[(10-Chlor-6,7-dihydro-4-oxo-3-phenyl-4H-benzo[a]chinolizin-1-yl)carbonyl]-3-äthoxypyrrolidin.

28. (R)-3-Methoxy-1-[(7-oxo-8-phenyl-7H-thieno[2,3-a]chinolizin-10-yl)carbonyl]pyrrolidin.

29. (S)-3-Methoxy-1-[(7-oxo-8-phenyl-7H-thieno[2,3-a]chinolizin-10-yl)carbonyl]pyrrolidin.

30. (S)-1-[(10-Chlor-6,7-dihydro-4-oxo-3-phenyl-4H-benzo[a]chinolizin-1-yl)carbonyl]-2-(methoxymethyl)pyrrolidin.

31. 1-[(4,5-Dihydro-7-oxo-8-phenyl-7H-thieno[2,3-a]chinolizin-10-yl)carbonyl]-3-methoxypyrrolidin.

32. 3-Methoxy-1-[(7-oxo-8-phenyl-7H-thieno[2,3-a]chinolizin-10-yl)carbonyl]azetidin.

33. (R)-1-[(10-Chlor-4-oxo-3-phenyl-4H-benzo[a]chinolizin-1-yl)carbonyl]-2-pyrrolidinmethanol.

34. (S)-1-[(10-Chlor-4-oxo-3-phenyl-4H-benzo[a]chinolizin-1-yl)carbonyl]-2-(methoxymethyl)pyrrolidin.

35. (R)-1-[(10-Chlor-4-oxo-3-phenyl-4H-benzo[a]chinolizin-1-yl)carbonyl]-2-(methoxymethyl)pyrrolidin.

36. Verbindungen der allgemeinen Formel

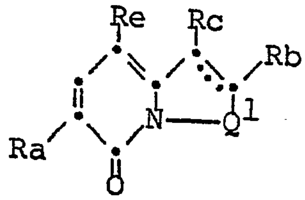

VIII

worin Re die Gruppe $-(A^1)_m-OOC-X^2$,
$-(A^1)_m-(CO)_n-(Q^2A^{22})_q-NR^{31}R^{41}$,
$-(A^1)_m-N{=}C{=}O$, $-(A^1)_m-CO-X^1$,
$-(A')_m-CO-CH^--N^+_2$ oder $-CH(OR^7)OR^8$ bedeutet,
und $A^1$, $Q^1$, $Q^2$, Ra, Rb, Rc, m, n, q und die gestrichelte Linie die in Anspruch 1 und A′, $A^{22}$, $R^7$, $R^8$, $R^{31}$, $R^{41}$, $X^1$ und $X^2$ die in Anspruch 41 angegebene Bedeutung besitzen.

37. Verbindungen der allgemeinen Formel

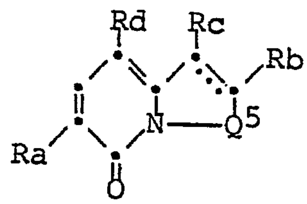

XV

worin $Q^5$ zusammen mit dem Stickstoffatom die Gruppe der Formel $-CH_2-CHX^1-S(O)_p-$ oder eine durch Halogen substituierte Dimethylengruppe und $X^1$ Halogen bedeuten, und Ra, Rb, Rc, Rd und p die in Anspruch 1 angegebene Bedeutung besitzen.

38. Verbindungen der allgemeinen Formel

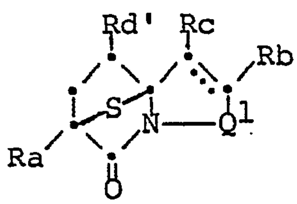

XIX

und

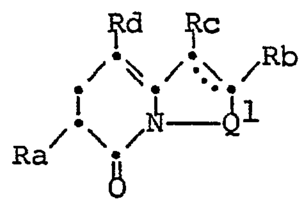

XXa

worin Ra, Rb, Rc, Rd, $Q^1$ und die gestrichelte Linie die in Anspruch 1 und Rd′ die in Anspruch 41 angegebene Bedeutung besitzen.

39. Verbindungen nach einem der Ansprüche 1–35 zur Anwendung als therapeutische Wirkstoffe.

40. Verbindungen nach einem der Ansprüche 1–35 zur Anwendung als muskelrelaxierend, sedativhypnotisch, anxiolytisch und/oder antikonvulsiv wirksame Stoffe.

41. Verfahren zur Herstellung von Verbindungen nach einem der Ansprüche 1–35 und pharmazeutisch annehmbaren Säureadditionssalzen davon, dadurch gekennzeichnet, daß man

a) eine Verbindung der allgemeinen Formel

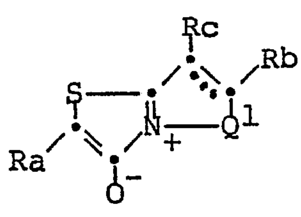

II

worin $Q^1$, Ra, Rb, Rc und die gestrichelte Linie die in Anspruch 1 angegebene Bedeutung besitzen,
bei erhöhter Temperatur mit einer Verbindung der allgemeinen Formel
$HC{\equiv}C-Rd'$ III oder $H_2C{=}CH-Rd'$ IV
worin Rd′ Cyano, Nitro oder die Gruppe der Formel $-CO-(Q^2A^2)_q-R^1$ bedeutet, und q, $A^2$, $Q^2$ und $R^1$ die in Anspruch 1 angegebene Bedeutung besitzen, oder mit Phenylvinylsulfoxid umsetzt und das erhaltene Cycloadditionsprodukt gegebenenfalls mit einer starken Base behandelt, oder

b) eine Verbindung der allgemeinen Formel
$ROOC-C(Ra)=CHR'$ V
worin R niederes Alkyl und R' Wasserstoff oder niederes Alkoxy bedeuten, und Ra die in Anspruch 1 angegebene Bedeutung besitzt, wenn R' Wasserstoff bedeutet, bei erhöhter Temperatur oder, wenn R' niederes Alkoxy bedeutet, in Gegenwart einer starken Base mit einer Verbindung der allgemeinen Formel

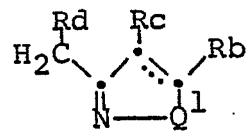
VI

worin $Q^1$, Rb, Rc, Rd und die gestrichelte Linie die in Anspruch 1 angegebene Bedeutung besitzen, umsetzt und das erhaltene Cyclokondensationsprodukt, wenn R' Wasserstoff bedeutet, dehydriert, oder
c) eine Verbindung der Formel I, welche eine veresterte Carboxygruppe besitzt, hydrolysiert, oder
d) eine Carbonsäure der allgemeinen Formel

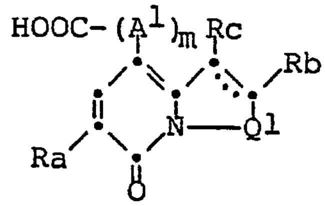
Ia

worin $A^1$, $Q^1$, Ra, Rb, Rc, m und die gestrichelte Linie die in Anspruch 1 angegebene Bedeutung besitzen, mit einem Alkohol der allgemeinen Formel
$HO-A^{21}-R^1$ VII
worin $A^{21}$ niederes Alkylen oder eine direkte Bindung bedeuten und $R^1$ die in Anspruch 1 angegebene Bedeutung besitzt, verestert, oder
e) eine Carbonsäure der obigen Formel Ia oder eine Carbonsäure der allgemeinen Formel

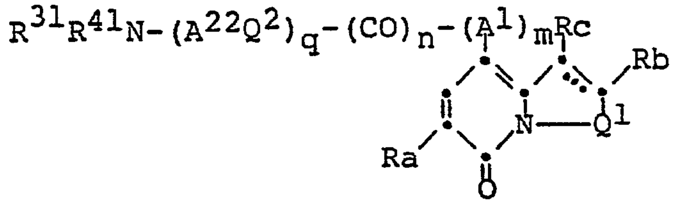
VIIIa

worin $A^{22}$ niederes Alkylen oder die Gruppe -CO- und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- oder Schwefelatom oder die Gruppe >N-$R^5$ enthalten kann, und $A^1$, $Q^1$, $Q^2$, Ra, Rb, Rc, $R^5$, m, n, q und die gestrichelte Linie die in Anspruch 1 angegebene Bedeutung besitzen, oder ein reaktives Derivat davon mit einem Amin der allgemeinen Formel
$HNR^2-A^{21}-R^1$ IX oder $HNR^3R^4$ X
worin $A^{21}$ obige und $R^1$, $R^2$, $R^3$ und $R^4$ die in Anspruch 1 angegebene Bedeutung besitzen, bzw. mit Ammoniak oder einem mono- oder di(niederen Alkyl)amin in das entsprechende Amid überführt, oder
f) eine Verbindung der allgemeinen Formel

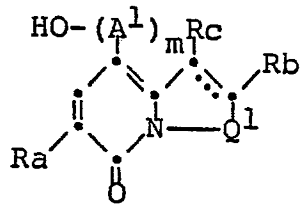
Ib'

worin $A^1$, $Q^1$, Ra, Rb, Rc, m und die gestrichelte Linie die in Anspruch 1 angegebene Bedeutung besitzen, in Gegenwart einer Base mit einer Verbindung der allgemeinen Formel

$X-A^{21}-R^1$ XI

worin X eine Abgangsgruppe bedeutet, $A^{21}$ obige und $R^1$ die in Anspruch 1 angegebene Bedeutung besitzen, bzw. eine Verbindung der Formel I, welche eine freie Hydroxygruppe besitzt, mit einer Verbindung der allgemeinen Formel

R-X XII

worin R niederes Alkyl bedeutet, und X obige Bedeutung besitzt, umsetzt, oder

g) eine Verbindung der allgemeinen Formel

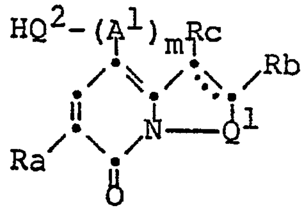

Ib

worin $A^1$, $Q^1$, $Q^2$, Ra, Rb, Rc, m und die gestrichelte Linie die in Anspruch 1 angegebene Bedeutung besitzen, in Gegenwart eines säurebindenden Mittels mit einem reaktiven Derivat einer Carbonsäure der allgemeinen Formel

$R^1$-COOH XIII

worin $R^1$ die in Anspruch 1 angegebene Bedeutung besitzt, umsetzt, oder

h) eine Verbindung der allgemeinen Formel

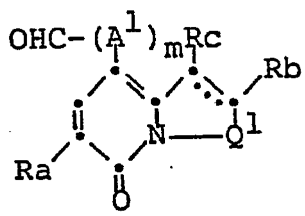

Ic

worin $A^1$, $Q^1$, Ra, Rb, Rc, m und die gestrichelte Linie die in Anspruch 1 angegebene Bedeutung besitzen, in Gegenwart eines Reduktionsmittels mit einem Amin der obigen Formel IX oder X umsetzt, oder

i) eine Verbindung der allgemeinen Formel

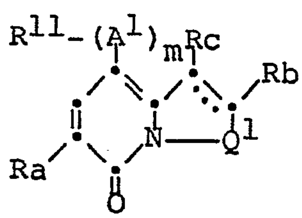

Id

worin $R^{11}$ Nitro, Cyano oder niederes Alkoxycarbonyl bedeutet, und $A^1$, $Q^1$, Ra, Rb, Rc, m und die gestrichelte Linie die in Anspruch 1 angegebene Bedeutung besitzen, oder eine Verbindung der obigen Formel Ia oder ein reaktives Derivat davon reduziert, oder

j) einen Alkohol der obigen Formel Ib' oder einen Alkohol der allgemeinen Formel

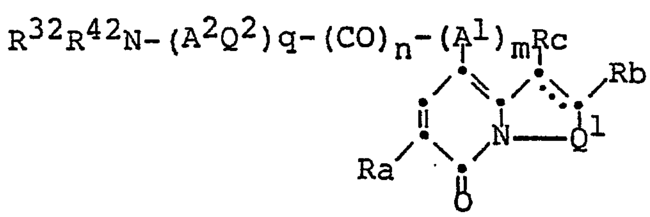

Ie

worin $A^1$, $A^2$, $Q^1$, $Q^2$, Ra, Rb, Rc, m, n, q und die gestrichelte Linie die in Anspruch 1 angegebene Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigten N-Heterocyclus bedeuten, der als Ringglied noch ein Sauerstoff- oder Schwefelatom

oder die Gruppe >N-$R^5$ enthalten kann, und $R^5$ die in Anspruch 1 angegebene Bedeutung besitzt, oxidiert, oder

k) ein Isocyanat der allgemeinen Formel

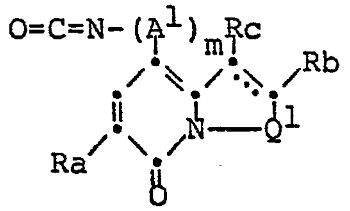

VIIIb

oder O=C-N-$R^{33}$ XIV

worin $A^1$, $Q^1$, Ra, Rb, Rc, m und die gestrichelte Linie die in Anspruch 1 angegebene Bedeutung besitzen, und $R^{33}$ Wasserstoff, niederes Alkyl oder ($C_{3-7}$)-Cycloalkyl bedeutet, mit einem niederen Alkohol oder einem Amin der obigen Formel X bzw. mit einer Verbindung der obigen Formel Ib umsetzt, oder

l) eine Verbindung der allgemeinen Formel

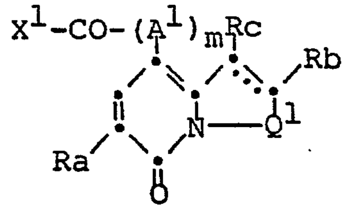

VIIIc

worin $X^1$ ein Halogenatom bedeutet, und $A^1$, $Q^1$, Ra, Rb, Rc, m und die gestrichelte Linie die in Anspruch 1 angegebene Bedeutung besitzen, mit einem niederen Alkylmagnesiumhalogenid umsetzt, oder

m) eine Verbindung der allgemeinen Formel

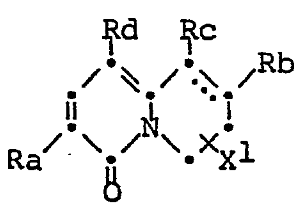

XVa oder

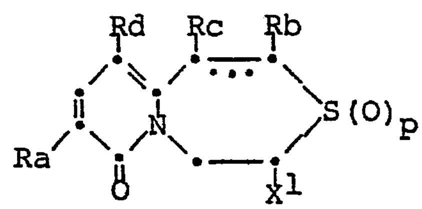

XVb

worin Ra, Rb, Rc, Rd, $X^1$, die gestrichelte Linie und p die in Anspruch 1 angegebene Bedeutung besitzen, in Gegenwart einer Base dehydrohalogeniert, oder

n) eine Verbindung der allgemeinen Formel

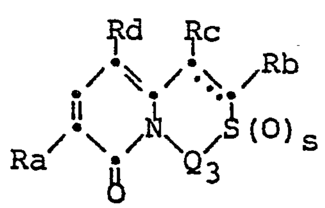

If

worin $Q^3$ die Gruppe $-CH_2-$, $-CH_2CH_2-$ oder -CH=CH- und s die Zahl 0 oder 1 bedeuten, und Ra, Rb, Rc, Rd und die gestrichelte Linie die in Anspruch 1 angegebene Bedeutung besitzen, S-oxidiert, oder

o) eine Verbindung der Wallgemeinen Formel

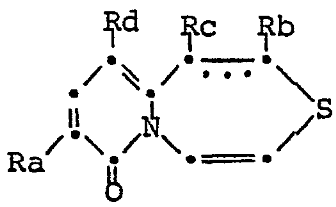

Ig

worin Ra, Rb, Rc, Rd und die gestrichelte Linie die in Anspruch 1 angegebene Bedeutung besitzen, erhitzt, oder

p) eine Verbindung der allgemeinen Formel

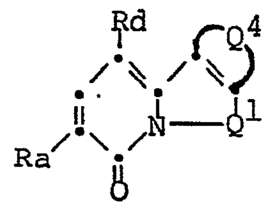

Ih

worin $Q^4$ die in Anspruch 1 angegebene Gruppe (h) oder (i) bedeutet und $Q^1$, Ra und Rd obige Bedeutung besitzen, am Thiophenring halogeniert, oder

q) eine Verbindung der obigen Formel VIIIc in Gegenwart einer Base mit einer Verbindung der allgemeinen Formel

$HYN{=}C(NH_2)\text{-}R''$ XVI, $H_2N\text{-}CHR''\text{-}CHR'''\text{-}Y'H$ XVII

oder $H_2N\text{-}NH\text{-}C(R'')=Y''$ XVIII

worin Y ein Sauerstoffatom oder die Gruppe -NR'''-, Y' ein Sauerstoffatom oder die Gruppe -NH-, Y'' ein Sauerstoff- oder Schwefelatom und R'' und R''' je Wasserstoff oder niederes Alkyl bedeuten, umsetzt und das erhaltene Produkt cyclisiert, oder

r) eine Verbindung der allgemeinen Formel

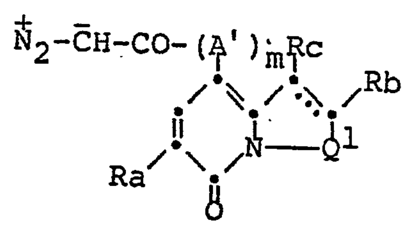

VIIId

worin A' $C_{1-6}$-Alkylen bedeutet, und $Q^1$, Ra, Rb, Rc, die gestrichelte Linie und m die in Anspruch 1 angegebene Bedeutung besitzen, mit einem niederen Alkohol umsetzt, oder

s) eine Carbonsäure der Formel Ia, worin m die Zahl 0 bedeutet, decarboxyliert, oder

t) eine Verbindung der Formel I, worin Rd Wasserstoff bedeutet, am Pyridonring halogeniert, oder

u) in einer Verbindung der allgemeinen Formel

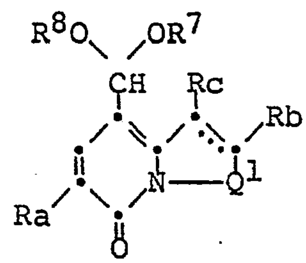

VIIIe

worin $R^7$ und $R^8$ je niederes Alkyl oder zusammen niederes Alkylen bedeuten, und $Q^1$, Ra, Rb, Rc und die gestrichelte Linie die in Anspruch 1 angegebene Bedeutung besitzen,

die Acetalgruppe spaltet, oder

v) eine Verbindung der Formel I, worin $Q^1$ zusammen mit dem Stickstoffatom die Gruppe >N–CH=CH– bedeutet, hydriert, oder

w) eine Verbindung der allgemeinen Formel

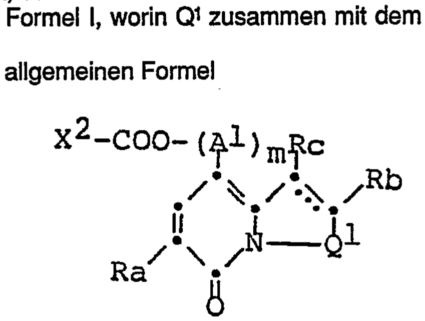

VIIIf

worin $X^2$ Phenoxy bedeutet, und $A^1$, $Q^1$, Ra, Rb, Rc, die gestrichelte Linie und m die in Anspruch 1 angegebene Bedeutung besitzen,
mit einem Amin der obigen Formel X umsetzt, und
x) erwünschtenfalls eine erhaltene Verbindung der Formel I mit einem basischen Substituenten in ein pharmazeutisch annehmbares Säureadditionssalz überführt.

42. Arzneimittel, enthaltend eine Verbindung nach einem der Ansprüche 1–35 und ein therapeutisch inertes Trägermaterial.

43. Muskelrelaxierend, sedativ-hypnotisch, anxiolytisch und/oder antikonvulsiv wirksame Mittel, enthaltend eine Verbindung nach einem der Ansprüche 1–35 und ein therapeutisch inertes Trägermaterial.

44. Verwendung von Verbindungen nach einem der Ansprüche 1–35 bei der Bekämpfung oder Verhütung von Krankheiten.

45. Verwendung von Verbindungen nach einem der Ansprüche 1–35 bei der Bekämpfung oder Verhütung von Muskelspannungen, Spannungszuständen, Schlaflosigkeit, Angstzuständen und/oder Konvulsionen.

46. Verwendung von Verbindungen nach einem der Ansprüche 1–35 zur Herstellung von muskelrelaxierend, sedativ-hypnotisch, anxiolytisch und/oder antikonvulsiv wirksamen Mitteln.

47. Verwendung von Verbindungen nach einem der Ansprüche 36–38 zur Herstellung von therapeutischen Wirkstoffen.

48. Verwendung von Verbindungen nach einem der Ansprüche 36–38 zur Herstellung von Verbindungen nach einem der Ansprüche 1–35.

**Patentansprüche für den Vertragsstaat: AT**

1. Verfahren zur Herstellung von Verbindungen der allgemeinen Formel

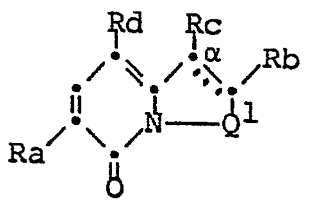

I

worin $Q^1$ zusammen mit dem Stickstoffatom eine Gruppe der Formel $>N-CH_2CH_2-$ (a), $>N-CH_2CH_2CH_2-$ (b), $>N-CH=CH-$ (c), $>N-CH_2-CH=CH-$ (d), $>N-CH_2-S(O)_p-$ (e), $>N-CH_2CH_2-S(O)_p-$ (f) oder $>N-CH=CH-S(O)_p-$ (g), p die Zahl 0, 1 oder 2 und Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_\alpha-S-CH=CH-$ (h), $>C_\alpha-CH=CH-S-$ (i) oder $>C_\alpha-CH=CH-CH=CH-$ (j), die gestrichelte Linie eine zusätzliche Bindung, Rd die Gruppe der Formel $-(A^1)_m-(CO)_n-(Q^2A^2)_q-R^1$, m, n und q je die Zahl 0 oder 1, $A^1$ niederes Alkylen, $A^2$ niederes Alkylen, eine direkte Bindung oder die Gruppe $-CO-$, $Q^2$ ein Sauerstoffatom oder die Gruppe $-NR^2-$, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylen dioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N-R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Massgabe, dass n die Zahl 0 bedeutet, wenn q die Zahl 1 und $A^2$ die Gruppe -CO- bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe –CO– bedeuten, und daß $R^1$ eine von Hydroxy, Cyano, Nitro, Halogen, niederes Alkoxycarbonyl, niederes Alkoxy und $-NR^3R^4$ verschiedene Bedeutung hat, wenn q die Zahl 1 und $A^2$ eine direkte Bindung bedeuten, und die mit nieder bezeichneten Reste höchstens 7 Kohlenstoffatome besitzen,
und von pharmazeutisch annehmbaren Säureadditionssalzen von Verbindungen der Formel I mit einem oder mehreren basischen Substituenten, dadurch gekennzeichnet, daß man

a) eine Verbindung der allgemeinen Formel

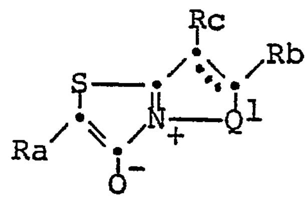

II

worin $Q^1$, Ra, Rb, Rc und die gestrichelte Linie obige Bedeutung besitzen,
bei erhöhter Temperatur mit einer Verbindung der allgemeinen Formel
$HC{\equiv}C{-}Rd'$ III oder $H_2C{=}CH{-}Rd'$ IV
worin Rd' Cyano, Nitro oder die Gruppe der Formel $-CO-(Q^2A^2)_q-R^1$ bedeutet, und q, $A^2$, $Q^2$ und $R^1$ obige Bedeutung besitzen,
oder mit Phenylvinylsulfoxid umsetzt und das erhaltene Cycloadditionsprodukt gegebenenfalls mit einer starken Base behandelt, oder

b) eine Verbindung der allgemeinen Formel
ROOC–C(Ra)=CHR' V
worin R niederes Alkyl und R' Wasserstoff oder niederes Alkoxy bedeuten, und Ra obige Bedeutung besitzt, wenn R' Wasserstoff bedeutet, bei erhöhter Temperatur oder,
wenn R' niederes Alkoxy bedeutet, in Gegenwart einer starken Base mit einer Verbindung der allgemeinen Formel

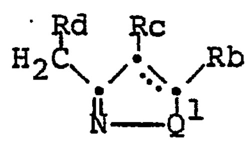

VI

worin $Q^1$, Rb, Rc, Rd und die gestrichelte Linie obige Bedeutung besitzen, umsetzt und das erhaltene Cyclokondensationsprodukt, wenn R' Wasserstoff bedeutet, dehydriert, oder

c) eine Verbindung der Formel I, welche eine veresterte Carboxygruppe besitzt, hydrolysiert, oder

d) eine Carbonsäure der allgemeinen Formel

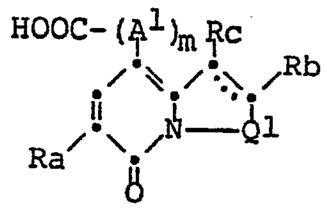

Ia

worin $A^1$, $Q^1$, Ra, Rb, Rc, m und die gestrichelte Linie obige Bedeutung besitzen, mit einem Alkohol der allgemeinen Formel
$HO{-}A^{21}{-}R^1$ VII
worin $A^{21}$ niederes Alkylen oder eine direkte Bindung bedeuten und $R^1$ obige Bedeutung besitzt, verestert, oder

e) eine Carbonsäure der obigen Formel Ia oder eine Carbonsäure der allgemeinen Formel

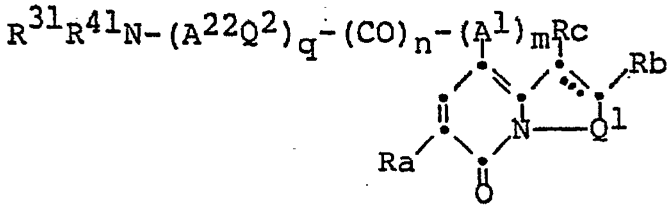

VIIIa

worin $A^{22}$ niederes Alkylen oder die Gruppe -CO- und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- oder Schwefelatom oder die Gruppe >N-$R^5$ enthalten kann, und $A^1$, $Q^1$, $Q^2$, Ra, Rb, Rc, $R^5$, m, n, q und die gestrichelte Linie obige Bedeutung besitzen, oder ein reaktives Derivat davon mit einem Amin der allgemeinen Formel

$HNR^2$-$A^{21}$-$R^1$ IX oder $HNR^3R^4$ X

worin $A^{21}$, $R^1$, $R^2$, $R^3$ und $R^4$ obige Bedeutung besitzen, bzw. mit Ammoniak oder einem mono- oder di(niederen Alkyl)amin in das entsprechende Amid überführt, oder

f) eine Verbindung der allgemeinen Formel

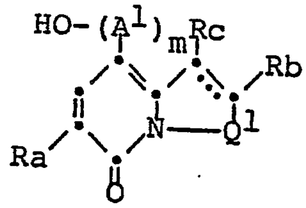

Ib'

worin $A^1$, $Q^1$, Ra, Rb, Rc, m und die gestrichelte Linie obige Bedeutung besitzen, in Gegenwart einer Base mit einer Verbindung der allgemeinen Formel

X-$A^{21}$-$R^1$ XI

worin X eine Abgangsgruppe bedeutet, $A^{21}$ obige und $R^1$ obige Bedeutung besitzen, bzw. eine Verbindung der Formel I, welche eine freie Hydroxygruppe besitzt, mit einer Verbindung der allgemeinen Formel

R-X XII

worin R niederes Alkyl bedeutet, und X obige Bedeutung besitzt, umsetzt, oder

g) eine Verbindung der allgemeinen Formel

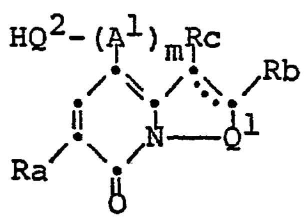

Ib

worin $A^1$, $Q^1$, $Q^2$, Ra, Rb, Rc, m und die gestrichelte Linie obige Bedeutung besitzen, in Gegenwart eines säurebindenden Mittels mit einem reaktiven Derivat einer Carbonsäure der allgemeinen Formel

$R^1$-COOH XIII

worin $R^1$ obige Bedeutung besitzt, umsetzt, oder

h) eine Verbindung der allgemeinen Formel

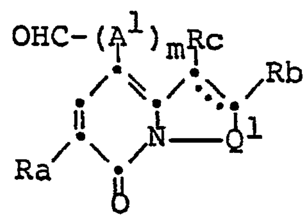

Ic

worin $A^1$, $Q^1$, Ra, Rb, Rc, m und die gestrichelte Linie obige Bedeutung besitzen, in Gegenwart eines Reduktionsmittels mit einem Amin der obigen Formel IX oder X umsetzt, oder
i) eine Verbindung der allgemeinen Formel

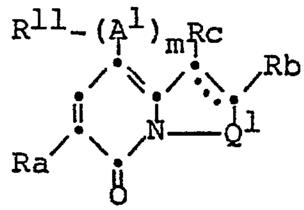

Id

worin $R^{11}$ Nitro, Cyano oder niederes Alkoxycarbonyl bedeutet, und $A^1$, $Q^1$, Ra, Rb, Rc, m und die gestrichelte Linie obige angegebene Bedeutung besitzen, oder eine Verbindung der obigen Formel Ia oder ein reaktives Derivat davon reduziert, oder
j) einen Alkohol der obigen Formel Ib' oder einen Alkohol der allgemeinen Formel

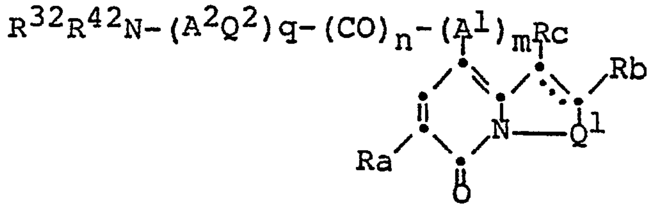

Ie

worin $A^1$, $A^2$, $Q^1$, $Q^2$, Ra, Rb, Rc, m, n, q und die gestrichelte Linie die in Anspruch 1 angegebene Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigten N-Heterocyclus bedeuten, der als Ringglied noch ein Sauerstoff- oder Schwefelatom oder die Gruppe >N-$R^5$ enthalten kann, und $R^5$ obige Bedeutung besitzt, oxidiert, oder
k) ein Isocyanat der allgemeinen Formel

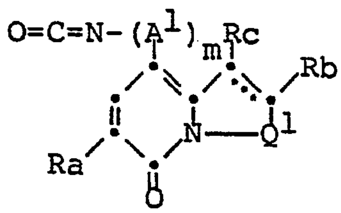

VIIIb

oder $O=C-N-R^{33}$ XIV

worin $A^1$, $Q^1$, Ra, Rb, Rc, m und die gestrichelte Linie die in Anspruch 1 angegebene Bedeutung besitzen, und $R^{33}$ Wasserstoff, niederes Alkyl oder ($C_{3-7}$)-Cycloalkyl bedeutet, mit einem niederen Alkohol oder einem Amin der obigen Formel X bzw. mit einer Verbindung der obigen Formel Ib umsetzt, oder
l) eine Verbindung der allgemeinen Formel

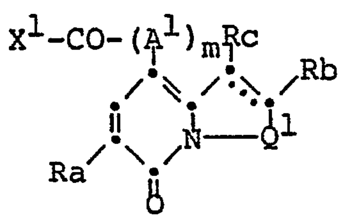

VIIIc

worin $X^1$ ein Halogenatom bedeutet, und $A^1$, $Q^1$, Ra, Rb, Rc, m und die gestrichelte Linie obige Bedeutung besitzen, mit einem niederen Alkylmagnesiumhalogenid umsetzt, oder
m) eine Verbindung der allgemeinen Formel

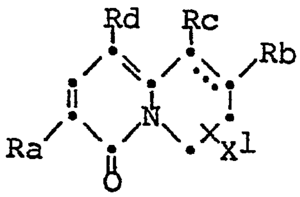 XVa | oder 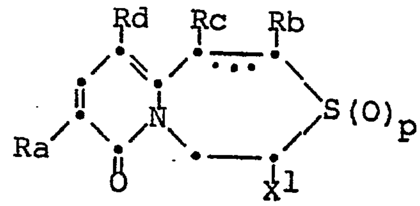 XVb

worin Ra, Rb, Rc, Rd, $X^1$, die gestrichelte Linie und p obige Bedeutung besitzen, in Gegenwart einer Base dehydrohalogeniert, oder

n) eine Verbindung der allgemeinen Formel

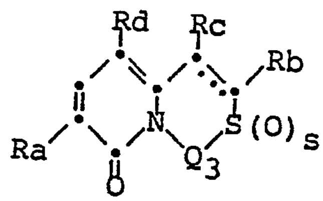 If

worin $Q^3$ die Gruppe $-CH_2-$, $-CH_2CH_2-$ oder $-CH{=}CH-$ und s die Zahl 0 oder 1 bedeuten, und Ra, Rb, Rc, Rd und die gestrichelte Linie obige Bedeutung besitzen, S-oxidiert, oder

o) eine Verbindung der allgemeinen Formel

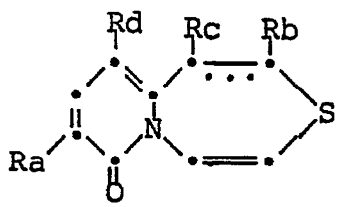 Ig

worin Ra, Rb, Rc, Rd und die gestrichelte Linie obige Bedeutung besitzen, erhitzt, oder

p) eine Verbindung der allgemeinen Formel

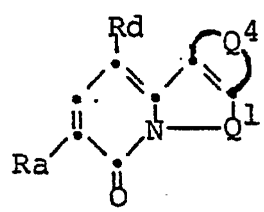 Ih

worin $Q^4$ die obige Gruppe (h) oder (i) bedeutet und $Q^1$, Ra und Rd obige Bedeutung besitzen, am Thiophenring halogeniert, oder

q) eine Verbindung der obigen Formel VIIIc in Gegenwart einer Base mit einer Verbindung der allgemeinen Formel

$HYN{=}C(NH_2)-R''$ XVI, $H_2N-CHR''-CHR'''-Y'H$ XVII

oder $H_2N-NH-C(R'')=Y''$ XVIII

worin Y ein Sauerstoffatom oder die Gruppe $-NR'''-$, Y' ein Sauerstoffatom oder die Gruppe -NH-, Y'' ein Sauerstoff- oder Schwefelatom und R'' und R''' je Wasserstoff oder niederes Alkyl bedeuten, umsetzt und das erhaltene Produkt cyclisiert, oder

r) eine Verbindung der allgemeinen Formel

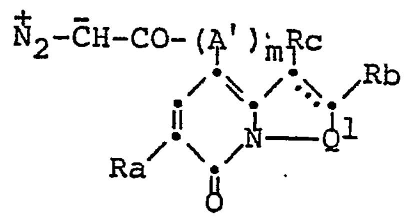

VIIId

worin A' $C_{1-6}$-Alkylen bedeutet, und $Q^1$, Ra, Rb, Rc, die gestrichelte Linie und m obige Bedeutung besitzen, mit einem niederen Alkohol umsetzt, oder

s) eine Carbonsäure der Formel Ia, worin m die Zahl 0 bedeutet, decarboxyliert, oder

t) eine Verbindung der Formel I, worin Rd Wasserstoff bedeutet, am Pyridonring halogeniert, oder

u) in einer Verbindung der allgemeinen Formel

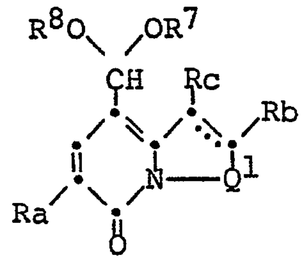

VIIIe

worin $R^7$ und $R^7$ je niederes Alkyl oder zusammen niederes Alkylen bedeuten, und $Q^1$, Ra, Rb, Rc und die gestrichelte Linie obige Bedeutung besitzen, die Acetalgruppe spaltet, oder

v) eine Verbindung der Formel I, worin $Q^1$ zusammen mit dem Stickstoffatom die Gruppe >N-CH=CH- bedeutet, hydriert, oder

w) eine Verbindung der allgemeinen Formel

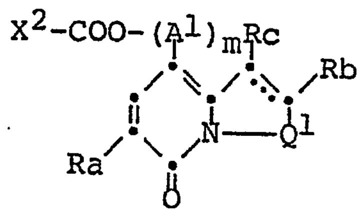

VIIIf

worin $X^2$ Phenoxy bedeutet, und $A^1$, $Q^1$, Ra, Rb, Rc, die gestrichelte Linie und m obige Bedeutung besitzen, mit einem Amin der obigen Formel X umsetzt, und

x) erwünschtenfalls eine erhaltene Verbindung der Formel I mit einem basischen Substituenten in ein pharmazeutisch annehmbares Säureadditionssalz überführt.

2. Verfahren nach Anspruch 1, worin Ra eine gegebenenfalls durch Halogen, Trifluormethyl, Nitro, niederes Alkyl oder niederes Alkoxy substituierte Phenylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >$C_\alpha$-S-CH=CH- (h), >$C_\alpha$-CH=CH-S- (i) oder >$C_\alpha$-CH=CH-CH=CH- (j), und die gestrichelte Linie eine zusätzliche Bindung, $A^2$ niederes Alkylen oder die Gruppe -CO-, $R^1$ Wasserstoff, Hydroxy, Cyano, Nitro, Halogen, niederes Alkoxy, niederes Alkyl, niederes Alkoxycarbonyl, Aryl, eine Gruppe der Formel $-NR^3R^4$ oder einen über ein Kohlenstoffatom gebundenen, 5-gliedrigen, gegebenenfalls durch eine oder zwei niedere Alkylgruppen substituierten, partiell ungesättigten oder aromatischen Heterocyclus, $R^3$ und $R^4$ je Wasserstoff, niederes Alkyl, $(C_{5-6})$-Cycloalkyl oder niederes Alkoxycarbonyl oder zusammen mit dem Stickstoffatom einen 5- oder 6-gliedrigen, gegebenenfalls durch eine oder zwei niedere Alkylgruppen substituierten, gesättigten N-Heterocyclus, der entweder noch an einem Kohlenstoffatom durch Oxo, Hydroxy, niederes Alkoxy, niederes Alkoxyalkyl, niederes Hydroxyalkyl, niederes Alkoxycarbonyl, Carbamoyl oder mono- oder di(niederes Alkyl)carbamoyl substituiert sein kann oder der als Ringglied noch ein Sauerstoff- oder Schwefelatom oder die Gruppe $>N-R^5$ enthalten kann, und $R^5$ Wasserstoff, niederes Alkyl, niederes Hydroxyalkyl, niederes Alkoxyalkyl, niederes Alkanoyl, niederes Alkoxycarbonyl, Carbamoyl oder mono- oder di(niederes Alkyl)carbamoyl bedeuten.

3. Verfahren nach Anspruch 1 oder 2, worin $Q^1$ zusammen mit dem Stickstoffatom die Gruppe der Formel $>N-CH_2CH_2-$ (a) oder >N-CH=CH- (c) bedeutet.

4. Verfahren nach einem der Ansprüche 1-3, worin Ra eine gegebenenfalls durch m-Halogen oder m-Trifluormethyl substituierte Phenylgruppe bedeutet.

5. Verfahren nach Anspruch 4, worin Ra Phenyl bedeutet.

6. Verfahren nach einem der Ansprüche 1-5, worin Rb und Rc zusammen mit dem mit $\alpha$ bezeichneten Kohlenstoffatom eine gegebenenfalls durch Halogen substituierte Gruppe der Formel $>C_\alpha$-S-CH=CH- oder $>C_\alpha$-CH=CH-CH=CH- und die gestrichelte Linie eine zusätzliche Bindung bedeuten.

7. Verfahren nach Anspruch 6, worin Rb und Rc zusammen mit dem mit $\alpha$ bezeichneten Kohlenstoffatom die Gruppe der Formel $>C_\alpha$-S-CH=CH- oder $>C_\alpha$-CH=CCl-CH=CH- bedeuten.

8. Verfahren nach einem der Ansprüche 1 und 3-7, worin $Q^2$ ein Sauerstoffatom, $A^2$ die Gruppe -CO-, $R^1$ die Gruppe $-NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann, und entweder m und q die Zahl 0 und n die Zahl 1 oder m und q die Zahl 1 und n die Zahl 0 bedeuten.

9. Verfahren nach Anspruch 8, worin $A^1$ Methylen und $R^3$ niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4-Morpholinylgruppe bedeuten.

10. Verfahren nach Anspruch 9, worin $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl, 2-(niederes Hydroxyalkyl)-1- pyrrolidinyl, 4-Hydroxy-1-piperidinyl, 4-(niederes Alkoxy)-1- piperidinyl, 4-Morpholinyl oder 2,6-di(niederes Alkyl)-4- morpholinyl bedeuten.

11. Verfahren nach einem der Ansprüche 1-7, worin m und q die Zahl 0, n die Zahl 1 und $R^1$ Hydroxy oder niederes Alkoxy bedeuten.

12. Verfahren nach Anspruch 1, dadurch gekennzeichnet, dass man 10-Chlor-6,7-dihydro-N-(2-methoxyäthyl)-4-oxo-3- phenyl-4H-benzo[a]chinolizin-1-carboxamid herstellt.

13. Verfahren nach Anspruch 1, dadurch gekennzeichnet, dass man 1-[(10-Chlor-6,7-dihydro-4-oxo-3-phenyl-4H-benzo[a]chinolizin-1-yl)carbonyl]-4-piperidinol herstellt.

14. Verfahren nach Anspruch 1, dadurch gekennzeichnet, dass man (4,5-Dihydro-7-oxo-8-phenyl-7H-thieno[2,3- a]chinolizin-10-yl)methyl-4-morpholincarboxylat herstellt.

15. Verfahren nach Anspruch 1, dadurch gekennzeichnet, dass man 4-[(6,7-Dihydro-4-oxo-3-phenyl-10-chlor-4H-benzo[a]- chinolizin-1-yl)carbonyl]-2,6-dimethylmorpholin herstellt.

16. Verfahren nach Anspruch 1, dadurch gekennzeichnet, dass man (S)-1-[(7-Oxo-8-phenyl-7H-thieno[2,3-a]chinolizin-10-yl)carbonyl]-2-pyrrolidinmethanol herstellt.

17. Verfahren nach Anspruch 1, dadurch gekennzeichnet, daß man (S)-2-Methoxymethyl-1-[(7-oxo-8-phenyl-7H-thieno[2,3-a]chinolizin-10-yl)carbonyl]pyrrolidin herstellt.

18. Verfahren nach Anspruch 1, dadurch gekennzeichnet, daß man 1-[(10-Chlor-4-oxo-3-phenyl-4H-benzo[a]chinolizin-1-yl)carbonyl]-3-methoxypyrrolidin herstellt.

19. Verfahren nach Anspruch 1, dadurch gekennzeichnet, daß man (S)-1-[(10-Chlor-4-oxo-3-phenyl-4H-benzo[a]chinolizin-1-yl)carbonyl]-2-pyrrolidinmethanol herstellt.

20. Verfahren nach Anspruch 1, dadurch gekennzeichnet, daß man cis-4-[(4,5-Dihydro-7-oxo-8-phenyl-7H-thieno[2,3-a]chinolizin-10-yl)carbonyl]-2,6-dimethylmorpholin herstellt.

21. Verfahren nach Anspruch 1, dadurch gekennzeichnet, daß man 1-[(10-Chlor-6,7-dihydro-4-oxo-3-phenyl-4H-benzo[a]chinolizin-1-yl)carbonyl]-4-methoxypiperidin herstellt.

22. Verfahren nach Anspruch 1, dadurch gekennzeichnet, daß man 1-[(10-Chlor-3-phenyl-4-oxo-4H-benzo[a]chinolizin-1-yl)carbonyl]-4-methoxypiperidin herstellt.

23. Verfahren nach Anspruch 1, dadurch gekennzeichnet, daß man N-Äthyl-N-(2-methoxyäthyl)-7-oxo-8-phenyl-7H-thieno[2,3-a]chinolizin-10-carboxamid herstellt.

24. Verfahren nach Anspruch 1, dadurch gekennzeichnet, daß man N-(2-Methoxyäthyl)-N-methyl-7-oxo-8-phenyl-7H-thieno[2,3-a]chinolizin-10-carboxamid herstellt.

25. Verfahren nach Anspruch 1, dadurch gekennzeichnet, daß man (R)-2-(Methoxymethyl)-1-[(7-oxo-8-phenyl-7H-thieno[2,3-a]chinolizin-10-yl)carbonyl]pyrrolidin herstellt.

26. Verfahren nach Anspruch 1, dadurch gekennzeichnet, dass man 1-[(10-Chlor-6,7-dihydro-4-oxo-3-phenyl-4H- benzo[a]chinolizin-1-yl)carbonyl]-3-methoxypyrrolidin herstellt.

27. Verfahren nach Anspruch 1, dadurch gekennzeichnet, dass man 1-[(10-Chlor-6,7-dihydro-4-oxo-3-phenyl-4H- benzo[a]chinolizin-1-yl)carbonyl]-3-äthoxypyrrolidin herstellt.

28. Verfahren nach Anspruch 1, dadurch gekennzeichnet, dass man (R)-3-Methoxy-1-[(7-oxo-8-phenyl-7H-thieno[2,3- a]chinolizin-10-yl)carbonyl]pyrrolidin herstellt.

29. Verfahren nach Anspruch 1, dadurch gekennzeichnet, dass man (S)-3-Methoxy-1-[(7-oxo-8-phenyl-7H-thieno[2,3- a]chinolizin-10-yl)carbonyl]pyrrolidin herstellt.

30. Verfahren nach Anspruch 1, dadurch gekennzeichnet, dass man (S)-1-[(10-Chlor-6,7-dihydro-4-oxo-3-phenyl-4H- benzo[a]chinolizin-1-yl)carbonyl]-2-(methoxymethyl)pyrrolidin herstellt.

31. Verfahren nach Anspruch 1, dadurch gekennzeichnet, dass man 1-[(4,5-Dihydro-7-oxo-8-phenyl-7H-thieno[2,3-a]chinolizin-10-yl)carbonyl]-3-methoxypyrrolidin herstellt.

32. Verfahren nach Anspruch 1, dadurch gekennzeichnet, dass man 3-Methoxy-1-[(7-oxo-8-phenyl-7H-thieno[2,3- a]chinolizin-10-yl)carbonyl]azetidin herstellt.

33. Verfahren nach Anspruch 1, dadurch gekennzeichnet, dass man (R)-1-[(10-Chlor-4-oxo-3-phenyl-4H-benzo[a]chinolizin- 1-yl)carbonyl]-2-pyrrolidinmethanol herstellt.

34. Verfahren nach Anspruch 1, dadurch gekennzeichnet, dass man (S)-1-[(10-Chlor-4-oxo-3-phenyl-4H-benzo[a]chinolizin- 1-yl)carbonyl]-2-(methoxymethyl)pyrrolidin herstellt.

35. Verfahren nach Anspruch 1, dadurch gekennzeichnet, dass man (R)-1-[(10-Chlor-4-oxo-3-phenyl-4H-benzo[a]chinolizin-1-yl)carbonyl]-2-(methoxymethyl)pyrrolidin herstellt.

**Claims for the Contracting States: BE, CH, DE, FR, IT, LI, LU, NL, SE**

1. Compounds of the general formula

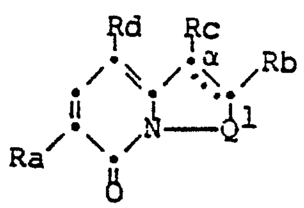

I

wherein $Q^1$ and the nitrogen atom together signify a group of the formula $>N-CH_2CH_2-$ (a), $>N-CH_2CH_2CH_2-$ (b), $>N-CH=CH-$ (c), $>N-CH_2-CH=CH-$ (d), $>N-CH_2-S(O)_p-$ (e), $>N-CH_2CH_2-S(O)_p-$ (f) or $>N-CH=CH-S(O)_p-$ (g), p signifies the number 0, 1 or 2 and Ra signifies a phenyl, pyridyl or thienyl group which is optionally substituted by halogen, trifluoromethyl, nitro, lower alkyl or lower alkoxy, Rb and Rc together with the carbon atom denoted by $\alpha$ signify a group of the formula $>C_\alpha-S-CH=CH-$ (h), $>C_\alpha-CH=CH-S-$ (i) or $>C_\alpha-CH=CH-CH=CH-$ (j) which is optionally substituted by halogen, trifluoromethyl, lower alkyl, lower alkoxy, nitro, amino or mono- or di(lower alkyl)amino, the dotted line signifies an additional bond, Rd signifies the group of the formula $-(A^1)_m-(CO)_n-(Q^2A^2)_q-R^1$, m, n and q each signify the number 0 or 1, $A^1$ signifies lower alkylene, $A^2$ signifies lower alkylene, a direct bond or the group $-CO-$, $Q^2$ signifies an oxygen atom or the group $-NR^2-$, $R^1$ signifies hydrogen, hydroxy, cyano, nitro, halogen, lower alkoxy, lower alkyl, lower alkoxycarbonyl, aryl, a group of the formula $-NR^3R^4$ or a 5-membered, saturated, partially unsaturated or aromatic heterocycle which is attached via a carbon atom and which is optionally substituted by one or two lower alkyl groups and optionally substituted by a $(C_{3-6})$-cycloalkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or alkylenedioxy group, $R^2$ signifies hydrogen, lower alkyl or aryl, $R^3$ and $R^4$ each signify hydrogen, lower alkyl, lower alkoxyalkyl, lower dialkoxyalkyl, lower alkylenedioxyalkyl, lower cyanoalkyl, lower haloalkyl, lower hydroxyalkyl, lower dihydroxyalkyl, lower alkanoyl, lower alkoxycarbonyl or a $(C_{3-7})$-cycloalkyl group which is optionally substituted by hydroxy, lower alkoxy, lower alkanoyloxy, lower hydroxyalkyl, lower alkoxyalkyl, lower alkanoyloxyalkyl, oxo, carbamoyl, mono- or di(lower alkyl)carbamoyl or by lower alkylenedioxy or together with the nitrogen atom signify a 3- to 7-membered, saturated N-heterocycle which is optionally substituted by one or two lower alkyl groups and optionally substituted by one or two hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or lower alkylenedioxy groups and which can contain as a ring member an oxygen or sulphur atom or the group $>N-R^5$ and $R^5$ signifies hydrogen, lower alkyl, lower hydroxyalkyl, lower alkoxyalkyl, lower alkanoyloxyalkyl, lower alkanoyl, lower alkoxycarbonyl, carbamoyl or mono- or di(lower alkyl)carbamoyl, with the proviso that n signifies the number 0 when q signifies the number 1 and $A^2$ signifies the group $-CO-$, that $R^1$ has a significance different from cyano, nitro, halogen or lower alkoxycarbonyl when q signifies the number 0 and n signifies the number 1 or when q signifies the number 1 and $A^2$ signifies the group $-CO-$, and that $R^1$ has a significance different from hydroxy, cyano, nitro, halogen, lower alkoxycarbonyl, lower alkoxy and $-NR^3R^4$ when q signifies the number 1 and $A^2$ signifies a direct bond, and the residues denoted by lower have a maximum of 7 carbon atoms,
and pharmaceutically acceptable acid addition salts of compounds of formula I which have one or more basic substituents.

2. Compounds according to claim 1, wherein Ra signifies a phenyl group which is optionally substituted by halogen, trifluoromethyl, nitro, lower alkyl or lower alkoxy, Rb and Rc together with the carbon atom denoted by $\alpha$ signify a group of the formula $>C_\alpha-S-CH=CH-$ (h), $>C_\alpha-CH=CH-S-$ (i) or $>C_\alpha-CH=CH-CH=CH-$ (j) which is optionally substituted by halogen, trifluoromethyl, lower alkyl, lower alkoxy, nitro, amino or mono- or di(lower alkyl)amino, and the dotted line signifies an additional bond, $A^2$

signifies lower alkylene or the group –CO–, $R^1$ signifies hydrogen, hydroxy, cyano, nitro, halogen, lower alkoxy, lower alkyl, lower alkoxycarbonyl, aryl, a group of the formula $-NR^3R^4$ or a 5-membered, partially unsaturated or aromatic heterocycle which is attached via a carbon atom and which is optionally substituted by one or two lower alkyl groups, $R^3$ and $R^4$ each signify hydrogen, lower alkyl, $(C_{5-6})$-cycloalkyl or lower alkoxycarbonyl or together with the nitrogen atom signify a 5- or 6-membered, saturated N-heterocycle which is optionally substituted by one or two lower alkyl groups and which either can be substituted on a carbon atom by oxo, hydroxy, lower alkoxy, lower alkoxyalkyl, lower hydroxyalkyl, lower alkoxycarbonyl, carbamoyl or mono- or di(lower alkyl)carbamoyl or which can contain as a ring member an oxygen or sulphur atom or the group $>N-R^5$ and $R^5$ signifies hydrogen, lower alkyl, lower hydroxyalkyl, lower alkoxyalkyl, lower alkanoyl, lower alkoxycarbonyl, carbamoyl or mono- or di(lower alkyl)carbamoyl.

3. Compounds according to claim 1 or 2, wherein $Q^1$ and the nitrogen atom together signify a group of the formula $>N-CH_2CH_2-$ (a) or $>N-CH=CH-$ (c).

4. Compounds according to any one of claims 1–3, wherein Ra signifies a phenyl group which is optionally substituted by m-halogen or m-trifluoromethyl.

5. Compounds according to claim 4, wherein Ra signifies phenyl.

6. Compounds according to any one of claims 1–5, wherein Rb and Rc together with the carbon atom denoted by α signify a group of the formula $>C_\alpha-S-CH=CH-$ or $>C_\alpha-CH=CH-CH=CH-$ which is optionally substituted by halogen and the dotted line signifies an additional bond.

7. Compounds according to claim 6, wherein Rb and Rc together with the carbon atom denoted by α signify the group of the formula $>C_\alpha-S-CH=CH-$ or $>C_\alpha-CH=CCl-CH=CH-$.

8. Compounds according to any one of claims 1 and 3–7, wherein $Q^2$ signifies an oxygen atom, $A^2$ signifies the group –CO–, $R^1$ signifies the group $-NR^3R^4$, $R^3$ signifies lower alkoxyalkyl and $R^4$ signifies hydrogen or lower alkyl or $R^3$ and $R^4$ together with the nitrogen atom signify a 4-, 5- or 6-membered, saturated N-heterocycle which is optionally substituted by one or two lower alkyl groups and optionally substituted by a hydroxy, lower alkoxy, lower hydroxyalkyl or lower alkoxyalkyl group and which can contain as a ring member an oxygen atom and either m and q signify the number 0 and n signifies the number 1 or m and q signify the number 1 and n signifies the number 0.

9. Compounds according to claim 8, wherein $A^1$ signifies methylene and $R^3$ signifies lower alkoxyalkyl and $R^4$ signifies hydrogen or lower alkyl or $R^3$ and $R^4$ together with the nitrogen atom signify a 1-azetidinyl, 1-pyrrolidinyl, 1-piperidinyl or 4-morpholinyl group which is optionally substituted by one or two lower alkyl groups and which is optionally substituted by a hydroxy, lower alkoxy, lower hydroxyalkyl or lower alkoxyalkyl group.

10. Compounds according to claim 9, wherein $R^3$ signifies 2-(lower alkoxy)ethyl and $R^4$ signifies hydrogen or lower alkyl or $R^3$ and $R^4$ together with the nitrogen atom signify 3-(lower alkoxy)-1-azetidinyl, 3-(lower alkoxy)-1-pyrrolidinyl, 2-(lower alkoxyalkyl)-1-pyrrolidinyl, 2-(lower hydroxyalkyl)-1-pyrrolidinyl, 4-hydroxy-1-piperidinyl, 4-(lower alkoxy)-1-piperidinyl, 4-morpholinyl or 2,6-di(lower alkyl)-4-morpholinyl.

11. Compounds according to any one of claims 1–7, wherein m and q signify the number 0, n signifies the number 1 and $R^1$ signifies hydroxy or lower alkoxy.

12. 10-Chloro-6,7-dihydro-N-(2-methoxyethyl)-4-oxo-3-phenyl-4H-benzo[a]quinolizine-1-carboxamide.

13. 1-[(10-Chloro-6,7-dihydro-4-oxo-3-phenyl-4H-benzo[a]quinolizin-1-yl)carbonyl]-4-piperidinol.

14. (4,5-Dihydro-7-oxo-8-phenyl-7H-thieno[2,3-a]quinolizin-10-yl)methyl-4-morpholinecarboxylate.

15. 4-[(6,7-Dihydro-4-oxo-3-phenyl-10-chloro-4H-benzo[a]quinolizin-1-yl)carbonyl]-2,6-dimethylmorpholine.

16. (S)-1-[(7-Oxo-8-phenyl-7H-thieno[2,3-a]quinolizin-10-yl)carbonyl]-2-pyrrolidinemethanol.

17. (S)-2-Methoxymethyl-1-[(7-oxo-8-phenyl-7H-thieno[2,3-a]quinolizin-10-yl)carbonyl]pyrrolidine.

18. 1-[(10-Chloro-4-oxo-3-phenyl-4H-benzo[a]quinolizin-1-yl)carbonyl]-3-methoxypyrrolidine.

19. (S)-1-[(10-Chloro-4-oxo-3-phenyl-4H-benzo[a]quinolizin-1-yl)carbonyl]-2-pyrrolidinemethanol.

20. cis-4-[(4,5-Dihydro-7-oxo-8-phenyl-7H-thieno[2,3-a]quinolizin-10-yl)carbonyl]-2,6-dimethylmorpholine.

21. 1-[(10-Chloro-6,7-dihydro-4-oxo-3-phenyl-4H-benzo[a]quinolizin-1-yl)carbonyl]-4-methoxypiperidine.

22. 1-[(10-Chloro-3-phenyl-4-oxo-4H-benzo[a]quinolizin-1-yl)carbonyl]-4-methoxypiperidine.

23. N-Ethyl-N-(2-methoxyethyl)-7-oxo-8-phenyl-7H-thieno[2,3-a]quinolizine-10-carboxamide.

24. N-(2-Methoxyethyl)-N-methyl-7-oxo-8-phenyl-7H-thieno[2,3-a]quinolizine-10-carboxamide.

25. (R)-2-(Methoxymethyl)-1-[(7-oxo-8-phenyl-7H-thieno[2,3-a]quinolizin-10-yl)carbonyl]pyrrolidine.

26. 1-[(10-Chloro-6,7-dihydro-4-oxo-3-phenyl-4H-benzo[a]quinolizin-1-yl)carbonyl]-3-methoxypyrrolidine.

27. 1-[(10-Chloro-6,7-dihydro-4-oxo-3-phenyl-4H-benzo[a]quinolizin-1-yl)carbonyl]-3-ethoxypyrrolidine.

28. (R)-3-Methoxy-1-[(7-oxo-8-phenyl-7H-thieno[2,3-a]quinolizin-10-yl)carbonyl]pyrrolidine.

29. (S)-3-Methoxy-1-[(7-oxo-8-phenyl-7H-thieno[2,3-a]quinolizin-10-yl)carbonyl]pyrrolidine.

30. (S)-1-[(10-Chloro-6,7-dihydro-4-oxo-3-phenyl-4H-benzo[a]quinolizin-1-yl)carbonyl]-2-(methoxymethyl)-pyrrolidine.

31. 1-[(4,5-Dihydro-7-oxo-8-phenyl-7H-thieno[2,3-a]quinolizin-10-yl)carbonyl]-3-methoxypyrrolidine.

32. 3-Methoxy-1-[(7-oxo-8-phenyl-7H-thieno[2,3-a]quinolizin-10-yl)carbonyl]azetidine.

33. (R)-1-[(10-Chloro-4-oxo-3-phenyl-4H-benzo[a]quinolizin-1-yl)carbonyl]-2-pyrrolidinemethanol.

34. (S)-1-[(10-Chloro-4-oxo-3-phenyl-4H-benzo[a]quinolizin-1-yl)carbonyl]-2-(methoxymethyl)pyrrolidine.

35. (R)-1-[(10-Chloro-4-oxo-3-phenyl-4H-benzo[a]quinolizin-1-yl)carbonyl]-2-(methoxymethyl)pyrrolidine.

36. Compounds of the general formula

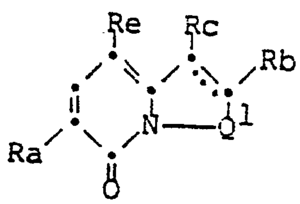

VIII

wherein Re signifies the group $-(A^1)_m-OOC-X^2$, $-(A^1)_m-(CO)_n-(Q^2A^{22})_q-NR^{31}R^{41}$, $-(A^1)_m-N=C=O$, $-(A^1)_m-CO-X^1$, $-(A')_m-CO-CH^{-}-N^{+}_2$ or $-CH(OR^7)OR^8$, and $A^1$, $Q^1$, $Q^2$, Ra, Rb, Rc, m, n, q and the dotted line have the significance given in claim 1 and A′, $A^{22}$, $R^7$, $R^8$, $R^{31}$, $R^{41}$, $X^1$ and $X^2$ have the significance given in claim 41.

37. Compounds of the general formula

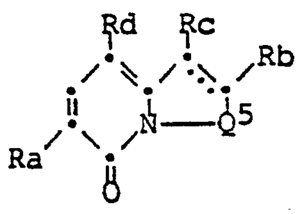

XV

wherein $Q^5$ and the nitrogen atom together signify the group of the formula $-CH_2-CHX^1-S(O)_p-$ or a dimethylene group which is substituted by halogen and $X^1$ signifies halogen, and Ra, Rb, Rc, Rd and p have the significance given in Claim 1.

38. Compounds of the general formula

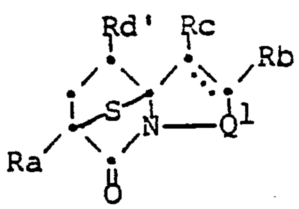

XIX and

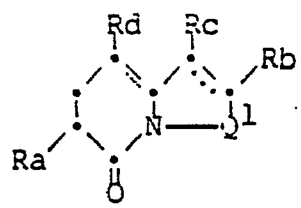

XXa

wherein Ra, Rb, Rc, Rd, $Q^1$ and the dotted line have the significance given in Claim 1 and Rd′ has the significance given in claim 41.

39. Compounds according to any one of claims 1–35 for use as therapeutically active substances.

40. Compounds according to any one of claims 1–35 for use as substances having muscle relaxant, sedative-hypnotic, anxiolytic and/or anticonvulsive activity.

41. A process for the manufacture of compounds according to any one of claims 1–35 and pharmaceutically acceptable acid addition salts thereof, characterized by

a) reacting a compound of the general formula

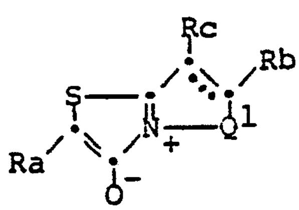

II

wherein $Q^1$, Ra, Rb, Rc and the dotted line have the significance given in claim 1,
at an elevated temperature with a compound of the general formula
HC≡C–Rd′ III or $H_2C$=CH–Rd′ IV
wherein Rd′ signifies cyano, nitro or the group of the formula $-CO-(Q^2A^2)_q-R^1$ and q, $A^2$, $Q^2$ and $R^1$ have the significance given in claim 1,
or with phenylvinyl sulphoxide and, if necessary, treating the cycloaddition product obtained with a strong base, or
b) reacting a compound of the general formula
ROOC–C(Ra)=CHR′ V
wherein R signifies lower alkyl and R′ signifies hydrogen or lower alkoxy, and Ra has the significance given in claim 1,
with a compound of the general formula

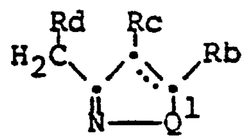
VI

wherein $Q^1$, Rb, Rc, Rd and the dotted line have the significance given in claim 1,
at an elevated temperature when R′ signifies hydrogen or in the presence of a base when R′ signifies lower alkoxy and dehydrogenating the cyclocondensation product obtained when R′ signifies hydrogen, or
c) hydrolyzing a compound of formula I which contains an esterified carboxy group, or
d) esterifying a carboxylic acid of the general formula

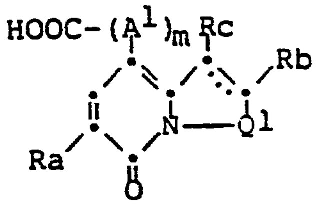
Ia

wherein $A^1$, $Q^1$, Ra, Rb, Rc, m and the dotted line have the significance given in claim 1,
with an alcohol of the general formula
$HO-A^{21}-R^1$ VII
wherein $A^{21}$ signifies lower alkylene or a direct bond and $R^1$ has the significance given in claim 1, or
e) converting a carboxylic acid of formula Ia above or a carboxylic acid of the general formula

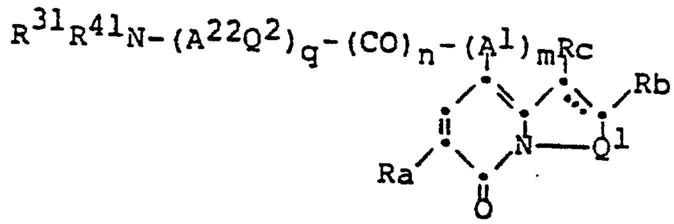
VIIIa

wherein $A^{22}$ signifies lower alkylene or the group –CO– and $R^{31}$ and $R^{41}$ together with the nitrogen atom signify a 3- to 7-membered, saturated N-heterocyle which is optionally substituted by one or two lower alkyl groups and which is substituted by a carboxy group and which can contain as a ring member an oxygen or sulphur atom or the group >N–$R^5$ and $A^1$, $Q^1$, $Q^2$, Ra, Rb, Rc, $R^5$, m, n, q and the dotted line have the significance given in claim 1,
or a reactive derivative thereof into the corresponding amide with an amine of the general formula
$HNR^2-A^{21}-R^1$ IX or $HNR^3R^4$ X
wherein $A^{21}$ has the significance given above and $R^1$, $R^2$, $R^3$ and $R^4$ have the significance given in claim 1,
or with ammonia or a mono- or di(lower alkyl)amine, respectively, or
f) reacting a compound of the general formula

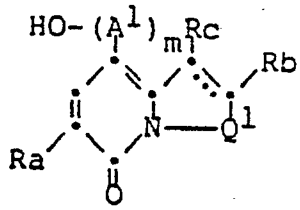

Ib'

wherein $A^1$, $Q^1$, Ra, Rb, Rc, m and the dotted line have the significance given in claim 1, in the presence of a base with a compound of the general formula

$X–A^{21}–R^1$ XI

wherein X signifies a leaving group, $A^{21}$ has the significance given above and $R^1$ has the significance given in claim 1,
or reacting a compound of formula I which contains a free hydroxy group with a compound of the general formula

R–X XII

wherein R signifies lower alkyl and X has the above significance, or
g) reacting a compound of the general formula

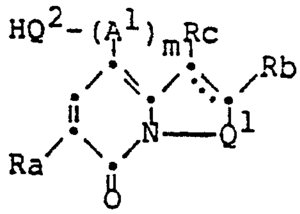

Ib

wherein $A^1$, $Q^1$, $Q^2$, Ra, Rb, Rc, m and the dotted line have the significance given in claim 1, in the presence of an acid-binding agent with a reactive derivative of a carboxylic acid of the general formula

$R^1–COOH$ XIII

wherein $R^1$ has the significance given in claim 1, or
h) reacting a compound of the general formula

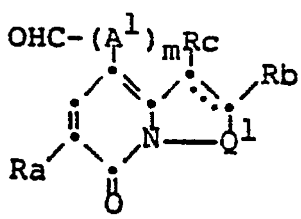

Ic

wherein $A^1$, $Q^1$, Ra, Rb, Rc, m and the dotted line have the significance given in claim 1, in the presence of a reduction agent with an amine of formula IX or X above, or
i) reducing a compound of the general formula

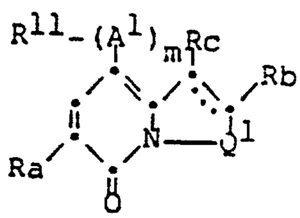

Id

wherein $R^{11}$ signifies nitro, cyano or lower alkoxycarbonyl and $A^1$, $Q^1$, Ra, Rb, Rc, m and the dotted line have the significance given in claim 1,
or a compound of formula Ia above or a reactive derivative thereof, or
j) oxidizing an alcohol of formula Ib′ above or an alcohol of the general formula

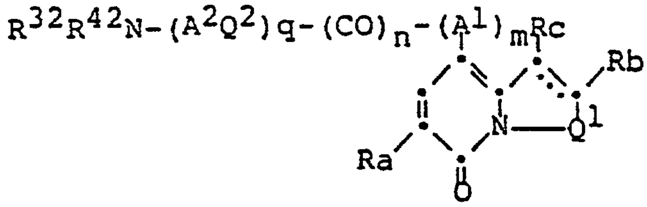

Ie

wherein $A^1$, $A^2$, $Q^1$, $Q^2$, Ra, Rb, Rc, m, n, q and the dotted line have the significance given in claim 1 and $R^{32}$ and $R^{42}$ together with the nitrogen atom signify a 3- to 7-membered, saturated N-heterocycle which is optionally substituted by one or two lower alkyl groups and which is substituted by a hydroxy group and which can contain as a ring member an oxygen or sulphur atom or the group >N–$R^5$ and $R^5$ has the significance given in claim 1, or

k) reacting an isocyanate of the general formula

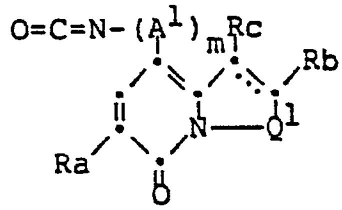

VIIIb or O=C-N-$R^{33}$ XIV

wherein $A^1$, $Q^1$, Ra, Rb, Rc, m and the dotted line have the significance given in claim 1 and $R^{33}$ signifies hydrogen, lower alkyl or ($C_{3-7}$)-cycloalkyl,

with a lower alcohol or an amine of formula X above or with a compound of formula Ib above, respectively, or

l) reacting a compound of the general formula

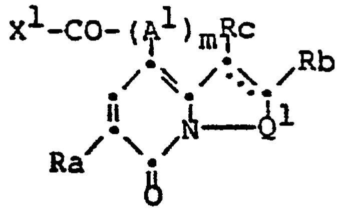

VIIIc

wherein $X^1$ signifies a halogen atom and $A^1$, $Q^1$, Ra, Rb, Rc, m and the dotted line have the significance given in claim 1,

with a lower alkylmagnesium halide, or

m) dehydrohalogenating a compound of the general formula

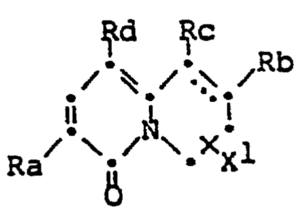

XVa or

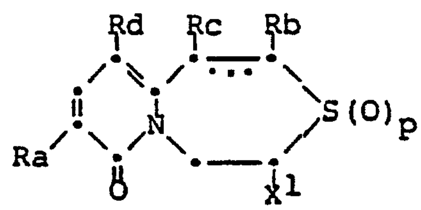

XVb

wherein Ra, Rb, Rc, Rd, $X^1$, the dotted line and p have the significance given in claim 1,

in the presence of a base, or

n) S-oxidizing a compound of the general formula

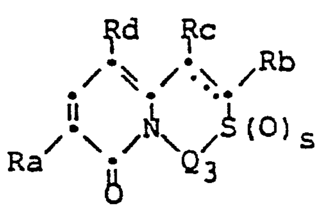

If

wherein $Q^3$ signifies the group $-CH_2-$, $-CH_2CH_2-$ or $-CH=CH-$ and s signifies the number 0 or 1, and Ra, Rb, Rc, Rd and the dotted line have the significance given in claim 1, or

o) heating a compound of the general formula

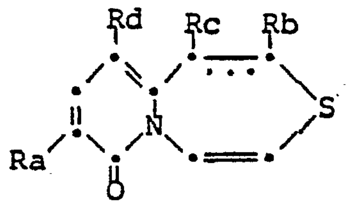

Ig

wherein Ra, Rb, Rc, Rd and the dotted line have the significance given in claim 1, or

p) halogenating a compound of the general formula

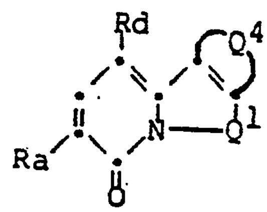

Ih

wherein $Q^4$ signifies the group (h) or (i) given in claim 1 and $Q^1$, Ra and Rd have the above significance, on the thiophene ring, or

q) reacting a compound of formula VIIIc above in the presence of a base with a compound of the general formula

$HYN=C(NH_2)-R''$ XVI, $H_2N-CHR''-CHR'''-Y'H$ XVII

or $H_2N-NH-C(R'')=Y''$ XVIII

wherein Y signifies an oxygen atom or the group $-NR'''$, Y' signifies an oxygen atom or the group $-NH-$, Y'' signifies an oxygen or sulphur atom and R'' and R''' each signify hydrogen or lower alkyl,

and cyclizing the product obtained, or

r) reacting a compound of the general formula

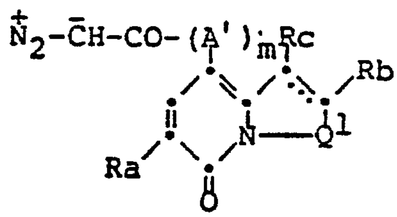

VIIId

wherein A' signifies $C_{1-6}$-alkylene and $Q^1$, Ra, Rb, Rc, the dotted line and m have the significance given in claim 1,

which a lower alcohol, or

s) decarboxylating a carboxylic acid of formula Ia in which m signifies the number 0, or

t) halogenating a compound of formula I in which Rd signifies hydrogen on the pyridone ring, or

u) cleaving the acetal group in a compound of the general formula

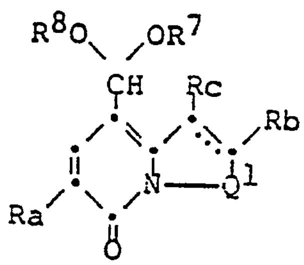

VIIIe

wherein $R^7$ and $R^8$ each signify lower alkyl or together signify lower alkylene and $Q^1$, Ra, Rb, Rc and the dotted line have the significance given in claim 1, or

v) hydrogenating a compound of formula I in which $Q^1$ and the nitrogen atom together signify the group >N–CH=CH–, or

w) reacting a compound of the general formula

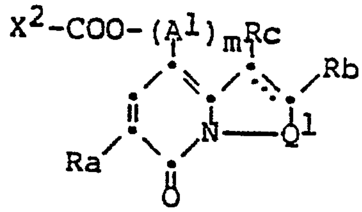

VIIIf

wherein $X^2$ signifies phenoxy and $A^1$, $Q^1$, Ra, Rb, Rc, the dotted line and m have the significance given in claim 1,

with an amine of formula X above, and

x) if desired, converting a compound of formula I obtained which has a basic substituent into a pharmaceutically acceptable acid addition salt.

42. A medicament containing a compound according to any one of claims 1–35 and a therapeutically inert carrier material.

43. A medicament having muscle relaxant, sedative-hypnotic, anxiolytic and/or anticonvulsive activity, containing a compound according to any one of claims 1–35 and a therapeutically inert carrier material.

44. The use of compounds according to any one of claims 1–35 in the control or prevention of illnesses.

45. The use of compounds according to any one of claims 1–35 in the control or prevention of muscle tensions, stress conditions, insomnia, anxiety states and/or convulsions.

46. The use of compounds according to any one of claims 1–35 for the manufacture of medicaments having muscle relaxant, sedative-hypnotic, anxiolytic and/or anticonvulsive activity.

47. The use of compounds according to any one of claims 36–38 for the manufacture of therapeutically active substances.

48. The use of compounds according to any one of claims 36–38 for the manufacture of compounds according to any one of claims 1–35.

**Claims for the Contracting State: AT**

1. A process for the manufacture of compounds of the general formula

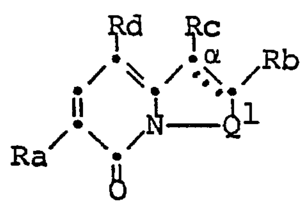

I

wherein $Q^1$ and the nitrogen atom together signify a group of the formula $>N-CH_2CH_2-$ (a), $>N-CH_2CH_2CH_2-$ (b), >N–CH=CH– (c), $>N-CH_2-CH=CH-$ (d), $N-CH_2-S(O)_p-$ (e), $>N-CH_2CH_2-S(O)_p-$ (f) or $>N-CH=CH-S(O)_p-$ (g), p signifies the number 0, 1 or 2 and Ra signifies a phenyl, pyridyl or thienyl group which is optionally substituted by halogen, trifluoromethyl, nitro, lower alkyl or lower alkoxy, Rb and Rc together with the carbon atom denoted by α signify a group of the formula $>C_\alpha-S-CH=CH-$ (h), $>C_\alpha-CH=CH-S-$ (i) or $>C_\alpha-CH=CH-CH=CH-$ (j) which is optionally substituted by halogen, trifluoromethyl, lower alkyl, lower alkoxy, nitro, amino or mono- or di(lower alkyl)amino, the dotted line signifies an additional bond, Rd signifies the group of the formula $-(A^1)_m-(CO)_n-(Q^2A^2)_q-R^1$, m, n and q each signify the number 0 or 1, $A^1$ signifies lower alkylene, $A^2$ signifies lower alkylene, a direct bond or the group –CO–, $Q^2$ signifies an oxygen atom or the group $-NR^2-$, $R^1$ signifies hydrogen, hydroxy, cyano, nitro, halogen, lower alkoxy, lower alkyl, lower alkoxycarbonyl, aryl, a group of the formula $-NR^3R^4$ or a 5-membered, saturated, partially unsaturated or aromatic heterocycle which is attached via a carbon atom and which is optionally substituted by one or two lower alkyl groups and optionally substituted by a $(C_{3-6})$-cycloalkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or alkylenedioxy group, $R^2$ signifies hydrogen, lower alkyl or aryl, $R^3$ and $R^4$ each signify hydrogen, lower alkyl, lower alkoxyalkyl, lower dialkoxyalkyl, lower alkylenedioxyalkyl, lower cyanoalkyl, lower haloalkyl, lower hydroxyalkyl, lower dihydroxyalkyl, lower alkanoyl, lower alkoxycarbonyl or a $(C_{3-7})$-cycloalkyl group which is optionally substituted by hydroxy, lower alkoxy, lower alkanoyloxy, lower hydroxyalkyl, lower alkoxyalkyl, lower alkanoyloxyalkyl, oxo, carbamoyl, mono- or di(lower alkyl)carbamoyl or by lower alkylenedioxy or together with the nitrogen atom signify a 3- to 7-membered, saturated N-heterocycle which is optionally substituted by one or two lower alkyl groups and optionally substituted by one or two hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or lower alkylenedioxy groups and which can contain as a ring member an oxygen or sulphur atom or the group >N–$R^5$ and $R^5$ signifies hydrogen, lower alkyl, lower hydroxyalkyl, lower alkoxyalkyl, lower alkanoyloxyalkyl, lower alkanoyl, lower alkoxycarbonyl, carbamoyl or mono- or di(lower alkyl)carbamoyl, with the proviso that n signifies the number 0 when q signifies the number 1 and $A^2$ signifies the group –CO–, that $R^1$ has a significance different from cyano, nitro, halogen or lower alkoxycarbonyl when q signifies the number 0 and n signifies the number 1 or when q signifies the number 1 and $A^2$ signifies the group –CO–, and that $R^1$ has a significance different from hydroxy, cyano, nitro, halogen, lower alkoxycarbonyl, lower alkoxy and $-NR^3R^4$ when q signifies the number 1 and $A^2$ signifies a direct bond, and the residues denoted by lower have a maximum of 7 carbon atoms,
and of pharmaceutically acceptable acid addition salts of compounds of formula I which have one or more basic substituents, characterized by

a) reacting a compound of the general formula

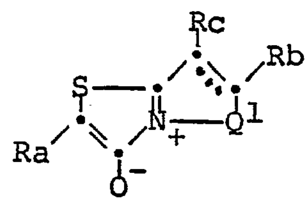

II

wherein $Q^1$, Ra, Rb, Rc and the dotted line have the above significance,
at an elevated temperature with a compound of the general formula
HC≡C–Rd′ III or $H_2C$=CH–Rd′ IV
wherein Rd′ signifies cyano, nitro or the group of the formula $-CO-(Q^2A^2)_q-R^1$ and q, $A^2$, $Q^2$ and $R^1$ have the above significance,
or with phenylvinyl sulphoxide and, if necessary, treating the cycloaddition product obtained with a strong base, or

b) reacting a compound of the general formula
ROOC–C(Ra)=CHR′ V
wherein R signifies lower alkyl and R′ signifies hydrogen or lower alkoxy, and Ra has the above significance,
with a compound of the general formula

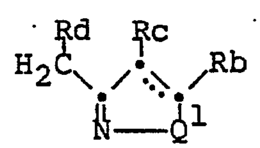

VI

wherein $Q^1$, Rb, Rc, Rd and the dotted line have the above significance,
at an elevated temperature when R′ signifies hydrogen or in the presence of a base when R′ signifies lower alkoxy and dehydrogenating the cyclocondensation product obtained when R′ signifies hydrogen, or

c) hydrolyzing a compound of formula I which contains an esterified carboxy group, or

d) esterifying a carboxylic acid of the general formula

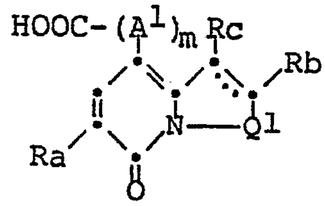

Ia

wherein $A^1$, $Q^1$, Ra, Rb, Rc, m and the dotted line have the above significance,
with an alcohol of the general formula
$HO-A^{21}-R^1$ VII

wherein $A^{21}$ signifies lower alkylene or a direct bond and $R^1$ has the above significance, or
e) converting a carboxylic acid of formula Ia above or a carboxylic acid of the general formula

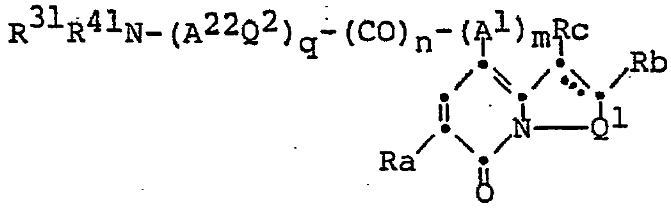

VIIIa

wherein $A^{22}$ signifies lower alkylene or the group –CO– and $R^{31}$ and $R^{41}$ together with the nitrogen atom signify a 3- to 7-membered, saturated N-heterocycle which is optionally substituted by one or two lower alkyl groups and which is substituted by a carboxy group and which can contain as a ring member an oxygen or sulphur atom or the group >N–$R^5$ and $A^1$, $Q^1$, $Q^2$, Ra, Rb, Rc, $R^5$, m, n, q and the dotted line have the above significance,
or a reactive derivative thereof into the corresponding amide with an amine of the general formula
$HNR^2–A^{21}–R^1$ IX or $HNR^3R^4$ X
wherein $A^{21}$, $R^1$, $R^2$, $R^3$ and $R^4$ have the above significance,
or with ammonia or a mono- or di(lower alkyl)amine, respectively, or
f) reacting a compound of the general formula

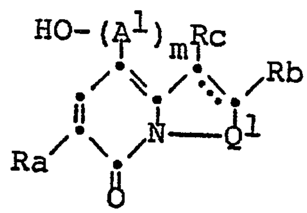

Ib'

wherein $A^1$, $Q^1$, Ra, Rb, Rc, m and the dotted line have the above significance,
in the presence of a base with a compound of the general formula
$X–A^{21}–R^1$ XI
wherein X signifies a leaving group, $A^{21}$ has the above significance and $R^1$ has the above significance,
or reacting a compound of formula I which contains a free hydroxy group with a compound of the general formula
R–X XII
wherein R signifies lower alkyl and X has the above significance, or
g) reacting a compound of the general formula

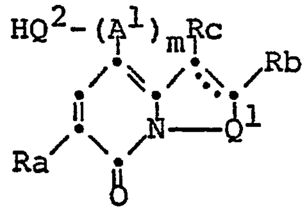

Ib

wherein $A^1$, $Q^1$, $Q^2$, Ra, Rb, Rc, m and the dotted line have the above significance,
in the presence of an acid-binding agent with a reactive derivative of a carboxylic acid of the general formula
$R^1–COOH$ XIII
wherein $R^1$ has the above significance, or
h) reacting a compound of the general formula

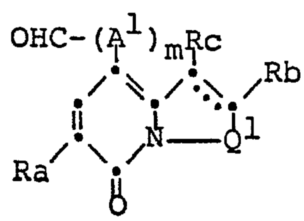

Ic

wherein $A^1$, $Q^1$, Ra, Rb, Rc, m and the dotted line have the above significance, in the presence of a reduction agent with an amine of formula IX or X above, or
i) reducing a compound of the general formula

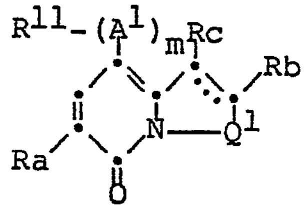

Id

wherein $R^{11}$ signifies nitro, cyano or lower alkoxycarbonyl and $A^1$, $Q^1$, Ra, Rb, Rc, m and the dotted line have the above significance,
or a compound of formula Ia above or a reactive derivative thereof, or
j) oxidizing an alcohol of formula Ib′ above or an alcohol of the general formula

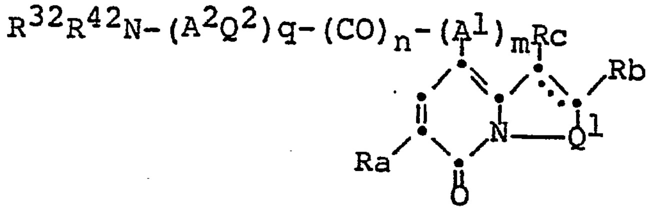

Ie

wherein $A^1$, $A^2$, $Q^1$, $Q^2$, Ra, Rb, Rc, m, n, q and the dotted line have the significance given in claim 1 and $R^{32}$ and $R^{42}$ together with the nitrogen atom signify a 3- to 7-membered, saturated N-heterocycle which is optionally substituted by one or two lower alkyl groups and which is substituted by a hydroxy group and which can contain as a ring member an oxygen or sulphur atom or the group >N–$R^5$ and $R^5$ has the above significance, or
k) reacting an isocyanate of the general formula

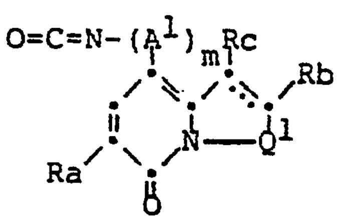

VIIIb

or $O{=}C{-}N{-}R^{33}$ XIV

wherein $A^1$, $Q^1$, Ra, Rb, Rc, m and the dotted line have the significance given in claim 1 and $R^{33}$ signifies hydrogen, lower alkyl or ($C_{3-7}$)-cycloalkyl,
with a lower alcohol or an amine of formula X above or with a compound of formula Ib above, respectively, or
l) reacting a compound of the general formula

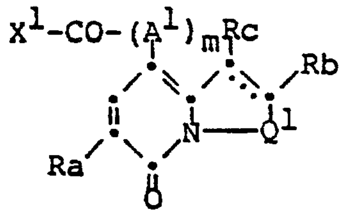

VIIIc

wherein $X^1$ signifies a halogen atom and $A^1$, $Q^1$, Ra, Rb, Rc, m and the dotted line have the above significance,
with a lower alkylmagnesium halide, or
m) dehydrohalogenating a compound of the general formula

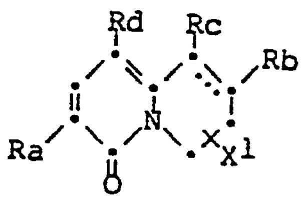

XVa or

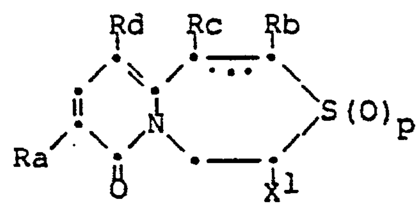

XVb

wherein Ra, Rb, Rc, Rd, $X^1$, the dotted line and p have the above significance,
in the presence of a base, or
n) S-oxidizing a compound of the general formula

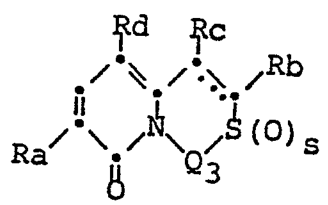

If

wherein $Q^3$ signifies the group $-CH_2-$, $-CH_2CH_2-$ or $-CH=CH-$ and s signifies the number 0 or 1, and Ra, Rb, Rc, Rd and the dotted line have the above significance, or
o) heating a compound of the general formula

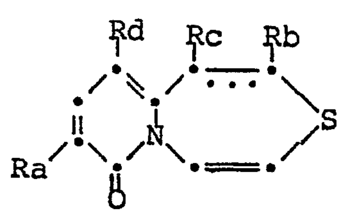

Ig

wherein Ra, Rb, Rc, Rd and the dotted line have the above significance, or
p) halogenating a compound of the general formula

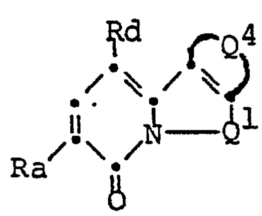

Ih

wherein $Q^4$ signifies the group (h) or (i) above and $Q^1$, Ra and Rd have the above significance, on the thiophene ring, or
q) reacting a compound of formula VIIIc above in the presence of a base with a compound of the general formula

HYN=C($NH_2$)–R″ XVI, $H_2N$–CHR″–CHR‴–Y′H XVII or $H_2N$–NH–C(R″)=Y″ XVIII
wherein Y signifies an oxygen atom or the group –NR‴–, Y′ signifies an oxygen atom or the group –NH–, Y″ signifies an oxygen or sulphur atom and R″ and R‴ each signify hydrogen or lower alkyl,
and cyclizing the product obtained, or
r) reacting a compound of the general formula

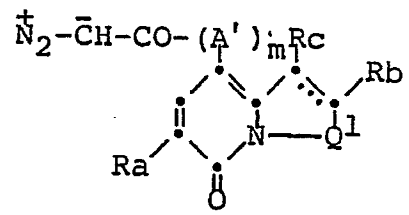

VIIId

wherein A′ signifies $C_{1-6}$-alkylene and $Q^1$, Ra, Rb, Rc, the dotted line and m have the above significance, with a lower alcohol, or
s) decarboxylating a carboxylic acid of formula Ia in which m signifies the number 0, or
t) halogenating a compound of formula I in which Rd signifies hydrogen on the pyridone ring, or
u) cleaving the acetal group in a compound of the general formula

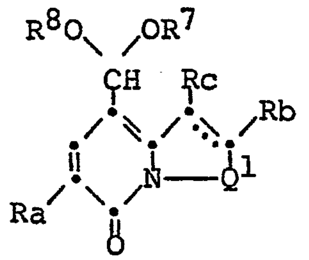

VIIIe

wherein $R^7$ and $R^8$ each signify lower alkyl or together signify lower alkylene and $Q^1$, Ra, Rb, Rc and the dotted line have the above significance, or
v) hydrogenating a compound of formula I in which $Q^1$ and the nitrogen atom together signify the group >N–CH=CH–, or
w) reacting a compound of the general formula

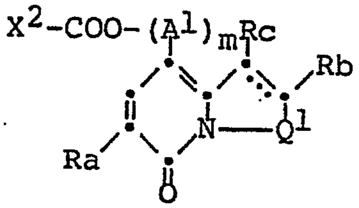

VIIIf

wherein $X^2$ signifies phenoxy and $A^1$, $Q^1$, Ra, Rb, Rc, the dotted line and m have the above significance,
with an amine of formula X above, and
x) if desired, converting a compound of formula I obtained which has a basic substituent into a pharmaceutically acceptable acid addition salt.

2. A process according to claim 1, wherein Ra signifies a phenyl group which is optionally substituted by halogen, trifluoromethyl, nitro, lower alkyl or lower alkoxy, Rb and Rc together with the carbon atom denoted by α signify a group of the formula >$C_\alpha$–S–CH=CH– (h), >$C_\alpha$–CH=CH–S– (i) or >$C_\alpha$–CH=CH–CH=CH– (j) which is optionally substituted by halogen, trifluoromethyl, lower alkyl, lower alkoxy, nitro, amino or mono- or di(lower alkyl)amino, and the dotted line signifies an additional bond, $A^2$ signifies lower alkylene or the group –CO–, $R^1$ signifies hydrogen, hydroxy, cyano, nitro, halogen, lower alkoxy, lower alkyl, lower alkoxycarbonyl, aryl, a group of the formula –$NR^3R^4$ or a 5-membered, partially unsaturated or aromatic heterocycle which is attached via a carbon atom and which is optionally substituted by one or two lower alkyl groups, $R^3$ and $R^4$ each signify hydrogen, lower alkyl, ($C_{5-6}$)-cycloalkyl or lower alkoxycarbonyl or together with the nitrogen atom signify a 5- or 6-membered, saturated N-heterocycle which is optionally substituted by one or two lower alkyl groups and which either can be substituted on a carbon atom by oxo, hydroxy, lower alkoxy, lower alkoxyalkyl, lower hydroxyalkyl, lower

alkoxycarbonyl, carbamoyl or mono- or di(lower alkyl)carbamoyl or which can contain as a ring member an oxygen or sulphur atom or the group $>N-R^5$ and $R^5$ signifies hydrogen, lower alkyl, lower hydroxyalkyl, lower alkoxyalkyl, lower alkanoyl, lower alkoxycarbonyl, carbamoyl or mono- or di(lower alkyl)carbamoyl.

3. A process according to claim 1 or 2, wherein $Q^1$ and the nitrogen atom together signify the group of the formula $>N-CH_2CH_2-$ (a) or $>N-CH=CH-$ (c).

4. A process according to any one of claims 1–3, wherein Ra signifies a phenyl group which is optionally substituted by m-halogen or m-trifluoromethyl.

5. A process according to claim 4, wherein Ra signifies phenyl.

6. A process according to any one of claims 1–5, wherein Rb and Rc together with the carbon atom denoted by $\alpha$ signify a group of the formula $>C_\alpha-S-CH=CH-$ or $>C_\alpha-CH=CH-CH=CH-$ which is optionally substituted by halogen and the dotted line signifies an additional bond.

7. A process according to claim 6, wherein Rb and Rc together with the carbon atom denoted by $\alpha$ signify the group of the formula $>C_\alpha-S-CH=CH-$ or $>C_\alpha-CH=CCl-CH=CH-$.

8. A process according to any one of claims 1 and 3–7, wherein $Q^2$ signifies an oxygen atom, $A^2$ signifies the group $-CO-$, $R^1$ signifies the group $-NR^3R^4$, $R^3$ signifies lower alkoxyalkyl and $R^4$ signifies hydrogen or lower alkyl or $R^3$ and $R^4$ together with the nitrogen atom signify a 4-, 5- or 6-membered, saturated N-heterocycle which is optionally substituted by one or two lower alkyl groups and optionally substituted by a hydroxy, lower alkoxy, lower hydroxyalkyl or lower alkoxyalkyl group and which can contain as a ring member an oxygen atom and either m and q signify the number 0 and n signifies the number 1 or m and q signify the number 1 and n signifies the number 0.

9. A process according to claim 8, wherein $A^1$ signifies methylene and $R^3$ signifies lower alkoxyalkyl and $R^4$ signifies hydrogen or lower alkyl or $R^3$ and $R^4$ together with the nitrogen atom signify a 1-azetidinyl, 1-pyrrolidinyl, 1-piperidinyl or 4-morpholinyl group which is optionally substituted by one or two lower alkyl groups and which is optionally substituted by a hydroxy, lower alkoxy, lower hydroxyalkyl or lower alkoxyalkyl group.

10. A process according to claim 9, wherein $R^3$ signifies 2-(lower alkoxy)ethyl and $R^4$ signifies hydrogen or lower alkyl or $R^3$ and $R^4$ together with the nitrogen atom signify 3-(lower alkoxy)-1-azetidinyl, 3-(lower alkoxy)-1-pyrrolidinyl, 2-(lower alkoxyalkyl)-1-pyrrolidinyl, 2-(lower hydroxyalkyl)-1-pyrrolidinyl, 4-hydroxy-1-piperidinyl, 4-(lower alkoxy)-1-piperidinyl, 4-morpholinyl or 2,6-di(lower alkyl)-4-morpholinyl.

11. A process according to any one of claims 1–7, wherein m and q signify the number 0, n signifies the number 1 and $R^1$ signifies hydroxy or lower alkoxy.

12. A process according to claim 1, characterized in that 10-chloro-6,7-dihydro-N-(2-methoxyethyl)-4-oxo-3-phenyl-4H-benzo[a]quinolizine-1-carboxamide is manufactured.

13. A process according to claim 1, characterized in that 1-[(10-chloro-6,7-dihydro-4-oxo-3-phenyl-4H-benzo[a]quinolizin-1-yl)carbonyl]-4-piperidinol is manufactured.

14. A process according to claim 1, characterized in that (4,5-dihydro-7-oxo-8-phenyl-7H-thieno[2,3-a]quinolizin-10-yl)methyl-4-morpholinecarboxylate is manufactured.

15. A process according to claim 1, characterized in that 4-[(6,7-dihydro-4-oxo-3-phenyl-10-chloro-4H-benzo[a]quinolizin-1-yl)carbonyl]-2,6-dimethylmorpholine is manufactured.

16. A process according to claim 1, characterized in that (S)-1-[(7-oxo-8-phenyl-7H-thieno[2,3-a]quinolizin-10-yl)carbonyl]-2-pyrrolidinemethanol is manufactured.

17. A process according to claim 1, characterized in that (S)-2-methoxymethyl-1-[(7-oxo-8-phenyl-7H-thieno[2,3-a]quinolizin-10-yl)carbonyl]pyrrolidine is manufactured.

18. A process according to claim 1, characterized in that 1-[(10-chloro-4-oxo-3-phenyl-4H-benzo[a]quinolizin-1-yl)carbonyl]-3-methoxypyrrolidine is manufactured.

19. A process according to claim 1, characterized in that (S)-1-[(10-chloro-4-oxo-3-phenyl-4H-benzo[a]quinolizin-1-yl)carbonyl]-2-pyrrolidinemethanol is manufactured.

20. A process according to claim 1, characterized in that cis-4-[(4,5-dihydro-7-oxo-8-phenyl-7H-thieno[2,3-a]quinolizin-10-yl)carbonyl]-2,6-dimethylmorpholine is manufactured.

21. A process according to claim 1, characterized in that 1-[(10-chloro-6,7-dihydro-4-oxo-3-phenyl-4H-benzo[a]quinolizin-1-yl)carbonyl]-4-methoxypiperidine is manufactured.

22. A process according to claim 1, characterized in that 1-[(10-chloro-3-phenyl-4-oxo-4H-benzo[a]quinolizin-1-yl)carbonyl]-4-methoxypiperidine is manufactured.

23. A process according to claim 1, characterized in that N-ethyl-N-(2-methoxyethyl)-7-oxo-8-phenyl-7H-thieno[2,3-a]quinolizine-10-carboxamide is manufactured.

24. A process according to claim 1, characterized in that N-(2-methoxyethyl)-N-methyl-7-oxo-8-phenyl-7H-thieno[2,3-a]quinolizine-10-carboxamide is manufactured.

25. A process according to claim 1, characterized in that (R)-2-(methoxymethyl)-1-[(7-oxo-8-phenyl-7H-thieno[2,3-a]quinolizin-10-yl)carbonyl]pyrrolidine is manufactured.

26. A process according to claim 1, characterized in that 1-[(10-chloro-6,7-dihydro-4-oxo-3-phenyl-4H-benzo[a]quinolizin-1-yl)carbonyl]-3-methoxypyrrolidine is manufactured.

27. A process according to claim 1, characterized in that 1-[(10-chloro-6,7-dihydro-4-oxo-3-phenyl-4H-benzo[a]quinolizin-1-yl)carbonyl]-3-ethoxypyrrolidine is manufactured.

28. A process according to claim 1, characterized in that (R)-3-methoxy-1-[(7-oxo-8-phenyl-7H-thieno[2,3-a]quinolizin-10-yl)carbonyl]pyrrolidine is manufactured.

29. A process according to claim 1, characterized in that (S)-3-methoxy-1-[(7-oxo-8-phenyl-7H-thieno[2,3-a]quinolizin-10-yl)carbonyl]pyrrolidine is manufactured.

30. A process according to claim 1, characterized in that (S)-1-[(10-chloro-6,7-dihydro-4-oxo-3-phenyl-4H-benzo[a]quinolizin-1-yl)carbonyl]-2-(methoxymethyl)pyrrolidine is manufactured.

31. A process according to claim 1, characterized in that 1-[(4,5-dihydro-7-oxo-8-phenyl-7H-thieno[2,3-a]quinolizin-10-yl)carbonyl]-3-methoxypyrrolidine is manufactured.

32. A process according to claim 1, characterized in that 3-methoxy-1-[(7-oxo-8-phenyl-7H-thieno[2,3-a]quinolizin-10-yl)carbonyl]azetidine is manufactured.

33. A process according to claim 1, characterized in that (R)-1-[(10-chloro-4-oxo-3-phenyl-4H-benzo[a]quinolizin-1-yl)carbonyl]-2-pyrrolidinemethanol is manufactured.

34. A process according to claim 1, characterized in that (S)-1-[(10-chloro-4-oxo-3-phenyl-4H-benzo[a]quinolizin-1-yl)carbonyl]-2-(methoxymethyl)pyrrolidine is manufactured.

35. A process according to claim 1, characterized in that (R)-1-[(10-chloro-4-oxo-3-phenyl-4H-benzo[a]quinolizin-1-yl)carbonyl]-2-(methoxymethyl)pyrrolidine is manufactured.

**Revendications pour les Etats Contractants: BE, CH, DE, FR, IT, LI, LU, NL, SE**

1. Composés de formule générale

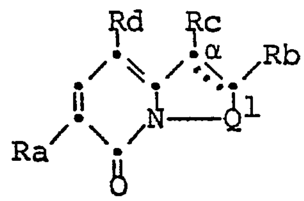

I

dans laquelle $Q^1$ forme avec l'atome d'azote un groupe de formule $>N-CH_2-CH_2-$ (a), $>N-CH_2CH_2CH_2-$ (b), $>N-CH=CH-$ (c), $>N-CH_2-CH=CH-$ (d), $>N-CH_2-S(O)_p-$ (e), $>N-CH_2CH_2-S(O)_p-$ (f) ou $>N-CH=CH-S(O)_p-$ (g), p est égal à 0, 1 ou 2 et Ra représente un groupe phényle, pyridyle ou thiényle éventuellement substitué par des halogènes, des groupes trifluorométhyle, nitro, alkyle inférieurs ou alcoxy inférieurs, Rb et Rc forment ensemble et avec l'atome de carbone marqué α un groupe de formule $>C_\alpha-S-CH=CH-$ (h), $>C_\alpha-CH=CH-S-$ (i) ou $>C_\alpha-CH=CH-CH=CH-$ (j) éventuellement substitué par des halogènes, des groupes trifluorométhyle, alkyle inférieurs, alcoxy inférieurs, nitro, amino ou mono- ou di-(alkyle inférieur)-amino, le trait en pointillé représente une liaison supplémentaire, Rd représente le groupe de formule $-(A^1)_m-(CO)_n-(Q^2A^2)_q-R^1$, m, n et q sont égaux chacun à 0 ou 1, $A^1$ représente un groupe alkylène inférieur, $A^2$ un groupe alkylène inférieur, une liaison directe ou le groupe $-CO-$, $Q^2$ représente un atome d'oxygène ou le groupe $-NR^2$, $R^1$ représente l'hydrogène, un groupe hydroxy, cyano, nitro, un halogène, un groupe alcoxy inférieur, alkyle inférieur, (alcoxy inférieur)-carbonyle, aryle, un groupe de formule $-NR^3R^4$ ou un hétérocycle saturé, partiellement insaturé ou aromatique à 5 chaînons, relié par l'intermédiaire d'un atome de carbone, éventuellement substitué par un ou deux groupes alkyle inférieurs et éventuellement substitué par un groupe cycloalkyle en C 3–C 6, hydroxy, alcoxy inférieur, alcanoyloxy inférieur, hydroxyalkyle inférieur, alcoxyalkyle inférieur, alcanoyloxyalkyle inférieur, alcoxycarbonyle inférieur, alcanoyle inférieur, carbamoyle, mono- ou di-(alkyle inférieur)-carbamoyle, oxo- ou alkylène-dioxy, $R^2$ représente l'hydrogène, un groupe alkyle inférieur ou aryle, $R^3$ et $R^4$ représentent chacun l'hydrogène, un groupe alkyle inférieur, alcoxyalkyle inférieur, dialcoxyalkyle inférieur, alkylène-dioxyalkyle inférieur, cyanalkyle inférieur, halogénoalkyle inférieur, hydroxyalkyle inférieur, dihydroxyalkyle inférieur, alcanoyle inférieur, alcoxycarbonyle inférieur ou un groupe cycloalkyle en C 3–7 C éventuellement substitué par des groupes hydroxy, alcoxy inférieurs, alcanoyloxy inférieurs, hydroxyalkyle inférieurs, alcoxyalkyle inférieurs, alcanoyloxyalkyle inférieurs, oxo, carbamoyle, mono- ou di-(alkyle inférieur)-carbamoyle ou alkylène-dioxy inférieurs ou forme avec l'atome d'azote un hétérocycle azoté saturé de 3 à 7 chaînons éventuellement substitué par un ou deux groupes alkyle inférieurs et éventuellement substitué par un ou deux groupes hydroxy, alcoxy inférieurs, alcanoyloxy inférieurs, hydroxyalkyle inférieurs, alcoxyalkyle inférieurs, alcanoyloxyalkyle inférieurs, alcoxycarbonyle inférieurs, alcanoyle inférieurs, carbamoyle, mono- ou di-(alkyle inférieur)-carbamoyle, oxo ou alkylène-dioxy inférieurs et qui peut encore contenir en tant que chaînon cyclique un atome d'oxygène ou de soufre ou le groupe $>N-R^5$, et $R^5$ représente l'hydrogène, un groupe alkyle inférieur, hydroxyalkyle inférieur, alcoxyalkyle inférieur, alcanoyloxyalkyle inférieur, alcanoyle inférieur, alcoxycarbonyle inférieur, carbamoyle ou mono- ou di-(alkyle inférieur)-carbamoyle, sous réserve toutefois que n est égal à 0 lorsque q est égal à 1 et que $A^2$ représente le groupe $-CO-$, que $R^1$ a une signification autre que cyano, nitro, halogène ou alcoxycarbonyle inférieur lorsque q est égal à 0 et que n est égal

à 1 ou que q est égal à 1 et que $A^2$ représente le groupe –CO–, et que $R^1$ a une signification autre que hydroxy, cyano, nitro, halogène, alcoxycarbonyle inférieur, alcoxy inférieur et $-NR^3R^4$ lorsque q est égal à 1 et que $A^2$ représente une liaison directe, et les groupes qualifiés d'"inférieurs" contiennent au maximum 7 atomes de carbone,
et les sels acceptables pour l'usage pharmaceutique, formés par addition avec des acides, des composés de formule I contenant un ou plusieurs substituants basiques.

2. Composés selon la revendication 1, dans lesquels Ra représente un groupe phényle éventuellement substitué par des halogènes, des groupes trifluorométhyle, nitro, alkyle inférieurs ou alcoxy inférieurs, Rb et Rc forment ensemble et avec l'atome de carbone marqué α un groupe de formule $>C_\alpha-S-CH=CH-$ (h), $>C_\alpha-CH=CH-S-$ (i) ou $>C_\alpha-CH=CH-CH=CH-$ (j) éventuellement substitué par des halogènes, des groupes trifluorométhyle, alkyle inférieurs, alcoxy inférieurs, nitro, amino ou mono- ou di-(alkyle inférieur)-amino, et le trait en pointillé représente une liaison supplémentaire, $A^2$ représente un groupe alkylène inférieur ou le groupe –CO–, $R^1$ représente l'hydrogène, un groupe hydroxy, cyano, nitro, un halogène, un groupe alcoxy inférieur, alkyle inférieur, alcoxycarbonyle inférieur, aryle, un groupe de formule $-NR^3R^4$ ou un hétérocycle partiellement insaturé ou aromatique à 5 chaînons, relié par l'intermédiaire d'un atome de carbone, éventuellement substitué par un ou deux groupes alkyle inférieurs, $R^3$ et $R^4$ représentent chacun l'hydrogène, un groupe alkyle inférieur, un groupe cycloalkyle en C 5–C 6 ou un groupe alcoxycarbonyle inférieur ou forment ensemble et avec l'atome d'azote un hétérocycle azoté saturé à 5 ou 6 chaînons éventuellement substitué par un ou deux groupes alkyle inférieurs et qui peut encore être substitué sur un atome de carbone par un groupe oxo, hydroxy, alcoxy inférieur, alcoxyalkyle inférieur, hydroxyalkyle inférieur, alcoxycarbonyle inférieur, carbamoyle ou mono- ou di-(alkyle inférieur)-carbamoyle ou qui peut encore contenir en tant que chaînon cyclique un atome d'oxygène ou de soufre ou le groupe $>N-R^5$, et $R^5$ représente l'hydrogène, un groupe alkyle inférieur, hydroxyalkyle inférieur, alcoxyalkyle inférieur, alcanoyle inférieur, alcoxycarbonyle inférieur, carbamoyle ou mono- ou di-(alkyle inférieur)-carbamoyle.

3. Composés selon la revendication 1 ou 2, dans lesquels $Q^1$ forme avec l'atome d'azote le groupe de formule $>N-CH_2CH_2-$ (a) ou $>N-CH=CH-$ (c).

4. Composés selon l'une des revendications 1 à 3, dans lesquels Ra représente un groupe phényle portant éventuellement un substituant m-halogéno ou m-trifluorométhyle.

5. Composés selon la revendication 4, dans lesquels Ra représente un groupe phényle.

6. Composés selon l'une des revendications 1 à 5, dans lesquels Rb et Rc forment ensemble et avec l'atome de carbone marqué α un groupe de formule $>C_\alpha-S-CH=CH-$ ou $>C_\alpha-CH=CH-CH=CH-$ éventuellement substitué par des halogènes et le trait en pointillé représente une liaison supplémentaire.

7. Composés selon la revendication 6, dans lesquels Rb et Rc forment avec l'atome de carbone marqué α le groupe de formule $>C_\alpha-S-CH=CH-$ ou $>C_\alpha-CH=CCl-CH=CH-$.

8. Composés selon l'une des revendications 1 et 3 à 7, dans lesquels $Q^2$ représente un atome d'oxygène, $A^2$ le groupe –CO–, $R^1$ le groupe $-NR^3R^4$, $R^3$ un groupe alcoxy alkyle inférieur et $R^4$ l'hydrogène ou un groupe alkyle inférieur, ou bien $R^3$ et $R^4$ forment ensemble et avec l'atome d'azote un hétérocycle azoté saturé à 4, 5 ou 6 chaînons, éventuellement substitué par un ou deux groupes alkyle inférieurs et éventuellement substitué par un groupe hydroxy, alcoxy inférieur, hydroxyalkyle inférieur ou alcoxyalkyle inférieur et qui peut encore contenir en tant que chaînons cyclique un atome d'oxygène, et ou bien m et q sont égaux à 0 et n à 1, ou bien m et q sont égaux à 1 et n à 0.

9. Composés selon la revendication 8, dans lesquels $A^1$ représente un groupe méthylène et $R^3$ un groupe alcoxyalkyle inférieur et $R^4$ l'hydrogène ou un groupe alkyle inférieur ou bien $R^3$ et $R^4$ forment ensemble et avec l'atome d'azote un groupe 1-azétidinyle, 1-pyrrolidinyle, 1-pipéridinyle ou 4-morpholinyle éventuellement substitué par un ou deux groupes alkyle inférieurs et éventuellement substitué par un groupe hydroxy, alcoxy inférieur, hydroxyalkyle inférieur ou alcoxyalkyle inférieur.

10. Composés selon la revendication 9, dans lesquels $R^3$ représente un groupe 2-(alcoxy inférieur)-éthyle et $R^4$ l'hydrogène ou un groupe alkyle inférieur ou bien $R^3$ et $R^4$ forment ensemble et avec l'atome d'azote un groupe 3-(alcoxy inférieur)-1-azétidinyle, 3-(alcoxy inférieur)-1-pyrrolidinyle, 2-(alcoxyalkyle inférieur)-1-pyrrolidinyle, 2-(hydroxyalkyle inférieur)-1-pyrrolidinyle, 4-hydroxy-1-pipéridinyle, 4-(alcoxy inférieur)-1-pipéridinyle, 4-morpholinyle ou 2,6-di-(alkyle inférieur)-4-morpholinyle.

11. Composés selon l'une des revendications 1 à 7, dans lesquels m et q sont égaux à 0, n est égal à 1 et $R^1$ représente un groupe hydroxy ou alcoxy inférieur.

12. Le 10-chloro-6,7-dihydro-N-(2-méthoxyéthyl)-4-oxo-3-phényl-4H-benzo[a]quinolizine-1-carboxamide.

13. Le 1-[(10-chloro-6,7-dihydro-4-oxo-3-phényl-4H-benzo[a]quinolizine-1-yl)-carbonyl]-4-pipéridinol.

14. Le (4,5-dihydro-7-oxo-8-phényl-7H-thiéno[2,3-a]quinolizine-10-yl)-méthyl-4-morpholine-carboxylate.

15. La 4-[(6,7-dihydro-4-oxo-3-phényl-10-chloro-4H-benzo[a]quinolizine-1-yl)-carbonyl]-2,6-diméthylmorpholine.

16. Le (S)-1-[(7-oxo-8-phényl-7H-thiéno[2,3-a]quinolizine-10-yl)-carbonyl]-2-pyrrolidine-méthanol.

17. La (S)-2-méthoxyméthyl-1-[(7-oxo-8-phényl-7H-thiéno[2,3-a]quinolizine-10-yl)-carbonyl]-pyrrolidine.

18. La 1-[(10-chloro-4-oxo-3-phényl-4H-benzo[a]quinolizine-1-yl)-carbonyl]-3-méthoxypyrrolidine.

19. Le (S)-1-[(10-chloro-4-oxo-3-phényl-4H-benzo[a]quinolizine-1-yl)-carbonyl]-2-pyrrolidine-méthanol.

20. La cis-4-[(4,5-dihydro-7-oxo-8-phényl-7H-thiéno[2,3-a]quinolizine-10-yl)-carbonyl]-2,6-diméthylmorpholine.

21. La 1-[(10-chloro-6,7-dihydro-4-oxo-3-phényl-4H-benzo[a]quinolizine-1-yl)-carbonyl]-4-méthoxypipéridine.

22. La 1-[(10-chloro-3-phényl-4-oxo-4H-benzo[a]quinolizine-1-yl)-carbonyl]-4-méthoxypipéridine.

23. Le N-éthyl-N-(2-méthoxyéthyl)-7-oxo-8-phényl-7H-thiéno[2,3-a]quinolizine-10-carboxamide.

24. Le N-(2-méthoxyéthyl)-N-méthyl-7-oxo-8-phényl-7H-thiéno[2,3-a]quinolizine-10-carboxamide.

25. La (R)-2-(méthoxyméthyl)-1-[(7-oxo-8-phényl-7H-thiéno[2,3-a]quinolizine-10-yl)-carbonyl]-pyrrolidine.

26. La 1-[(10-chloro-6,7-dihydro-4-oxo-3-phényl-4H-benzo[a]quinolizine-1-yl)-carbonyl]-3-méthoxypyrrolidine.

27. La 1-[(10-chloro-6,7-dihydro-4-oxo-3-phényl-4H-benzo[a]quinolizine-1-yl)-carbonyl]-3-éthoxypyrrolidine.

28. La (R)-3-méthoxy-1-[(7-oxo-8-phényl-7H-thiéno[2,3-a]quinolizine-10-yl)-carbonyl]-pyrrolidine.

29. La (S)-3-méthoxy-1-[(7-oxo-8-phényl-7H-thiéno[2,3-a]quinolizine-10-yl)-carbonyl]-pyrrolidine.

30. La (S)-1-[(10-chloro-6,7-dihydro-4-oxo-3-phényl-4H-benzo[a]quinolizine-1-yl)-carbonyl]-2-(méthoxyméthyl)-pyrrolidine.

31. La 1-[(4,5-dihydro-7-oxo-8-phényl-7H-thiéno[2,3-a]quinolizine-10-yl)-carbonyl]-3-méthoxypyrrolidine.

32. La 3-méthoxy-1-[(7-oxo-8-phényl-7H-thiéno[2,3-a]quinolizine-10-yl)-carbonyl]-azétidine.

33. Le (R)-1-[(10-chloro-4-oxo-3-phényl-4H-benzo[a]quinolizine-1-yl)-carbonyl]-2-pyrrolidine-méthanol.

34. La (S)-1-[(10-chloro-4-oxo-3-phényl-4H-benzo[a]quinolizine-1-yl)-carbonyl]-2-(méthoxyméthyl)-pyrrolidine.

35. La (R)-1-[(10-chloro-4-oxo-3-phényl-4H-benzo[a]quinolizine-1-yl)-carbonyl]-2-(méthoxyméthyl)-pyrrolidine.

36. Composés de formule générale

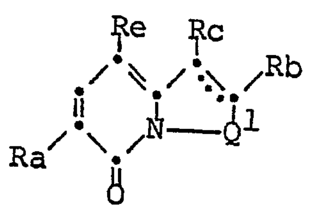

VIII

dans laquelle Re représente le groupe $-(A^1)_m-OOC-X^2$, $-(A^1)_m-(CO)_n-(Q^2A^{22})_q-NR^{31}R^{41}$, $-(A^1)_m-N=C=O$, $-(A^1)_m-CO-X^1$, $-(A')_m-CO-CH^{-}-N^{+}_2$ ou $-CH(OR^7)OR^8$ et $A^1$, $Q^1$, $Q^2$, Ra, Rb, Rc, m, n, q et le trait en pointillé ont les significations indiquées dans la revendication 1, et A', $A^{22}$, $R^7$, $R^8$, $R^{31}$, $R^{41}$, $X^1$ et $X^2$ ont les significations indiquées dans la revendication 41.

37. Composés de formule générale

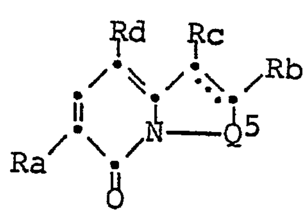

XV

dans laquelle $Q^5$ forme avec l'atome d'azote le groupe de formule $-CH_2-CHX^1-S(O)_p-$ ou un groupe diméthylène substitué par un halogène et $X^1$ représente un halogène, et Ra, Rb, Rc, Rd et p ont les significations indiquées dans la revendication 1.

38. Composés de formules générales

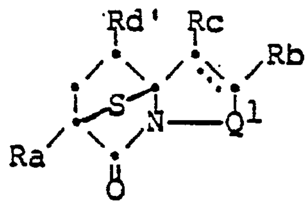

XIX

et

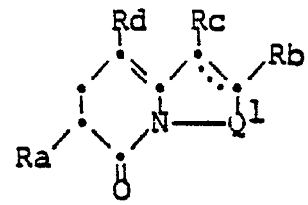

XXa

dans lesquelles Ra, Rb, Rc, Rd, $Q^1$ et le trait en pointillé ont les significations indiquées dans la revendication 1 et Rd' les significations indiquées dans la revendication 41.

39. Composés selon l'une des revendications 1 à 35, pour l'utilisation en tant que substances actives thérapeutiques.

40. Composés selon l'une des revendications 1 à 35, pour l'utilisation en tant que substances actives myorelaxantes, sédatives-hypnotiques, anxiolytiques et/ou anticonvulsives.

41. Procédé de préparation des composés selon l'une des revendications 1 à 35 et de leurs sels acceptables pour l'usage pharmaceutique formés par addition avec des acides, caractérisé en ce que:

a) on fait réagir un composé de formule générale

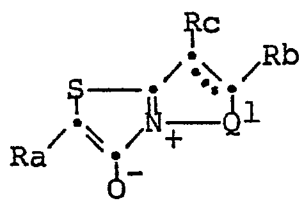

II

dans laquelle $Q^1$, Ra, Rb, Rc et le trait en pointillé ont les significations indiquées dans la revendication 1, à température élevée, avec un composé de formule générale

$HC\equiv C-Rd'$ III ou $H_2C=CH-Rd'$ IV

dans laquelle Rd' représente un groupe cyano, nitro ou le groupe de formule $-CO-(Q^2A^2)_q-R^1$ et q, $A^2$, $Q^2$ et $R^1$ ont les significations indiquées dans la revendication 1,

ou avec le phénylvinylsulfoxyde et le cas échéant on traite le produit de cycloaddition ainsi obtenu par une base forte, ou bien

b) on fait réagir un composé de formule générale

ROOC–C(Ra)=CHR' V

dans laquelle R représente un groupe alkyle inférieur et R' l'hydrogène ou un groupe alcoxy inférieur, et Ra a les significations indiquées dans la revendication 1, à température élevée lorsque R' représente l'hydrogène ou bien en présence d'une base forte lorsque R' représente un groupe alcoxy inférieur, avec un composé de formule générale

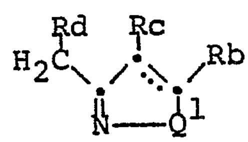

VI

dans laquelle $Q^1$, Rb, Rc, Rd et le trait en pointillé ont les significations indiquées dans la revendication 1, et

lorsque R' représente l'hydrogène, on soumet le produit de cyclocondensation ainsi obtenu à déshydrogénation, ou bien

c) on hydrolyse un composé de formule I contenant un groupe carboxy estérifié, ou bien

d) on estérifie un acide carboxylique de formule générale

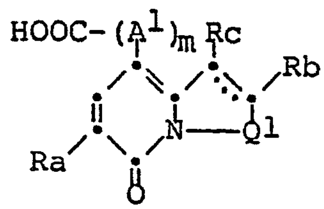

Ia

dans laquelle $A^1$, $Q^1$, Ra, Rb, Rc, m et le trait en pointillé ont les significations indiquées dans la revendication 1,
par un alcool de formule générale
$HO–A^{21}–R^1$ VII
dans laquelle $A^{21}$ représente un groupe alkylène inférieur ou une liaison directe et $R^1$ a les significations indiquées dans la revendication 1, ou bien
e) on convertit un acide carboxylique de formule la ci-dessus ou un acide carboxylique de formule générale

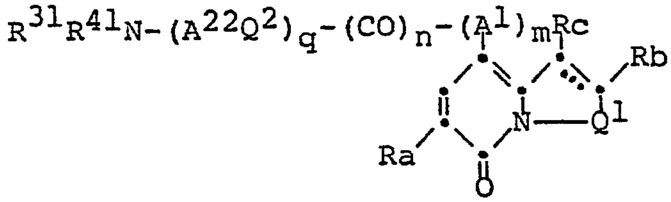

VIIIa

dans laquelle $A^{22}$ représente un groupe alkylène inférieur ou le groupe –CO– et $R^{31}$ et $R^{41}$ forment ensemble et avec l'atome d'azote un hétérocycle azoté saturé de 3 à 7 chaînons éventuellement substitué par un ou deux groupes alkyle inférieurs et par un groupe carboxy et qui peut encore contenir en tant que chaînon cyclique un atome d'oxygène ou de soufre ou le groupe >N–$R^5$, et $A^1$, $Q^1$, $Q^2$, Ra, Rb, Rc, $R^5$, m, n, q et le trait en pointillé ont les significations indiquées dans la revendication 1, ou un dérivé réactif d'un tel acide, par réaction avec une amine de formule générale
$HNR^2–A^{21}–R^1$ IX ou $HNR^3R^4$ X
dans lesquelles $A^{21}$ a les significations indiquées ci-dessus et $R^1$, $R^2$, $R^3$ et $R^4$ les significations indiquées dans la revendication 1,
ou respectivement avec l'ammoniac ou une mono- ou di-(alkyle inférieur)-amine, en l'amide correspondant, ou bien
f) on fait réagir un composé de formule générale

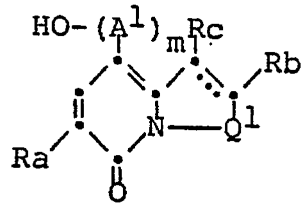

Ib'

dans laquelle $A^1$, $Q^1$, Ra, Rb, Rc, m et le trait en pointillé ont les significations indiquées dans la revendication 1, en présence d'une base, avec un composé de formule générale
$X–A^{21}–R^1$ XI
dans laquelle X représente un groupe éliminable, $A^{21}$ a les significations indiquées ci-dessus et $R^1$ les significations indiquées dans la revendication 1,
ou bien on fait réagir un composé de formule I contenant un groupe hydroxy libre avec un composé de formule générale
R–X XII
dans laquelle R représente un groupe alkyle inférieur et X a les significations indiquées ci-dessus, ou bien
g) on fait réagir un composé de formule générale

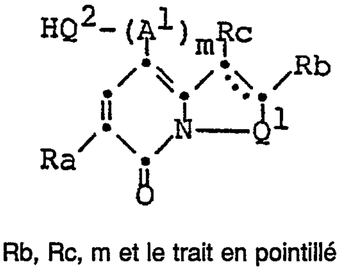

Ib

dans laquelle $A^1$, $Q^1$, $Q^2$, Ra, Rb, Rc, m et le trait en pointillé ont les significations indiquées dans la revendication 1,
en présence d'un accepteur d'acide, avec un dérivé réactif d'un acide carboxylique de formule générale

$R^1$–COOH XIII

dans laquelle $R^1$ a les significations indiquées dans la revendication 1, ou bien

h) on fait réagir un composé de formule générale

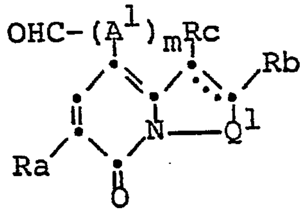

Ic

dans laquelle $A^1$, $Q^1$, Ra, Rb, Rc, m et le trait en pointillé ont les significations indiquées dans la revendication 1,

en présence d'un agent réducteur, avec une amine de formule IX ou X ci-dessus, ou bien

i) on réduit un composé de formule générale

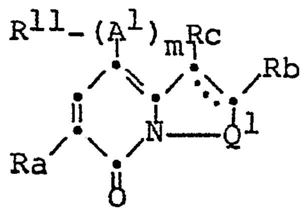

Id

dans laquelle $R^{11}$ représente un groupe nitro, cyano ou alcoxycarbonyle inférieur et $A^1$, $Q^1$, Ra, Rb, Rc, m et le trait en pointillé ont les significations indiquées dans la revendication 1,

ou un composé de formule Ia ci-dessus ou un dérivé réactif d'un tel composé, ou bien

j) on oxyde un alcool de formule Ib′ ou un alcool de formule générale

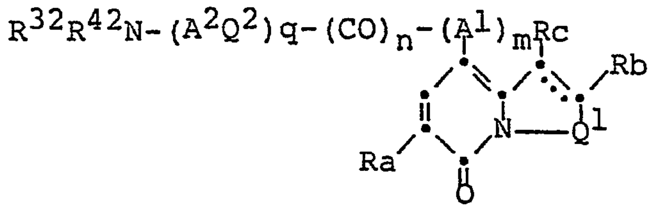

Ie

dans laquelle $A^1$, $A^2$, $Q^1$, $Q^2$, Ra, Rb, Rc, m, n, q et le trait en pointillé ont les significations indiquées dans la revendication 1, et $R^{32}$ et $R^{42}$ forment ensemble et avec l'atome d'azote un hétérocycle azoté saturé de 3 à 7 chaînons éventuellement substitué par un ou deux groupes alkyle inférieurs et par un groupe hydroxy et qui peut encore contenir en tant que chaînon cyclique un atome d'oxygène ou de soufre ou le groupe >N–$R^5$, et $R^5$ a les significations indiquées dans la revendication 1, ou bien

k) on fait réagir un isocyanate de formule générale

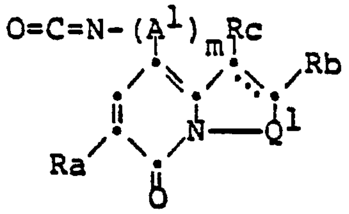

VIIIb

ou O=C–N–$R^{33}$ XIV

dans lesquelles $A^1$, $Q^1$, Ra, Rb, Rc, m et le trait en pointillé ont les significations indiquées dans la revendication 1 et $R^{33}$ représente l'hydrogène, un groupe alkyle inférieur ou cycloalkyle en C 3–C 7, avec un alcool inférieur ou une amine de formule X ci-dessus ou respectivement avec un composé de formule Ib ci-dessus, ou bien

l) on fait réagir un composé de formule générale

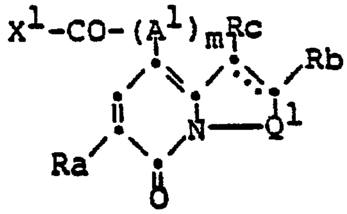

VIIIc

dans laquelle $X^1$ représente un atome d'halogène et $A^1$, $Q^1$, Ra, Rb, Rc, m et le trait en pointillé ont les significations indiquées dans la revendication 1,
avec un halogénure d'alkyl-magnésium, ou bien
m) on soumet à déshydrohalogénation en présence d'une base un composé de formule générale

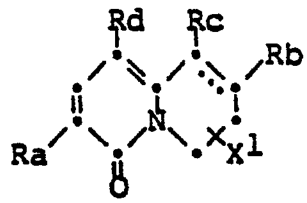

XVa

ou

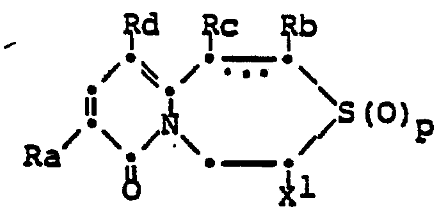

XVb

dans lesquelles Ra, Rb, Rc, Rd, $X^1$, le trait en pointillé et p ont les significations indiquées dans la revendication 1, ou bien
n) on soumet à S-oxydation un composé de formule générale

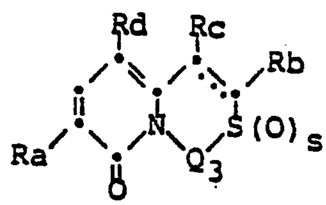

If

dans laquelle $Q^3$ représente un groupe $-CH_2-$, $-CH_2CH_2-$ ou $-CH=CH-$ et s est égal à 0 ou 1, et Ra, Rb, Rc, Rd, et le trait en pointillé ont les significations indiquées dans la revendication 1, ou bien
o) on chauffe un composé de formule générale

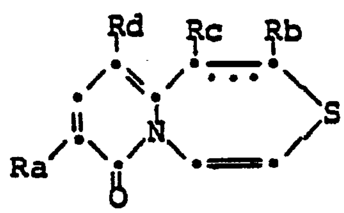

Ig

dans laquelle Ra, Rb, Rc, Rd et le trait en pointillé ont les significations indiquées dans la revendication 1, ou bien
p) on halogène sur le cycle thiophène un composé de formule générale

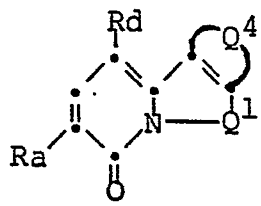

Ih

dans laquelle $Q^4$ représente un groupe (h) ou (i) tel que défini dans la revendication 1 et $Q^1$, Ra et Rb ont les significations indiquées ci-dessus, ou bien

q) on fait réagir un composé de formule VIIIc ci-dessus, en présence d'une base, avec un composé de formule générale

HYN=C($NH_2$)–R″ XVI, $H_2N$–CHR″–CHR‴–Y′H XVII

ou $H_2N$–NH–C(R″)=Y″ XVIII

dans lesquelles Y représente un atome d'oxygène ou le groupe –NR‴–, Y′ représente un atome d'oxygène ou le groupe –NH–, Y″ représente un atome d'oxygène ou de soufre et R‴ et R″ représentant chacun l'hydrogène ou un groupe alkyle inférieur,

et on cyclise le produit obtenu, ou bien

r) on fait réagir un composé de formule générale

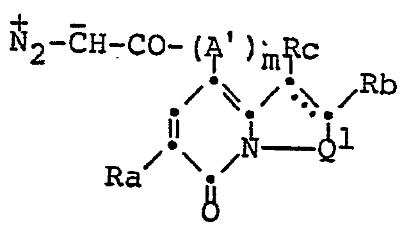

VIIId

dans laquelle A′ représente un groupe alkylène en C 1–C 6 et $Q^1$, Ra, Rb, Rc, le trait en pointillé et m ont les significations indiquées dans la revendication 1, avec un alcool inférieur, ou bien

s) on soumet à décarboxylation un acide carboxylique de formule Ia dans laquelle m est égal à 0, ou bien

t) on halogène sur le cycle pyridone un composé de formule I dans laquelle Rd représente l'hydrogène, ou bien

u) dans un composé de formule générale

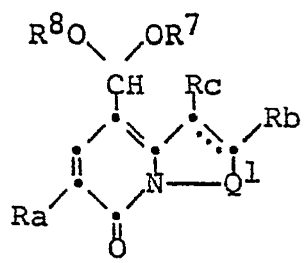

VIIIe

dans laquelle $R^7$ et $R^8$ représentent chacun un groupe alkyle inférieur ou forment ensemble un groupe alkylène inférieur et $Q^1$, Ra, Rb, Rc et le trait en pointillé ont les significations indiquées dans la revendication 1,

on scinde le groupe acétal, ou bien

v) on hydrogène un composé de formule I dans laquelle $Q^1$ forme avec l'atome d'azote le groupe >N–CH=CH–, ou bien

w) on fait réagir un composé de formule générale

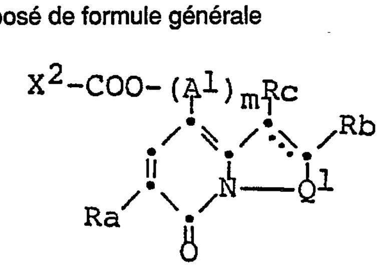

VIIIf

dans laquelle $X^2$ représente un groupe phénoxy et $A^1$, $Q^1$, Ra, Rb, Rc, le trait en pointillé et m ont les significations indiquées dans la revendication 1,

avec une amine de formule X ci-dessus, et

x) si on le désire, on convertit un composé obtenu, répondant à la formule I et portant un substituant basique, par addition avec un acide, en un sel acceptable pour l'usage pharmaceutique.

42. Médicament contenant un composé selon l'une des revendications 1 à 35 et un véhicule inerte du point de vue thérapeutique.

43. Agent actif myorelaxant, sédatif-hypnotique, anxiolytique et/ou anticonvulsif, contenant un composé selon l'une des revendications 1 à 35 et un véhicule inerte du point de vue thérapeutique.

44. Utilisation des composés selon l'une des revendications 1 à 35 pour le traitement ou la prévention de maladies.

45. Utilisation des composés selon l'une des revendications 1 à 35 pour le traitement ou la prévention des tensions musculaires, des états de tension, de l'insomnie, des états d'angoisse et/ou des convulsions.

46. Utilisation des composés selon l'une des revendications 1 à 35 pour la préparation d'agents actifs myorelaxants, sédatifs-hypnotiques, anxiolytiques et/ou anticonvulsifs.

47. Utilisation des composés selon l'une des revendications 36 à 38 pour la préparation de substances actives thérapeutiques.

48. Utilisation des composés selon l'une des revendications 36 à 38 pour la préparation de composés selon l'une des revendications 1 à 35.

**Revendications pour l'Etat Contractant: AT**

1. Procédé de préparation des composés de formule générale

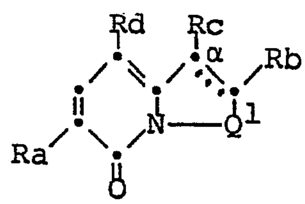

I

dans laquelle $Q^1$ forme avec l'atome d'azote un groupe de formule $>N-CH_2-CH_2-$ (a), $>N-CH_2CH_2CH_2-$ (b), $>N-CH=CH-$ (c), $>N-CH_2-CH=CH-$ (d), $>N-CH_2-S(O)_p-$ (e), $>N-CH_2CH_2-S(O)_p-$ (f) ou $>N-CH=CH-S(O)_p-$ (g), p est égal à 0, 1 ou 2 et Ra représente un groupe phényle, pyridyle ou thiényle éventuellement substitué par des halogènes, des groupes trifluorométhyle, nitro, alkyle inférieurs ou alcoxy inférieurs, Rb et Rc forment ensemble et avec l'atome de carbone marqué α un groupe de formule $>C_\alpha-S-CH=CH-$ (h), $>C_\alpha-CH=CH-S-$ (i) ou $>C_\alpha-CH=CH-CH=CH-$ (j) éventuellement substitué par des halogènes, des groupes trifluorométhyle, alkyle inférieurs, alcoxy inférieurs, nitro, amino ou mono- ou di-(alkyle inférieur)-amino, le trait en pointillé représente une liaison supplémentaire, Rd représente le groupe de formule $-(A^1)_m-(CO)_n-(Q^2A^2)_q-R^1$, m, n et q sont égaux chacun à 0 ou 1, $A^1$ représente un groupe alkylène inférieur, $A^2$ un groupe alkylène inférieur, une liaison directe ou le groupe $-CO-$, $Q^2$ représente un atome d'oxygène ou le groupe $-NR^2$, $R^1$ représente l'hydrogène, un groupe hydroxy, cyano, nitro, un halogène, un groupe alcoxy inférieur, alkyle inférieur, (alcoxy inférieur)-carbonyle, aryle, un groupe de formule $-NR^3R^4$ ou un hétérocycle saturé, partiellement insaturé ou aromatique à 5 chaînons, relié par l'intermédiaire d'un atome de carbone, éventuellement substitué par un ou deux groupes alkyle inférieurs et éventuellement substitué par un groupe cycloalkyle en C 3–C 6, hydroxy, alcoxy inférieur, alcanoyloxy inférieur, hydroxyalkyle inférieur, alcoxyalkyle inférieur, alcanoyloxyalkyle inférieur, alcoxycarbonyle inférieur, alcanoyle inférieur, carbamoyle, mono- ou di-(alkyle inférieur)-carbamoyle, oxo- ou alkylène-dioxy, $R^2$ représente l'hydrogène, un groupe alkyle inférieur ou aryle, $R^3$ et $R^4$ représentent chacun l'hydrogène, un groupe alkyle inférieur, alcoxyalkyle inférieur, dialcoxyalkyle inférieur, alkylène-dioxyalkyle inférieur, cyanalkyle inférieur, halogénoalkyle inférieur, hydroxyalkyle inférieur, dihydroxyalkyle inférieur, alcanoyle inférieur, alcoxycarbonyle inférieur ou un groupe cycloalkyle en C 3–C 7 éventuellement substitué par des groupes hydroxy, alcoxy inférieurs, alcanoyloxy inférieurs, hydroxyalkyle inférieurs, alcoxyalkyle inférieurs, alcanoyloxyalkyle inférieurs, oxo, carbamoyle, mono- ou di-(alkyle inférieur)-carbamoyle ou alkylène-dioxy inférieurs ou forme avec l'atome d'azote un hétérocycle azoté saturé de 3 à 7 chaînons éventuellement substitué par un ou deux groupes alkyle inférieurs et éventuellement substitué par un ou deux groupes hydroxy, alcoxy inférieurs, alcanoyloxy inférieurs, hydroxyalkyle inférieurs, alcoxyalkyle inférieurs, alcanoyloxyalkyle inférieurs, alcoxycarbonyle inférieurs, alcanoyle inférieurs, carbamoyle, mono- ou di-(alkyle inférieur)-carbamoyle, oxo ou alkylène-dioxy inférieurs et qui peut encore contenir en tant que chaînon cyclique un atome d'oxygène ou de soufre ou le troupe $>N-R^5$, et $R^5$ représente l'hydrogène, un groupe alkyle inférieur, hydroxyalkyle inférieur, alcoxyalkyle inférieur, alcanoyloxyalkyle inférieur, alcanoyle inférieur,

alcoxycarbonyle inférieur, carbamoyle ou mono- ou di-(alkyle inférieur)-carbamoyle, sous réserve toutefois que n est égal à 0 lorsque q est égal à 1 et que $A^2$ représente le groupe –CO–, que $R^1$ a une signification autre que cyano, nitro, halogène ou alcoxycarbonyle inférieur lorsque q est égal à 0 et que n est égal à 1 ou que q est égal à 1 et que $A^2$ représente le groupe –CO–, et que $R^1$ a une signification autre que hydroxy, cyano, nitro, halogène, alcoxycarbonyle inférieur, alcoxy inférieur et $-NR^3R^4$ lorsque q est égal à 1 et que $A^2$ représente une liaison directe, et les groupes qualifiés d'"inférieurs" contiennent au maximum 7 atomes de carbone,
et des sels acceptables pour l'usage pharmaceutique, formés par addition avec des acides, des composés de formule I contenant un ou plusieurs substituants basiques, caractérisé en ce que:
a) on fait réagir un composé de formule générale

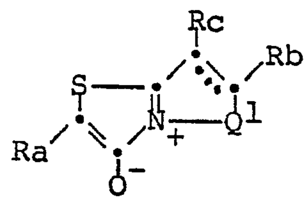

II

dans laquelle $Q^1$, Ra, Rb, Rc et le trait en pointillé ont les significations indiquées ci-dessus,
à température élevée, avec un composé de formule générale
HC≡C–Rd' III ou $H_2C$=CH–Rd' IV
dans lesquelles Rd' représente un groupe cyano, nitro ou le groupe de formule $-CO-(Q^2A^2)_q-R^1$ et q, $A^2$, $Q^2$ et $R^1$ ont les significations indiquées ci-dessus,
ou avec le phénylvinylsulfoxyde et le cas échéant, on traite le produit de cycloaddition ainsi obtenu par une base forte, ou bien
b) on fait réagir un composé de formule générale
ROOC–C(Ra)=CHR' V
dans laquelle R représente un groupe alkyle inférieur et R' l'hydrogène ou un groupe alcoxy inférieur et Ra a les significations indiquées ci-dessus,
à température élevée lorsque R' représente l'hydrogène, ou bien
en présence d'une base forte lorsque R' représente un groupe alcoxy inférieur, avec un composé de formule générale

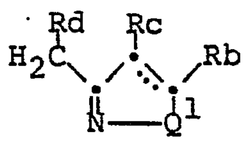

VI

dans laquelle $Q^1$, Rb, Rc, Rd et le trait en pointillé ont les significations indiquées ci-dessus, et, lorsque R' représente l'hydrogène, on soumet le produit de cyclocondensation ainsi obtenu à déshydrogénation, ou bien
c) on hydrolyse un composé de formule I qui contient un groupe carboxy estérifié, ou bien
d) on estérifie un acide carboxylique de formule générale

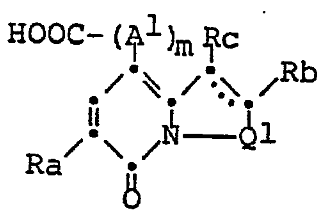

Ia

dans laquelle $A^1$, $Q^1$, Ra, Rb, Rc, m et le trait en pointillé ont les significations indiquées ci-dessus, par un alcool de formule générale
$HO-A^{21}-R^1$ VII
dans laquelle $A^{21}$ représente un groupe alkylène inférieur ou une liaison directe et $R^1$ a les significations indiquées ci-dessus, ou bien
e) on convertit un acide carboxylique de formule Ia ci-dessus ou un acide carboxylique de formule générale

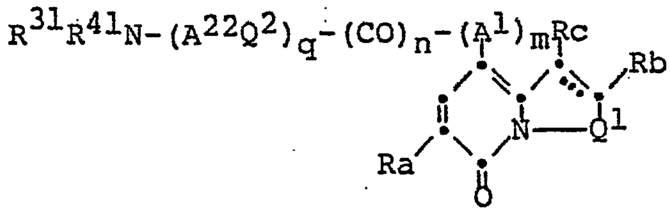

VIIIa

dans laquelle $A^{22}$ représente un groupe alkylène inférieur ou le groupe –CO– et $R^{31}$ et $R^{41}$ forment ensemble et avec l'atome d'azote un hétérocycle azoté saturé de 3 à 7 chaînons éventuellement substitué par un ou deux groupes alkyle inférieurs et par un groupe carboxy et qui peut encore contenir en tant que chaînon cyclique un atome d'oxygène ou de soufre ou le groupe >N–$R^5$, et $A^1$, $Q^1$, $Q^2$, Ra, Rb, Rc, $R^5$, m, n, q et le trait en pointillé ont les significations indiquées ci-dessus, ou un dérivé réactif d'un tel acide, par réaction avec une amine de formule générale

$HNR^2–A^{21}–R^1$ IX ou $HNR^3R^4$ X

dans lesquelles $A^{21}$, $R^1$, $R^2$, $R^3$ et $R^4$ ont les significations indiquées ci-dessus, ou respectivement avec l'ammoniac ou une mono- ou di-(alkyle inférieur)-amine, en l'amide correspondant, ou bien

f) on fait réagir un composé de formule générale

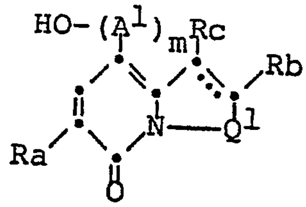

Ib'

dans laquelle $A^1$, $Q^1$, Ra, Rb, Rc, m et le trait en pointillé ont les significations indiquées ci-dessus, en présence d'une base, avec un composé de formule générale

$X–A^{21}–R^1$ XI

dans laquelle X représente un groupe éliminable, $A^{21}$ a les significations indiquées ci-dessus et $R^1$ les significations indiquées ci-dessus,

ou respectivement on fait réagir un composé de formule I contenant un groupe hydroxy libre avec un composé de formule générale

R–X XII

dans laquelle R représente un groupe alkyle inférieur et X a les significations indiquées ci-dessus, ou bien

g) on fait réagir un composé de formule générale

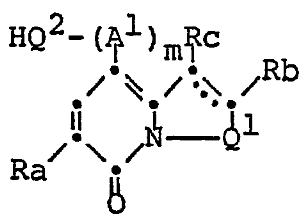

Ib

dans laquelle $A^1$, $Q^1$, $Q^2$, Ra, Rb, Rc, m et le trait en pointillé ont les significations indiquées ci-dessus, en présence d'un accepteur d'acide, avec un dérivé réactif d'un acide carboxylique de formule générale

$R^1–COOH$ XIII

dans laquelle $R^1$ a les significations indiquées ci-dessus, ou bien

h) on fait réagir un composé de formule générale

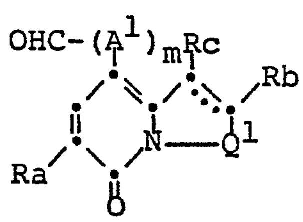

Ic

dans laquelle $A^1$, $Q^1$, Ra, Rb, Rc, m et le trait en pointillé ont les significations indiquées ci-dessus, en présence d'un agent réducteur, avec une amine de formule IX ou X ci-dessus, ou bien

i) on réduit un composé de formule générale

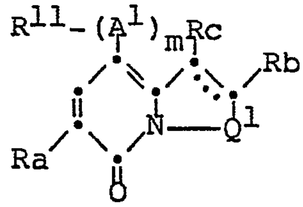

Id

dans laquelle $R^{11}$ représente un groupe nitro, cyano ou alcoxycarbonyle inférieur et $A^1$, $Q^1$, Ra, Rb, Rc, m et le trait en pointillé ont les significations indiquées ci-dessus, ou un composé de formule Ia ci-dessus ou un dérivé réactif d'un tel composé, ou bien

j) on oxyde un alcool de formule Ib′ ci-dessus ou un alcool de formule générale

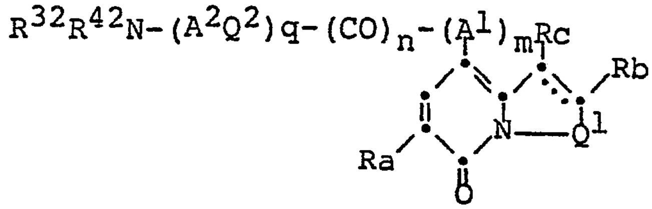

Ie

dans laquelle $A^1$, $A^2$, $Q^1$, $Q^2$, Ra, Rb, Rc, m, n, q et le trait en pointillé ont les significations indiquées dans la revendication 1 et $R^{32}$ et $R^{42}$ forment ensemble et avec l'atome d'azote un hétérocycle azoté saturé de 3 à 7 chaînons éventuellement substitué par un ou deux groupes alkyle inférieurs et par un groupe hydroxy et qui peut encore contenir en tant que chaînon cyclique un atome d'oxygène ou de soufre ou le groupe >N–$R^5$, et $R^5$ a les significations indiquées ci-dessus, ou bien

k) on fait réagir un isocyanate de formule générale

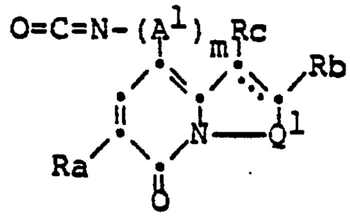

VIIIb

ou

O=C–N–$R^{33}$ XIV

dans lesquelles $A^1$, $Q^1$, Ra, Rb, Rc, m et le trait en pointillé ont les significations indiquées dans la revendication 1, et $R^{33}$ représente l'hydrogène, un groupe alkyle inférieur ou cycloalkyle en C 3–C 7, avec un alcool inférieur ou une amine de formule X ci-dessus ou respectivement avec un composé de formule Ib ci-dessus, ou bien

l) on fait réagir un composé de formule générale

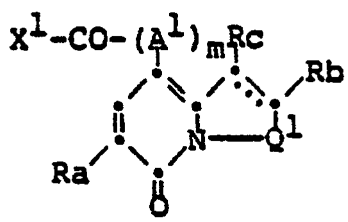

VIIIc

dans laquelle $X^1$ représente un atome d'halogène et $A^1$, $Q^1$, Ra, Rb, Rc, m et le trait en pointillé ont les significations indiquées ci-dessus,

avec un halogénure d'alkylmagnésium inférieur, ou bine

m) on soumet à déshydrohalogénation en présence d'une base un composé de formule générale

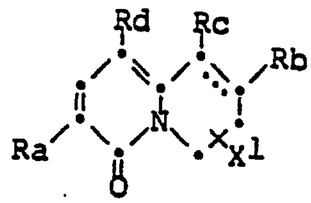
XVa

ou

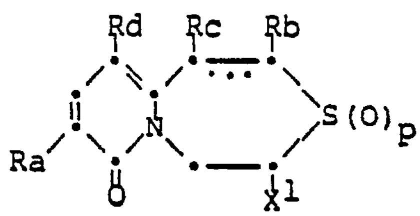
XVb

dans laquelle Ra, Rb, Rc, Rd, $X^1$, le trait en pointillé et p ont les significations indiquées ci-dessus, ou bien

n) on soumet à S-oxydation un composé de formule générale

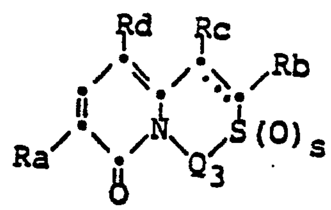
If

dans laquelle $Q^3$ représente un groupe $-CH_2-$, $-CH_2CH_2-$ ou $-CH=CH-$ et s est égal à 0 ou 1, et Ra, Rb, Rc, Rd et le trait en pointillé ont les significations indiquées ci-dessus, ou bien

o) on chauffe un composé de formule générale

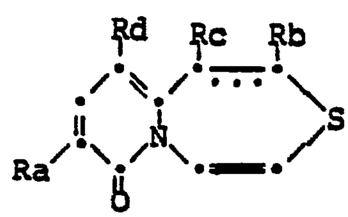
Ig

dans laquelle Ra, Rb, Rc, Rd et le trait en pointillé ont les significations indiquées ci-dessus, ou bien

p) on halogène sur le cycle thiophène un composé de formule générale

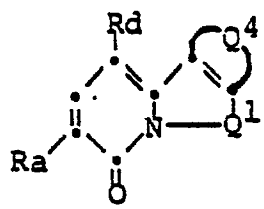
Ih

dans laquelle $Q^4$ représente le groupe (h) ou (i) ci-dessus et $Q^1$, Ra et Rd ont les significations indiquées ci-dessus, ou bien

q) on fait réagir un composé de formule VIIIc ci-dessus en présence d'une base avec un composé de formule générale

$HYN=C(NH_2)-R''$ XVI, $H_2N-CHR''-CHR'''-Y'H$ XVII

ou $H_2N-NH-C(R'')=Y''$ XVIII

dans lesquelles Y représente un atome d'oxygène ou le groupe $-NR'''-$, Y' représente un atome d'oxygène ou le groupe $-NH-$, Y'' représente un atome d'oxygène ou de soufre et R'' et R''' représentant

chacun l'hydrogène ou un groupe alkyle inférieur,
et on cyclise le produit obtenu, ou bien
r) on fait réagir un composé de formule générale

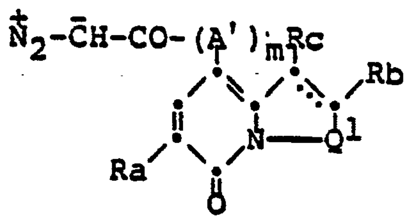

VIIId

dans laquelle A′ représente un groupe alkylène en C 1–C 6 et $Q^1$, Ra, Rb, Rc, le trait en pointillé et m ont les significations indiquées ci-dessus,
avec un alcool inférieur, ou bien
s) on soumet à décarboxylation un acide carboxylique de formule Ia dans laquelle m est égal à 0, ou bien
t) on halogène sur le cycle pyridone un composé de formule I dans laquelle Rd représente l'hydrogène, ou bien
u) dans un composé de formule générale

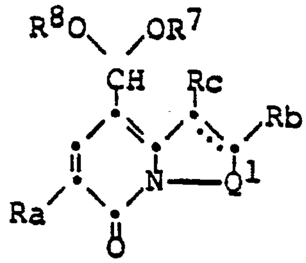

VIIIe

dans laquelle $R^7$ et $R^8$ représentent chacun un groupe alkyle inférieur ou forment ensemble un groupe alkylène inférieur et $Q^1$, Ra, Rb, Rc et le trait en pointillé ont les significations indiquées ci-dessus,
on scinde le groupe acétal, ou bien
v) on hydrogène un composé de formule I dans laquelle $Q^1$ forme avec l'atome d'azote le groupe >N–CH=CH–, ou bien
w) on fait réagir un composé de formule générale

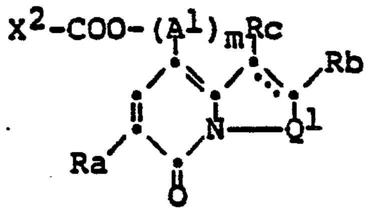

VIIIf

dans laquelle $X^2$ représente un groupe phénoxy et $A^1$, $Q^1$, Ra, Rb, Rc, le trait en pointillé et m ont les significations indiquées ci-dessus,
avec une amine de formule X ci-dessus, et
x) si on le désire, on convertit un composé obtenu, répondant à la formule I et contenant un substituant basique, en un sel acceptable pour l'usage pharmaceutique formé par addition avec un acide.

2. Procédé selon la revendication 1, dans lequel Ra représente un groupe phényle éventuellement substitué par des halogènes, des groupes trifluorométhyle, nitro, alkyle inférieurs ou alcoxy inférieurs, Rb et Rc forment ensemble et avec l'atome de carbone marqué α un groupe de formule >$C_\alpha$–S–CH=CH– (h), >$C_\alpha$–CH=CH–S– (i) ou >$C_\alpha$–CH=CH–CH=CH– (j) éventuellement substitué par des halogènes, des groupes trifluorométhyle, alkyle inférieurs, alcoxy inférieurs, nitro, amino ou mono- ou di-(alkyle inférieur)-amino, et le trait en pointillé représente une liaison supplémentaire, $A^2$ représente un groupe alkylène inférieur ou le groupe –CO–, $R^1$ représente l'hydrogène, un groupe hydroxy, cyano, nitro, un halogène, un groupe alcoxy inférieur, alkyle inférieur, alcoxycarbonyle inférieur, aryle, un groupe de formule $-NR^3R^4$ ou un hétérocycle partiellement insaturé ou aromatique à 5 chaînons, relié par l'intermédiaire d'un atome de carbone, éventuellement substitué par un ou deux groupes alkyle inférieurs, $R^3$ et $R^4$ représentent chacun l'hydrogène, un groupe alkyle inférieur, un groupe cycloalkyle en C 5–C 6 ou un

groupe alcoxycarbonyle inférieur ou forment ensemble et avec l'atome d'azote un hétérocycle azoté saturé à 5 ou 6 chaînons éventuellement substitué par un ou deux groupes alkyle inférieurs et qui peut encore être substitué sur un atome de carbone par un groupe oxo, hydroxy, alcoxy inférieur, alcoxyalkyle inférieur, hydroxyalkyle inférieur, alcoxycarbonyle inférieur, carbamoyle ou mono- ou di-(alkyle inférieur)-carbamoyle ou qui peut encore contenir en tant que chaînon cyclique un atome d'oxygène ou de soufre ou le groupe >N–$R^5$, et $R^5$ représente l'hydrogène, un groupe alkyle inférieur, hydroxyalkyle inférieur, alcoxyalkyle inférieur, alcanoyle inférieur, alcoxycarbonyle inférieur, carbamoyle ou mono- ou di-(alkyle inférieur)-carbamoyle.

3. Procédé selon la revendication 1 ou 2, dans lequel $Q^1$ forme avec l'atome d'azote le groupe de formule >N–$CH_2CH_2$– (a) ou >N–CH=CH– (c).

4. Procédé selon l'une des revendications 1 à 3, dans lequel Ra représente un groupe phényle éventuellement substitué en méta par un halogène ou un groupe trifluorométhyle.

5. Procédé selon la revendication 4, dans lequel Ra représente un groupe phényle.

6. Procédé selon l'une des revendications 1 à 5, dans lequel Rb et Rc forment ensemble et avec l'atome de carbone marqué α un groupe de formule >$C_\alpha$–S–CH=CH– ou >$C_\alpha$–CH=CH–CH=CH– éventuellement substitué par des halogènes et le trait en pointillé représente une liaison supplémentaire.

7. Procédé selon la revendication 6, dans lequel Rb et Rc forment avec l'atome de carbone marqué α le groupe de formule >$C_\alpha$–S–CH=CH– ou >$C_\alpha$–CH=CCl–CH=CH–.

8. Procédé selon l'une des revendications 1 et 3 à 7, dans lequel $Q^2$ représente un atome d'oxygène, $A^2$ le groupe –CO–, $R^1$ le groupe –$NR^3R^4$, $R^3$ un groupe alcoxy alkyle inférieur et $R^4$ l'hydrogène ou un groupe alkyle inférieur, ou bien $R^3$ et $R^4$ forment ensemble et avec l'atome d'azote un hétérocycle azoté saturé à 4, 5 ou 6 chaînons, éventuellement substitué par un ou deux groupes alkyle inférieurs et éventuellement substitué par un groupe hydroxy, alcoxy inférieur, hydroxyalkyle inférieur ou alcoxyalkyle inférieur et qui peut encore contenir en tant que chaînon cyclique un atome d'oxygène, et ou bien m et q sont égaux à 0 et n à 1, ou bien m et q sont égaux à 1 et n à 0.

9. Procédé selon la revendication 8, dans lequel $A^1$ représente un groupe méthylène et $R^3$ un groupe alcoxyalkyle inférieur et $R^4$ l'hydrogène ou un groupe alkyle inférieur ou bien $R^3$ et $R^4$ forment ensemble et avec l'atome d'azote un groupe 1-azétidinyle, 1-pyrrolidinyle, 1-pipéridinyle ou 4-morpholinyle éventuellement substitué par un ou deux groupes alkyle inférieurs et éventuellement substitué par un groupe hydroxy, alcoxy inférieur, hydroxyalkyle inférieur ou alcoxyalkyle inférieur.

10. Procédé selon la revendication 9, dans lequel $R^3$ représente un groupe 2-(alcoxy inférieur)-éthyle et $R^4$ l'hydrogène ou un groupe alkyle inférieur ou bien $R^3$ et $R^4$ forment ensemble et avec l'atome d'azote un groupe 3-(alcoxy inférieur)-1-azétidinyle, 3-(alcoxy inférieur)-1-pyrrolidinyle, 2-(alcoxyalkyle inférieur)-1-pyrrolidinyle, 2-(hydroxyalkyle inférieur)-1-pyrrolidinyle, 4-hydroxy-1-pipéridinyle, 4-(alcoxy inférieur)-1-pipéridinyle, 4-morpholinyle ou 2,6-di-(alkyle inférieur)-4-morpholinyle.

11. Procédé selon l'une des revendications 1 à 7, dans lequel m et q sont égaux à 0, n est égal à 1 et $R^1$ représente un groupe hydroxy ou alcoxy inférieur.

12. Procédé selon la revendication 1, caractérisé en ce que l'on prépare le 10-chloro-6,7-dihydro-N-(2-méthoxyéthyl)-4-oxo-3-phényl-4H-benzo[a]quinolizine-1-carboxamide.

13. Procédé selon la revendication 1, caractérisé en ce que l'on prépare le 1-[(10-chloro-6,7-dihydro-4-oxo-3-phényl-4H-benzo[a]quinolizine-1-yl)-carbonyl]-4-pipéridinol.

14. Procédé selon la revendication 1, caractérisé en ce que l'on prépare le (4,5-dihydro-7-oxo-8-phényl-7H-thiéno[2,3-a]quinolizine-10-yl)-méthyl-4-morpholine-carboxylate.

15. Procédé selon la revendication 1, caractérisé en ce que l'on prépare la 4-[(6,7-dihydro-4-oxo-3-phényl-10-chloro-4H-benzo[a]quinolizine-1-yl)-carbonyl]-2,6-diméthylmorpholine.

16. Procédé selon la revendication 1, caractérisé en ce que l'on prépare le (S)-1-[(7-oxo-8-phényl-7H-thiéno[2,3-a]quinolizine-10-yl)-carbonyl]-2-pyrrolidine-méthanol.

17. Procédé selon la revendication 1, caractérisé en ce que l'on prépare la (S)-2-méthoxyméthyl-1-[(7-oxo-8-phényl-7H-thiéno[2,3-a]quinolizine-10-yl)-carbonyl]-pyrrolidine.

18. Procédé selon la revendication 1, caractérisé en ce que l'on prépare la 1-[(10-chloro-4-oxo-3-phényl-4H-benzo[a]quinolizine-1-yl)-carbonyl]-3-méthoxypyrrolidine.

19. Procédé selon la revendication 1, caractérisé en ce que l'on prépare le (S)-1-[(10-chloro-4-oxo-3-phényl-4H-benzo[a]quinolizine-1-yl)-carbonyl]-2-pyrrolidine-méthanol.

20. Procédé selon la revendication 1, caractérisé en ce que l'on prépare la cis-4-[(4,5-dihydro-7-oxo-8-phényl-7H-thiéno[2,3-a]quinolizine-10-yl)-carbonyl]-2,6-diméthylmorpholine.

21. Procédé selon la revendication 1, caractérisé en ce que l'on prépare la 1-[(10-chloro-6,7-dihydro-4-oxo-3-phényl-4H-benzo[a]quinolizine-1-yl)-carbonyl]-4-méthoxypipéridine.

22. Procédé selon la revendication 1, caractérisé en ce que l'on prépare la 1-[(10-chloro-3-phényl-4-oxo-3-benzo[a]quinolizine-1-yl)-carbonyl]-4-méthoxypipéridine.

23. Procédé selon la revendication 1, caractérisé en ce que l'on prépare le N-éthyl-N-(2-méthoxyéthyl)-7-oxo-8-phényl-7H-thiéno[2,3-a]quinolizine-10-carboxamide.

24. Procédé selon la revendication 1, caractérisé en ce que l'on prépare le N-(2-méthoxyéthyl)-N-méthyl-7-oxo-8-phényl-7H-thiéno[2,3-a]quinolizine-10-carboxamide.

25. Procédé selon la revendication 1, caractérisé en ce que l'on prépare la (R)-2-(méthoxyméthyl)-1-[(7-oxo-8-phényl-7H-thiéno[2,3-a]quinolizine-10-yl)-carbonyl]-pyrrolidine.

26. Procédé selon la revendication 1, caractérisé en ce que l'on prépare la 1-[(10-chloro-6,7-dihydro-4-oxo-3-phényl-4H-benzo[a]quinolizine-1-yl)-carbonyl]-3-méthoxypyrrolidine.

27. Procédé selon la revendication 1, caractérisé en ce que l'on prépare la 1-[(10-chloro-6,7-dihydro-4-oxo-3-phényl-4H-benzo[a]quinolizine-1-yl)-carbonyl]-3-éthoxypyrrolidine.

28. Procédé selon la revendication 1, caractérisé en ce que l'on prépare la (R)-3-méthoxy-1-[(7-oxo-8-phényl-7H-thiéno[2,3-a]quinolizine-10-yl)-carbonyl]-pyrrolidine.

29. Procédé selon la revendication 1, caractérisé en ce que l'on prépare la (S)-3-méthoxy-1-[(7-oxo-8-phényl-7H-thiéno[2,3-a]quinolizine-10-yl)-carbonyl]-pyrrolidine.

30. Procédé selon la revendication 1, caractérisé en ce que l'on prépare la (S)-1-[(10-chloro-6,7-dihydro-4-oxo-3-phényl-4H-benzo[a]quinolizine-1-yl)-carbonyl]-2-(méthoxyméthyl)-pyrrolidine.

31. Procédé selon la revendication 1, caractérisé en ce que l'on prépare la 1-[(4,5-dihydro-7-oxo-8-phényl-7H-thiéno[2,3-a]quinolizine-10-yl)-carbonyl]-3-méthoxypyrrolidine.

32. Procédé selon la revendication 1, caractérisé en ce que l'on prépare la 3-méthoxy-1-[(7-oxo-8-phényl-7H-thiéno[2,3-a]quinolizine-10-yl)-carbonyl]-azétidine.

33. Procédé selon la revendication 1, caractérisé en ce que l'on prépare le (R)-1-[(10-chloro-4-oxo-3-phényl-4H-benzo[a]quinolizine-1-yl)-carbonyl]-2-pyrrolidine-méthanol.

34. Procédé selon la revendication 1, caractérisé en ce que l'on prépare la (S)-1-[(10-chloro-4-oxo-3-phényl-4H-benzo[a]quinolizine-1-yl)-carbonyl]-2-(méthoxyméthyl)-pyrrolidine.

35. Procédé selon la revendication 1, caractérisé en ce que l'on prépare la (R)-1-[(10-chloro-4-oxo-3-phényl-4H-benzo[a]quinolizine-1-yl)-carbonyl]-2-(méthoxyméthyl)-pyrrolidine.